(19) Europäisches Patentamt
European Patent Office
Office européen des brevets

(11) Veröffentlichungsnummer: **0 288 758 B1**

(12) # EUROPÄISCHE PATENTSCHRIFT

(45) Veröffentlichungstag der Patentschrift: **14.08.91**

(51) Int. Cl.⁵: **C07D 211/90**, C07D 401/12, A61K 31/445

(21) Anmeldenummer: **88104951.4**

(22) Anmeldetag: **28.03.88**

Die Akte enthält technische Angaben, die nach dem Eingang der Anmeldung eingereicht wurden und die nicht in dieser Patentschrift enthalten sind.

(54) **Dihydropyridinamide, Verfahren zu ihrer Herstellung und ihre Verwendung in Arzneimitteln.**

(30) Priorität: **09.04.87 DE 3711991**

(43) Veröffentlichungstag der Anmeldung: **02.11.88 Patentblatt 88/44**

(45) Bekanntmachung des Hinweises auf die Patenterteilung: **14.08.91 Patentblatt 91/33**

(84) Benannte Vertragsstaaten: **AT BE CH DE ES FR GB GR IT LI LU NL SE**

(56) Entgegenhaltungen:
**EP-A- 0 069 033**
**EP-A- 0 111 453**
**EP-A- 0 255 710**
**FR-A- 2 187 349**

(73) Patentinhaber: **BAYER AG**
**W-5090 Leverkusen 1 Bayerwerk(DE)**

(72) Erfinder: **Stoltefuss, Jürgen, Dipl.-Ing.**
**Parkstrasse 20**
**W-5657 Haan(DE)**
Erfinder: **Schwenner, Eckhard, Dr.**
**Paul-Ehrlich-Strasse 29**
**W-5600 Wuppertal 1(DE)**
Erfinder: **Bechem, Martin, Dr.**
**Obere Bergerheide 4**
**W-5600 Wuppertal 1(DE)**
Erfinder: **Hirth, Claudia, Dr.**
**Stockmannsmuehle 127**
**W-5600 Wuppertal 1(DE)**
Erfinder: **Kayser, Michael, Dr.**
**Walter-Flex-Strasse 18**
**W-5090 Leverkusen(DE)**
Erfinder: **Schramm, Matthias, Dr.**
**Paffrather Strasse 38**
**W-5000 Koeln 80(DE)**
Erfinder: **Stasch, Johannes-Peter, Dr.**
**Schneewittchenweg 37**
**W-5600 Wuppertal 1(DE)**

**EP 0 288 758 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Dihydropyridinamide, Verfahren zu ihrer Herstellung sowie ihre Verwendung in Arzneimitteln, insbesondere als kreislaufbeeinflussende Arzneimittel.

Es ist bekannt, daß man 1,4-Dihydro-2,6-dimethyl-4-phenyl-pyridin-3,5-dicarbonsäurediethylester erhält, wenn man Benzylidenacetessigsäureethylester mit β-Aminocrotonsäureethylester oder Acetessigsäureethylester und Ammoniak umsetzt (E. Knoevenagel, Ber. Dtsch. Chem. Ges. 31, 743 (1898)).

Weiterhin ist bekannt, daß bestimmte 1,4-Dihydropyndine interessante pharmakologische Eigenschaften aufweisen (F. Bossert, W. Vater, Naturwissenschaften 58, 578 (1971)).

Im Vergleich zu strukturell ähnlichen Dihydropyndinen, wie sie z.B. aus FR-A-2 187 349, DE-A-2 228 377 und US-A-4 492 703 beschrieben sind, zeigen die erfindungsgemäßen Verbindungen unerwartete bessere pharmakologische Wirkungen.

Die vorliegende Erfindung betrifft neue Dihydropyndinamide der allgemeinen Formel (I)

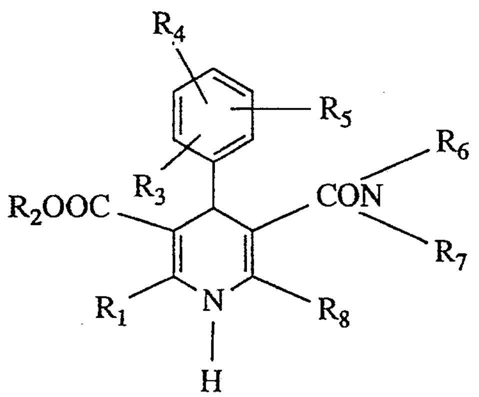

(I)

in welcher

$R_1$ und $R_8$ gleich oder verschieden sind und jeweils für geradkettiges oder verzweigtes Alkyl mit bis zu 4 Kohlenstoffatomen stehen, das gegebenenfalls durch Phenyl substituiert ist,

$R_2$ für einen geradkettiges oder verzweigten, gesättigten oder ungesättigten Kohlenwasserstoffrest mit bis zu 8 Kohlenstoffatomen steht, der durch ein Sauerstoffatom in der Kette unterbrochen sein kann und/oder der substituiert sein kann durch Fluor, Chlor, Cyano, Hydroxy oder durch eine Phenylgruppe oder durch α-, β- oder γ-Pyridyl,

$R_3$ und $R_4$ gleich oder verschieden sind und jeweils für Wasserstoff, Fluor, Chlor, Alkyl mit bis zu 4 Kohlenstoffatomen, Alkoxy mit bis zu 4 Kohlenstoffatomen, Nitro oder Trifluormethyl stehen,

$R_5$ für eine Gruppe der Formel $-O-(CH_2)_n-R_{11}$, $-S-(CH_2)_n-R_{11}$, $-O-SO_2-R_{11}$ oder $-O-CO-(CH_2)_n-R_{11}$ steht,

worin

n 0 bis 3 bedeutet, und

$R_{11}$ Phenyl bedeutet, das bis zu 3-fach gleich oder verschieden substituiert sein kann durch Fluor, Chlor, Nitro, Trifluormethyl, Alkyl mit bis zu 4 Kohlenstoffatomen, Alkoxy mit bis zu 4 Kohlenstoffatomen, Amino, Alkylamino mit bis zu 4 Kohlenstoffatomen, Dialkylamino mit jeweils bis zu 4 Kohlenstoffatomen je Alkylgruppe, Acetylamino oder Pyridyl bedeutet, und

$R_6$ und $R_7$ gleich oder verschieden sind und jeweils für Wasserstoff oder Cycloalkyl mit 3 bis 7 Kohlenstoffatomen stehen oder für geradkettiges oder verzweigtes Alkyl oder Alkenyl mit bis zu 14 Kohlenstoffatomen stehen, die substituiert sein können durch Fluor, Chlor, Hydroxy, Alkoxy mit bis zu 6 Kohlenstoffatomen, Alkylthio mit bis zu 6 Kohlenstoffatomen, Carboxy, Alkoxycarbonyl mit bis zu 6 Kohlenstoffatomen, durch Phenyl und/oder durch eine Gruppe der Formel $-NR_9R_{10}$,

worin

$R_9$ und $R_{10}$ gleich oder verschieden sind und jeweils Wasserstoff, Alkyl mit bis zu 6 Kohlenstoffatomen, Benzyl, Phenyl oder Acetyl bedeuten, oder für Phenyl stehen, das bis zu 3-fach gleich oder verschieden substituiert sein kann durch Nitro, Fluor, Chlor, Alkyl mit

bis zu 4 Kohlenstoffatomen, Alkoxy mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino oder für Pyridyl stehen,

und deren physiologisch unbedenklichen Salze.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vergl. E.L. Eliel, Stereochemistry of Carbon Compounds, McGraw Hill, 1962).

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Apfelsäure, Zitronensäure, Weinsäure, Milchsäure, Benzoesäure, oder Methansulfonsäure, Ethansulfonsäure, Phenylsulfonsäure, Toluolsulfonsäure oder Naphthalidinsulfonsäure.

Bevorzugt sind Verbindungen der allgemeinen Formel (I), in welcher

$R_{11}$ Phenyl bedeutet, das bis zu 3-fach gleich oder verschieden substituiert sein kann durch Fluor, Chlor, Nitro, Trifluormethyl, Alkyl mit bis zu 4 Kohlenstoffatomen, Alkoxy mit bis zu 4 Kohlenstoffatomen oder Pyridyl bedeutet,

und

$R_6$ und $R_7$ gleich oder verschieden sind und jeweils für Wasserstoff oder Cycloalkyl mit 3 bis 7 Kohlenstoffatomen stehen oder für geradkettiges oder verzweigtes Alkyl oder Alkenyl mit bis zu 14 Kohlenstoffatomen stehen, die substituiert sein können durch Fluor, Chlor, Hydroxy, Alkoxy mit bis zu 6 Kohlenstoffatomen, Carboxy, Alkoxycarbonyl mit bis zu 6 Kohlenstoffatomen, durch Phenyl und/oder durch eine Gruppe der Formel $-NR_9R_{10}$, worin $R_9$ und $R_{10}$ gleich oder verschieden sind und jeweils Wasserstoff, Alkyl mit bis zu 6 Kohlenstoffatomen, Benzyl, Phenyl, Acetyl bedeuten oder für Phenyl stehen, das bis zu 3-fach gleich oder verschieden substituiert sein kann durch Nitro, Fluor, Chlor, Alkyl mit bis zu 4 Kohlenstoffatomen, Alkoxy mit bis zu 4 Kohlenstoffatomen, Trifluormethyl, Amino, Acetylamino oder durch Benzoylamino oder für Pyridyl stehen,

und deren physiologisch unbedenklichen Salze.

Die erfindungsgemäßen Verbindungen der allgemeinen Formel (I), in welcher $R_1$ bis $R_8$ die oben angegebene Bedeutung haben,

erhält man, indem man

[A] Aldehyde der allgemeinen Formel (II)

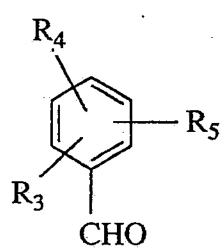 (II)

in welcher

$R_3$, $R_4$ und $R_5$ die oben angegebene Bedeutung haben,

und $\beta$-Ketocarbonsäureester der allgemeinen Formel (III)

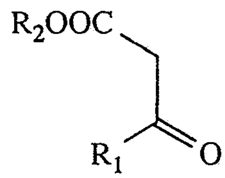

(III)

in welcher
$R_1$ und $R_2$ die oben angegebene Bedeutung haben,
mit $\beta$-Ketocarbonsäureamiden der allgemeinen Formel (IV)

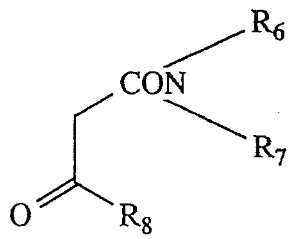

(IV)

in welcher
$R_6$, $R_7$ und $R_8$ die oben angegebene Bedeutung haben,
und Ammoniak, gegebenenfalls in Anwesenheit von inerten Lösemitteln umsetzt,
oder daß
[B] Aldehyde der allgemeinen Formel (II) mit $\beta$-Ketocarbonsäureestern der allgemeinen Formel (III) und Enaminocarbonsäureamiden der allgemeinen Formel (V)

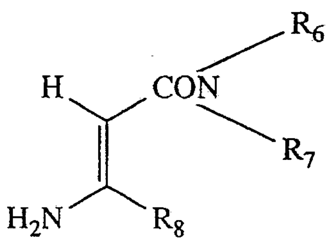

(V)

in welcher
$R_6$, $R_7$ und $R_8$ die oben angegebene Bedeutung haben,
gegebenenfalls in Anwesenheit von inerten Lösemitteln umsetzt,
oder daß
[C] Aldehyde der allgemeinen Formel (II) mit $\beta$-Ketocarbonsäureamiden der allgemeinen Formel (IV) und Enaminocarbonsäureestern der allgemeinen Formel (VI)

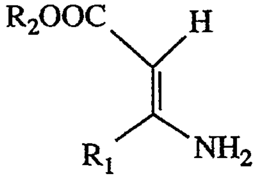

(VI)

in welcher
$R_1$ und $R_2$ die oben angegebene Bedeutung haben,
gegebenenfalls in Anwesenheit von inerten Lösemitteln umsetzt,

oder daß
[D] β-Ketocarbonsäureester der allgemeinen Formel (III) mit Ammoniak und Yliden-β-ketocarbonsäureamiden der allgemeinen Formel (VII)

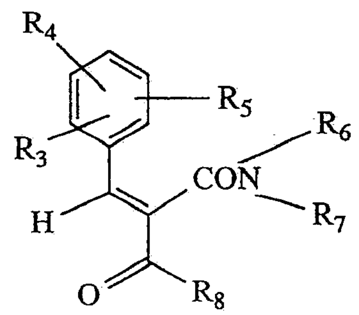

(VII)

in welcher
$R_3$ - $R_8$ die oben angegebene Bedeutung haben,
gegebenenfalls in Anwesenheit von inerten Lösemitteln umsetzt,
oder daß
[E] β-Ketocarbonsäureamide der allgemeinen Formel (IV) mit Ammoniak und Yliden-β-ketocarbonsäureestern der allgemeinen Formel (VIII)

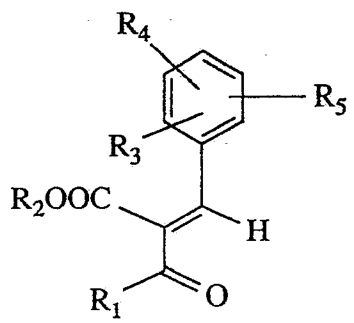

(VIII)

in welcher
$R_1$ - $R_5$ die oben angegebene Bedeutung haben,
gegebenenfalls in Anwesenheit von inerten Lösemitteln umsetzt,
oder daß
[Fl Yliden-β-ketocarbonsäureamide der allgemeinen Formel (VII) mit Enaminocarbonsäureestern der allgemeinen Formel (VI) gegebenenfalls in Anwesenheit von inerten Lösemitteln umsetzt,
oder daß
[G] Yliden-β-ketocarbonsäureester der allgemeinen Formel (VIII) mit Enaminocarbonsäureamiden der allgemeinen Formel (V) gegebenenfalls in Anwesenheit von inerten Lösemitteln umsetzt,
oder daß
[H] Dihydropyridinmonocarbonsäuren der allgemeinen Formel (IX)

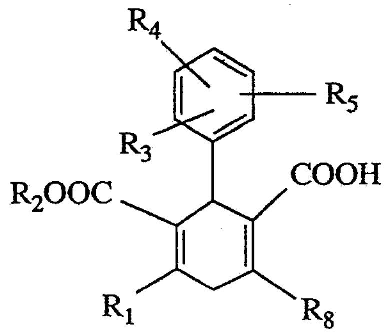

(IX)

in welcher
$R_1$ - $R_5$ und $R_8$ die oben angegebene Bedeutung haben,
gegebenenfalls über ein reaktives Säurederivat mit Aminen der allgemeinen Formel (X)

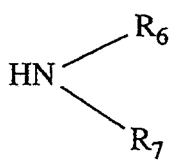

(X)

in welcher
$R_6$ und $R_7$ die oben angegebene Bedeutung haben,
gegebenenfalls in Anwesenheit eines inerten organischen Lösemittels umsetzt.

Als reaktive Säurederivate seien beispielsweise genannt: aktivierte Ester, Hydroxysuccinimidester, Säureimidazolide, Säurehalogenide, gemischte Anhydride oder die Umsetzung in Anwesenheit von Cyclohexylcarbodiimid.

Je nach Art der verwendeten Ausgangsstoffe können die Synthesevarianten für die erfindungsgemäßen Verbindungen durch folgende Formelschemata wiedergegeben werden:

[A]

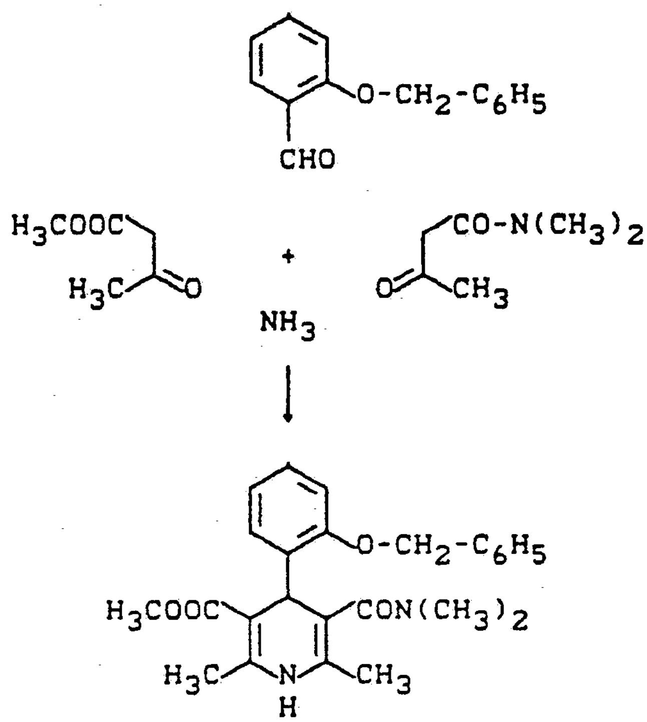

[B]

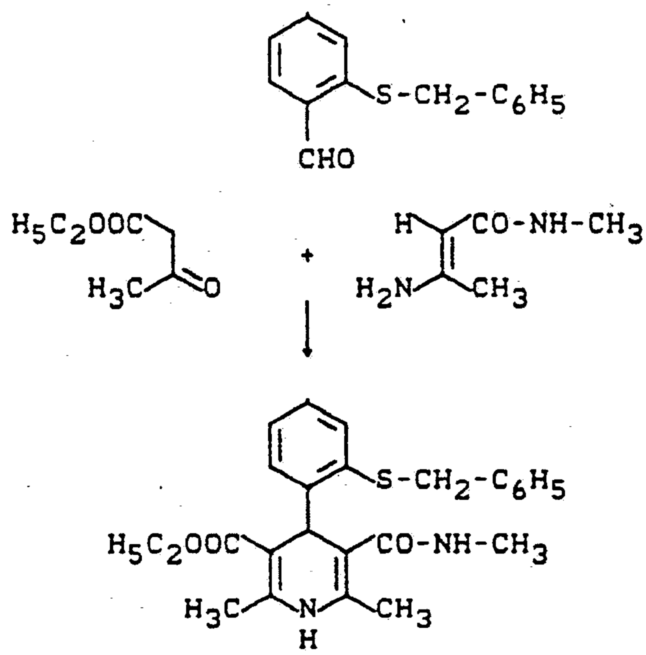

[C]

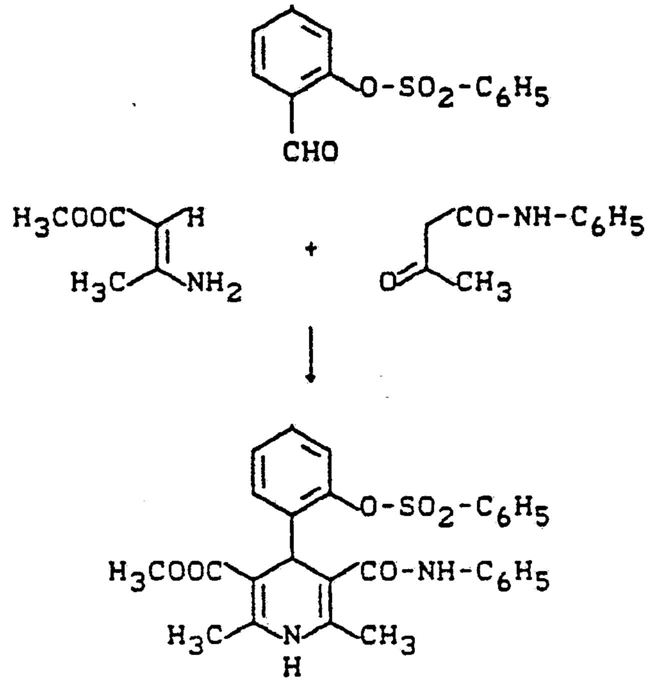

[D]

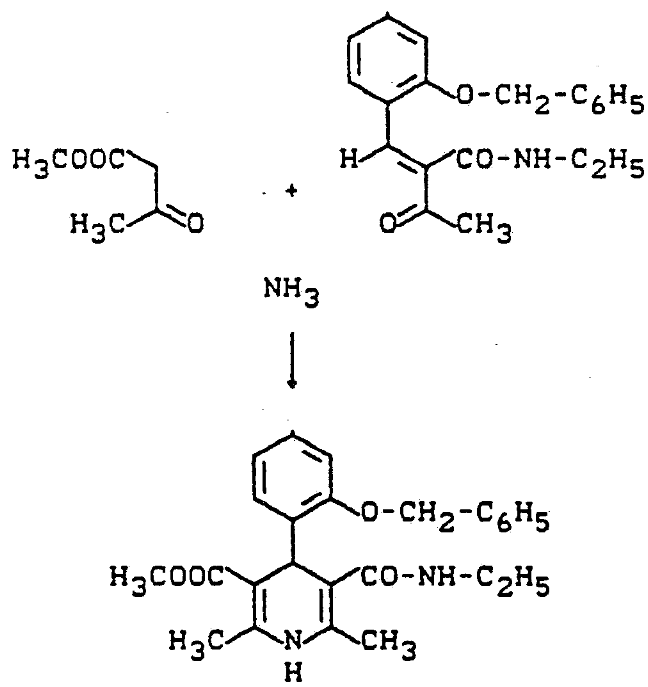

[E]

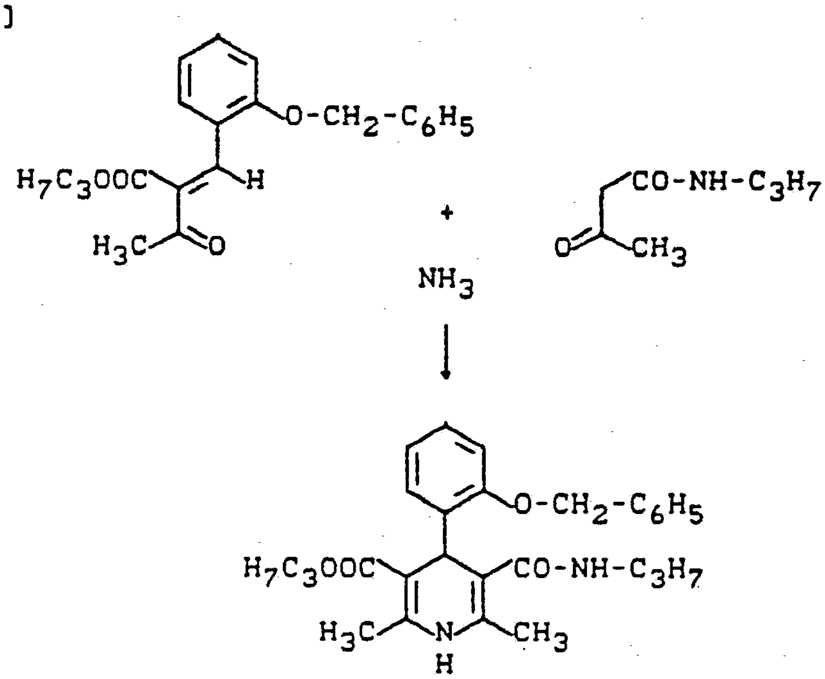


[F]

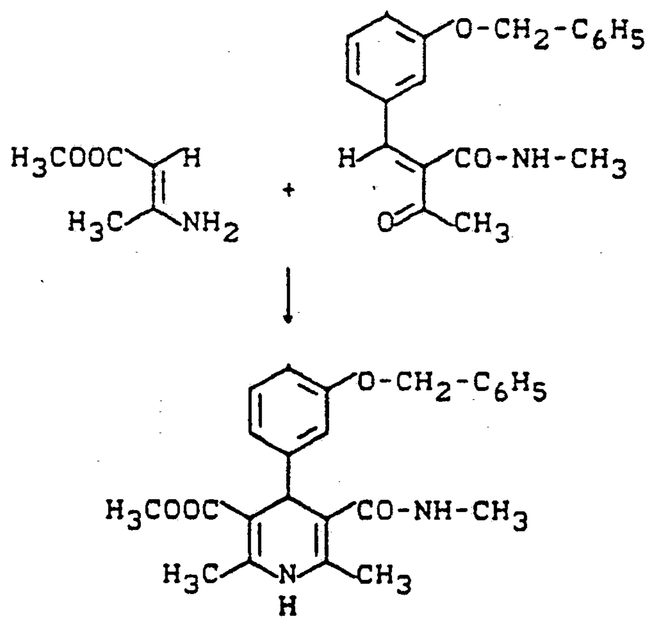

[G]

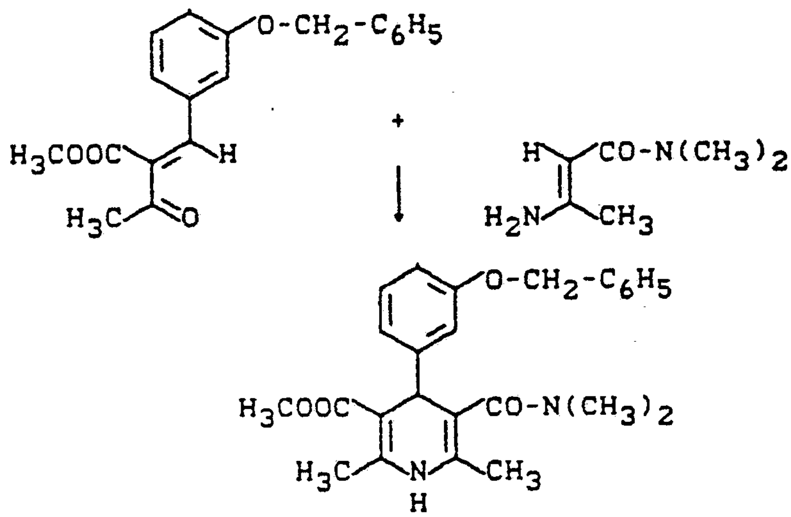

[H]

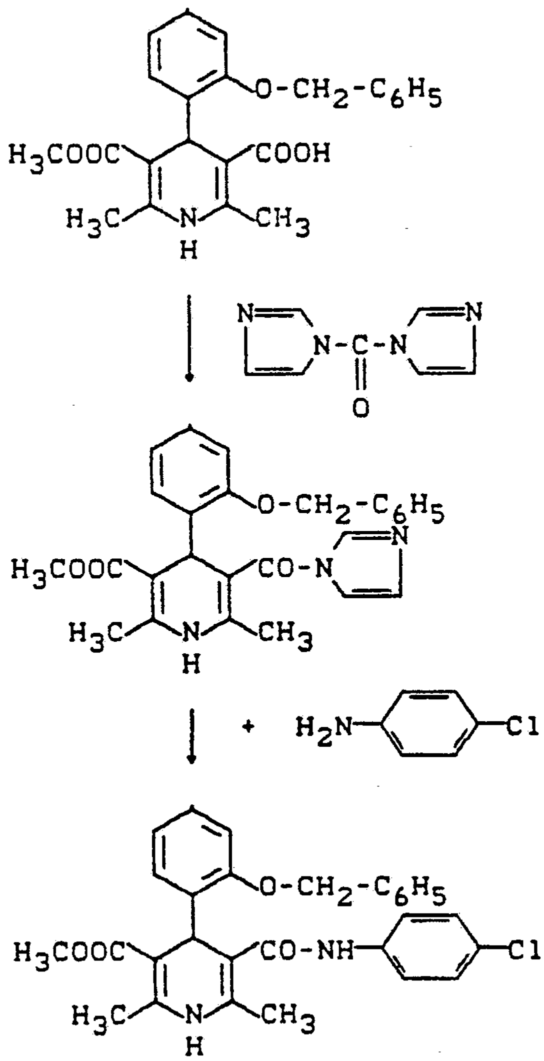

Verfahrensvarianten A - G

Als Lösemittel kommen Wasser oder alle inerten organischen Lösemittel in Frage, die sich unter den Reaktionsbedingungen nicht verändern. Hierzu gehören vorzugsweise Alkohole wie Methanol, Ethanol,

Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin.

Die Reaktionstemperaturen können in einem größeren Bereich variiert werden. Im allgemeinen arbeitet man zwischen +10 °C und +150 °C, vorzugsweise zwischen +20 °C und +100 °C. Insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A - G ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die als Ausgangsstoffe eingesetzten Aldehyde der allgemeinen Formel (II) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die als Ausgangsstoffe eingesetzten β-Ketocarbonsäureester der allgemeinen Formel (III) sind bekannt oder können nach bekannten Methoden hergestellt werden [D. Borrmann in Houben Weyl's "Methoden der organischen Chemie" Bd. VII/4, 230 (1968); Y. Oikawa, K. Sugano, O. Yonemitsu, J. Org. Chem. 43, 2087 (1978)].

Die als Ausgangsstoffe eingesetzten β-Ketocarbonsäureamide der allgemeinen Formel (IV) sind bekannt oder können nach bekannten Methoden hergestellt werden [DOS 11 42 859].

Die als Ausgangsstoffe eingesetzten Enaminocarbonsäureamide der allgemeinen Formel (V) sind bekannt oder können nach bekannten Methoden hergestellt werden [DOS 2 228 377].

Die als Ausgangsstoffe eingesetzten Enaminocarbonsäureester der allgemeinen Formel (VI) sind bekannt oder können nach bekannten Methoden hergestellt werden [F.A. Glickman, A.C. Cope, J. Am. Chem. Soc. 67, 1017 (1945)].

Die als Ausgangsstoffe eingesetzen Yliden-β-ketocarbonsäureamide der allgemeinen Formel (VII) und die als Ausgangsstoffe eingesetzten Yliden-β-ketocarbonsäureester der allgemeinen Formel (VIII) sind bekannt oder können nach bekannten Methoden hergestellt werden [G. Jones "The Knoevenagel Condensation" in Organic Reactions Bd. XV, 204 (1967)].

Die Durchführung der erfindungsgemäßen Verfahrensvariante H lehnt sich an die literaturbekannte Methodik zur Überführung von Carbonsäuren in Carbonsäureamide an. Dabei wird die Carbonsäure zunächst in eine aktivierte Form wie z.B. das Säurechlorid oder das Imidazolid überführt, die entweder als solche isoliert und in einem zweiten Reaktionsschritt umgesetzt werden, oder die in situ direkt zu den erfindungsgemäßen Verbindungen amidiert werden. Als aktivierende Reagenzien seien neben den anorganischen Halogeniden wie Thionylchlorid, Phosphortrichlorid oder Phosphorpentachlorid, oder dem Carbonyldiimidazol, Carbodiimide wie Cyclohexylcarbodiimid oder 1-Cyclohexyl-3-[2-(N-methyl-morpholino)ethyl]-carbodiimid-p-toluolsulfonat oder N-Hydroxyphthalimid oder N-Hydroxy-benztriazol in Gegenwart von Dicylohexylcarbodiimid beispielhaft erwähnt. Naturgemäß lassen sich die Dihydropyridinmonocarbonsäuren auch in Form ihrer Salze einsetzen. [Die Methodik der Amidierung wird beispielsweise beschrieben: Fieser & Fieser, Reagents for Organic Synthesis, John Wiley & Sons Inc. (1967), Seite 231 - 236; J.C. Shihan and G.P. Hess, J. Am. Chem. Soc. 77, 1067 (1955); U. Goodman, G.W. Kenner, Adv. in Protein Chem. 12, 488 (1957); W.A. Bonner, P.I. McNamee, J. Org. Chem. 26, 254 (1961); H.A. Staab, Angew. Chemie Int. Ed. 1, 351 (1962); Fieser & Fieser, Reagents for Organic Synthesis, John Wiley & Sons Inc. 1967, 116, 114; H.C. Beyerman, U.O. van der Brink, Re. Trav. 80, 1372 (1961); C.A. Buehler, D.E. Pearson, John Wiley & Sons, Volume I (1970), Seite 895 ff, Volume II, (1977)].

Als Lösemittel für Verfahrensvariante H kommen neben Wasser alle inerten organischen Lösemittel in Frage, die sich unter den Reaktionsbedingungen nicht verändern. Hierzu gehören vorzugsweise Ether wie Diethylether, Dioxan, Tetrahydrofuran, Glykolmonomethylether oder Glykoldimethylether, oder halogenierte Kohlenwasserstoffe wie Dichlormethan, Trichlormethan oder Tetrachlormethan, oder Amide wie Dimethylformamid, Dimethylacetamid oder Hexamethylphosphorsäuretriamid, oder Kohlenwasserstoffe wie Benzol, Toluol oder Xylol, oder Acetonitril, Nitromethan, Pyridin, Dimethylsulfoxid oder Essigester. Ebenso können Gemische der genannten Lösemittel verwendet werden. Isoliert man die aktivierten Zwischenstufen der Dihydropyridinmonocarbonsäuren, so können auch die Amine der Formel (X) alleine als Verdünnungsmittel verwendet werden.

Die Reaktionstemperaturen können in einem breiten Bereich variiert werden. Im allgemeinen arbeitet man in einem Bereich von -70 °C bis +140 °C, bevorzugt von -20 °C bis +100 °C.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvariante H ist das Verhältnis der an der Reaktion beteiligten Stoffe beliebig. Im allgemeinen arbeitet man jedoch mit molaren Mengen der Reaktanden. Es hat sich jedoch als günstig erwiesen, das Amin in einem 5- bis 10-fach molaren Überschuß einzusetzen. Besonders zweckmäßig wird das Amin in einem großen Überschuß direkt als Lösemittel eingesetzt.

Die als Ausgangsstoffe eingesetzten Dihydropyridinmonocarbonsäuren der allgemeinen Formel (IX) sind bekannt oder können nach bekannten Methoden hergestellt werden [DOS 2 847 236; 3 206 671; 2 962 241].

Die als Ausgangsstoffe eingesetzten Amine der allgemeinen Formel (X) sind bekannt oder können nach bekannten Methoden hergestellt werden [Houben Weyl's "Methoden der organischen Chemie" Bd XI/1; Paulsen, Angewandte Chemie 78, 501 - 566 (1966)].

Die erfindungsgemäßen Verbindungen zeigen ein nicht vorhersehbares, wertvolles pharmakologisches Wirkspektrum. Sie beeinflussen die Konktraktionskraft des Herzens, den Tonus der glatten Muskulatur sowie den Elektrolyt- und Flüssigkeitshaushalt.

Sie können deshalb in Arzneimitteln zur Behandlung des pathologisch veränderten Blutdrucks und der Herzinsuffizienz, sowie als Koronartherapeutika eingesetzt werden.

Darüber hinaus können sie zur Behandlung von Herzrhythmusstörungen, Niereninsuffizienz, Leberzirrhose, Aszites, Lungenödem, Hirnödem, Schwangerschaftsödem, Glaukom oder Diabetes mellitus eingesetzt werden.

Die Herzwirkung der erfindungsgemäßen Verbindungen wurde am isolierten, stimulierten Papillarmuskel des Meerschweinchenherzens gefunden. Dazu wurden die Versuchstiere (200 g schwere Meerschweinchen beiderlei Geschlechts) getötet, der Thorax geöffnet und das Herz entnommen. Für die Versuche wurden anschließend jeweils möglichst kleine Papillarmuskeln aus der rechten Herzkammer herauspräpariert und horizontal in einem Organbad fixiert. Dabei wurde das eine Ende des Muskels durch zwei Metallelektroden gehalten, die gleichzeitig zur Reizung des Präparates dienten, während das andere Ende des Muskels über einen Faden mit einem Kraftaufnehmer verbunden war. Der Papillarmuskel wurde mit einer Frequenz von 1 Hz überschwellig gereizt. Das Organbad mit einem Volumen von ca. 2 ml wurde kontinuierlich mit einer Krebs-Henseleit-Lösung (Konzentration in mM: NaCl 118; $NaCO_3$ 25; KCl 10; $KH_2PO_4$ 1.2; $MgSO_4$ 1.2; $CaCl_2$ 1.8; Glukose 10, pH 7.4) mit einer Geschwindigkeit von 4 ml/min bei einer Temperatur von 32 °C durchströmt. Die Kontraktionen des Papillarmuskels wurden isometrisch über den angeschlossenen Kraftaufnehmer gemessen und auf einem Schreiber registriert.

Die erfindungsgemäßen Stoffe wurden in der Krebs-Henseleit-Lösung in einer Konzentration von 10 $\mu$g/ml gegebenenfalls mit einem Lösungsvermittler (DMSO bis zu einer Konzentration von 0.5%) gelöst. Die erfindungsgemäßen Dihydropyridincarbonamide zeigten dabei bezogen auf die Kontrollwerte eine Hemmung der Kontraktionskraft des Papillarmuskels um mehr als 10%.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate, Arylsulfonate), Detergentien (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten

Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

$R_f$-Werte: DC-Alufolie Merck, Schichtdicke 0.2 mm, Kieselgel 60 F 254; Fließmittel Toluol/Essigester im Volumenverhältnis 1:2

Beispiel 1

4-(2-Benzyloxy-phenyl)-1,4-dihydro-2,6-dimethyl-pyridin-3-carbonsäuremethylester-5-carbonsäuredimethylamid

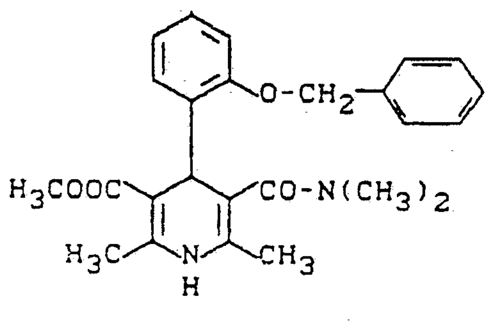

Verfahrensvariante A

2,12 g (10 mmol) 2-Benzyloxy-benzaldehyd werden mit 1,16 g (10 mmol) Acetessigsäuremethylester, 1,29 g Acetessigsäuredimethylamid und 1 ml Ammoniak 18 Stdn. gekocht. Es wird abgekühlt und eingeengt. Der Eindampfrückstand wird in Essigester aufgenommen, 2 mal mit Wasser gewaschen, getrocknet und eingeengt. Es wird über eine Kieselgelsäule mit Toluol/Essigester-Gemischen gereinigt. Die reinen Fraktionen werden gesammelt und eingeengt. Das Produkt kristal lisiert beim Verreiben mit Ether / Essigester 10:1. Man erhält 0,8 g (19% der Theorie) farblose Kristalle vom Schmp.: 162 - 163 °C.

Verfahrensvariante E

2 g (6,45 mmol) 2-Benzyloxy-benzyliden-acetessigsäuremethylester werden mit 0,83 g (6,45 mmol) Acetessigsäuredimethylamid und 0,53 ml Ammoniak 18 Stdn zum Rückfluß erhitzt. Es wird abgekühlt und eingeengt. Der Eindampfrückstand wird in Essigester aufgenommen, 2 mal mit Wasser geschüttelt, getrocknet und eingeengt. Das Produktgemisch wird über eine Kieselgelsäule mit einem Toluol/Essigester-Gemisch gereinigt. Die sauberen Fraktionen werden zusammengefaßt und eingeengt. Das Produkt kristallisiert beim Verreiben mit Ether und wenig Essigester. Es wird abgesaugt. Man erhält 0,6 g (22,9% der Theorie) einer farblosen Substanz vom Schmp.: 163 - 165° C, $R_f$-Wert = 0,118

Beispiel 2

4-(2-Benzyloxy-phenyl)-1,4-dihydro-2,6-dimethyl-pyridin-3-carbonsäuremethylester-5-carbonsäurephenylamid

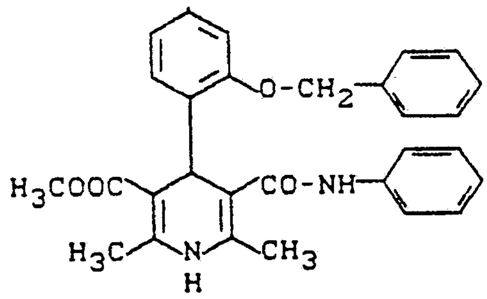

Verfahrensvariante G

3,1 g (10 mmol) 2-Benzyloxy-benzyliden-acetessigsäuremethylester werden in 20 ml Ethanol mit 1,76 g (10 mmol) β-Aminocrotonsäurebenzamid 3 Stdn. gekocht. Es wird abgekühlt und eingeengt. Der feste Eindampfrückstand wird mit Ether verrührt, abgesaugt und aus Acetonitril umkristallisiert. Man erhält 2,4 g (51,3 der Theorie) farblose Kristalle vom Schmp.: 194° C

Beispiel 3

4-(2-Benzyloxy-phenyl)-1,4-dihydro-2,6-dimethyl-pyridin-3-carbonsäuremethylester-5-carbonsäurecyclopropylamid

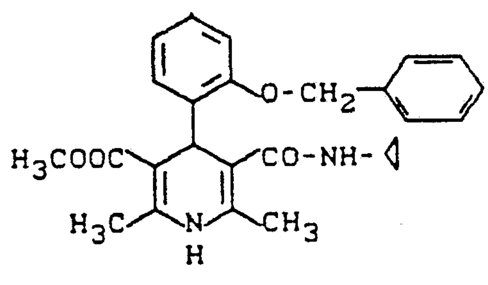

Verfahrensvariante H

20 g (45,14 mmol) 4-(2-Benzyloxy-phenyl)-1,4-dihydro-2,6-dimethyl-pyridin-3-carbonsäuremethylester-5-carbonsäure-imidazolid werden in 130 ml abs. Dimethylformamid gelöst mit 5,63 ml (80,1 mmol) Cyclopropylamin versetzt und unter Argon 20 Stunden bei 90-100° C gerührt. Es wird abgekühlt und eingeengt. Der Eindampfrückstand wird in Essigester aufgenommen, mit Wasser, 1N Salzsäure, Wasser, Natriumhydrogencarbonatlösung und wieder Wasser gewaschen, getrocknet, und eingeengt. Der Eindampfrückstand wird mit Toluol verrührt, abgesaugt und mit Toluol gewaschen. Nach Umkristallisieren aus etwa 60 ml Toluol erhält man 14,95 g (76,7% der Theorie) farblose Kristalle.
Schmp.: 191 - 193° C

Beispiel 4

1,4-Dihydro-2,6-dimethyl-4-[2-(3-trifluormethyl-benzyloxy)phenyl]pyridin-3-carbonsäuremethylester-5-carbonsäuremethylamid

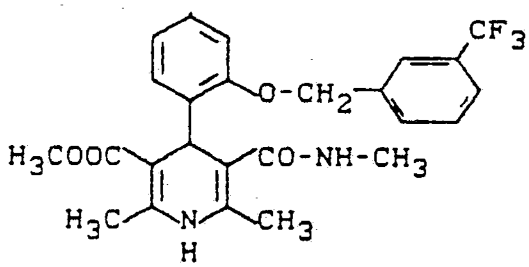

3,5 g (6,8 mmol) 1,4-Dihydro-2,6-dimethyl-4-[2-(3-trifluormethyl-benzyloxy)phenyl[pyridin-3-carbonsäuremethylester-5-carbonsäureimidazolid (erhalten aus 1,4-Dihydro-2,6-dimethyl-4-[2-(3-trifluormethylbenzyloxy)phenyl]pyridin-3,5-dicarbonsäuremonomethylester durch Umsetzung mit Carbonylbisimidazol in Tetrahydrofuran) wird ohne weitere Reinigung mit 40 ml Methylaminlösung (40%) über Nacht gerührt. Das ausgefallene Produkt wird abgesaugt und gut mit Wasser gewaschen. Es wird getrocknet und aus wenig Toluol umkristallisiert. Man erhält 1,3 g (40,3% der Theorie) eines farblosen Produktes.
Schmp.: 156-157° C
Analog den Beispielen 1 bis 4 wurden hergestellt:

Beispiel 5

1,4-Dihydro-2,6-dimethyl-4-[2-(3-methyl-benzyloxy)phenyl]-pyridin-3-carbonsäuremethylester-5-carbonsäurecyclopropylamid

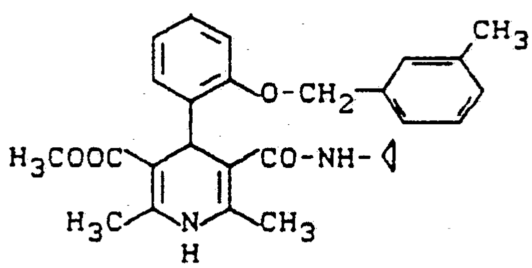

Schmp.: 176° C

Beispiel 6

4-[2-(4-Chlor-benzyloxy)phenyl]-1,4-dihydro-2,6-dimethyl-pyridin-3-carbonsäuremethylester-5-carbonsäurecyclopropyl-amid

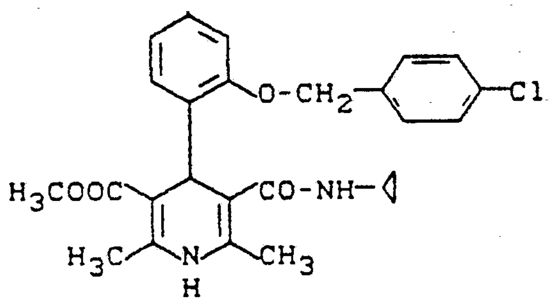

Schmp.: 178° C

Beispiel 7

4-[2-(4-Chlor-benzyloxy)phenyl]-1,4-dihydro-2,6-dimethyl-pyridin-3-carbonsäuremethylester-5-carbonsäure-(2-pyridyl)amid

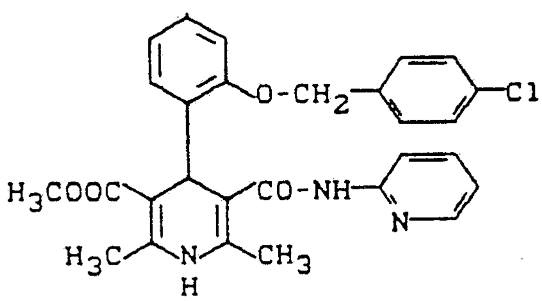

Schmp.: 201-204° C

Beispiel 8

4-(2-Benzyloxy-phenyl)-1,4-dihydro-2,6-dimethyl-pyridin-3-carbonsäuremethylester-5-carbonsäure-(2-pyridyl)amid

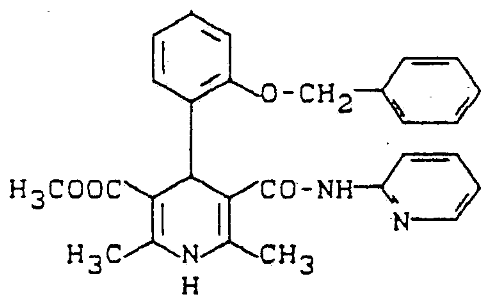

Schmp.: 164 - 165 °C

Beispiel 9

4-[2-(4-Chlor-benzyloxy)phenyl]-1,4-dihydro-2,6-dimethyl-pyridin-3-carbonsäuremethylester-5-carbonsäure-(2-diethyl-amino-ethyl)amid

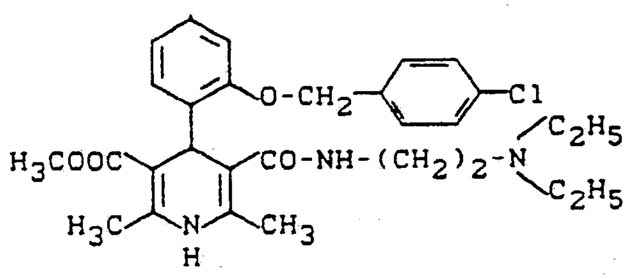

Schmp.: ab 80 °C

Beispiel 10

4-(2-Benzyloxy-phenyl)-1,4-dihydro-2,6-dimethyl-pyridin-3-carbonsäuremethylester-5-carbonsäure-(2-diethylamino-ethyl)amid

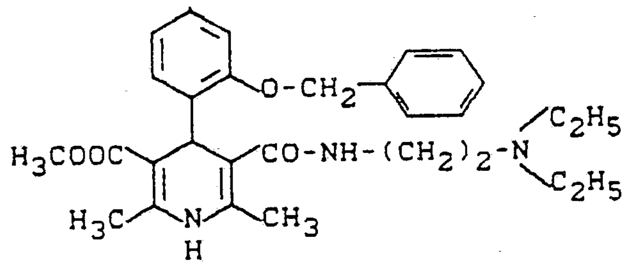

Schmp.: ab 95° C

Beispiel 11

4-(2-Benzyloxy-phenyl)-1,4-dihydro-2,6-dimethyl-pyridin-3-carbonsäuremethylester-5-carbonsäure-(4-carbamoyl-phenyl)-amid

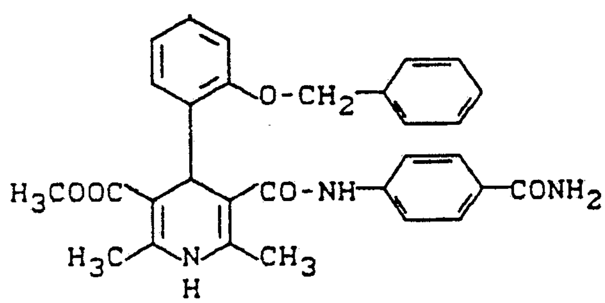

Schmp.: >300° C

Beispiel 12

4-(2-Benzyloxy-phenyl)-1,4-dihydro-2,6-dimethyl-pyridin-3-carbonsäuremethylester-5-carbonsäure-(4-acetylamino-phenyl)amid

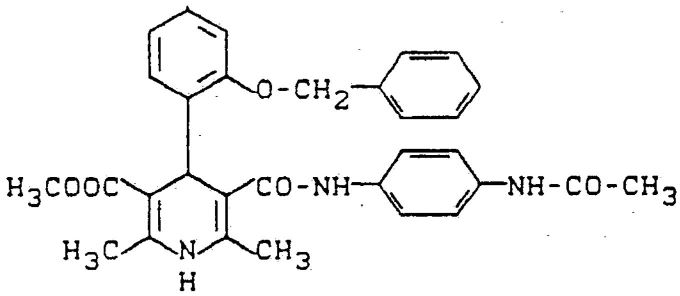

Schmp.: 298° C Zersetzung

Beispiel 13

4-(2-Benzyloxy-phenyl)-1,4-dihydro-2,6-dimethyl-pyridin-3-carbonsäuremethylester-5-carbonsäure-(4-benzoylamino-phenyl)amid

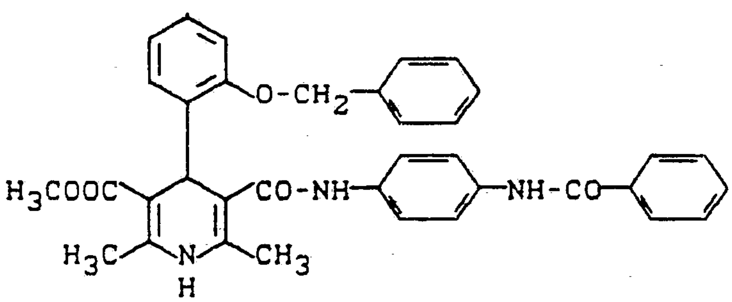

Schmp.: 197° C

Beispiel 14

(R,S)-4-(2-Benzyloxy-phenyl)-1,4-dihydro-2,6-dimethyl-pyridin-3-carbonsäuremethylester-5-carbonsäure-(1-phenyl-ethyl)amid (R,S-Gemisch)

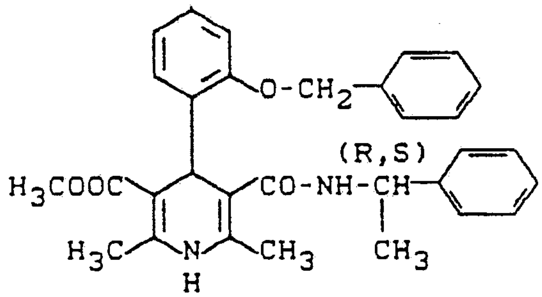

Schmp.: ab 162° C

Beispiel 15

(R)-4-(2-Benzyloxy-phenyl)-1,4-dihydro-2,6-dimethyl-pyridin-3-carbonsäuremethylester-5-carbonsäure-(1-phenyl-ethyl)amid (R-Form)

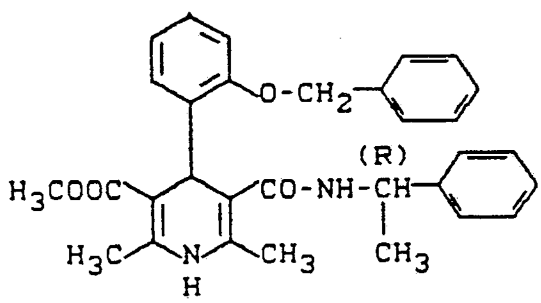

Schmp.: 82° C

Beispiel 16

4-(2-Benzyloxy-phenyl)-1,4-dihydro-2,6-dimethyl-pyridin-3-carbonsäuremethylester-5-carbonsäure-(3-dimethylamino-propyl)amid

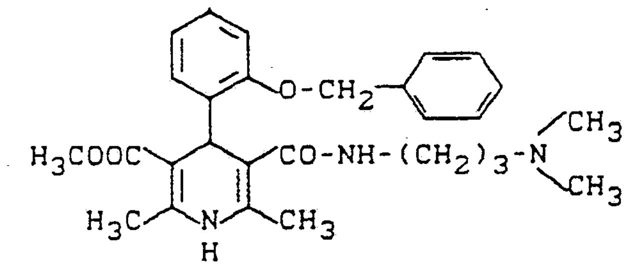

Schmp.: 89 - 90 °C

Beispiel 17

4-(2-Benzyloxy-phenyl)-1,4-dihydro-2,6-dimethyl-pyridin-3-carbonsäuremethylester-5-carbonsäurecyclohexylamid

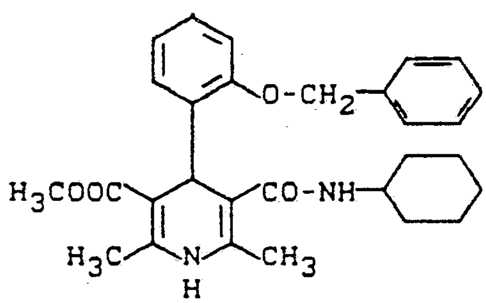

Schmp.: 108 - 111 °C

Beispiel 18

4-(2-Benzyloxy-phenyl)-1,4-dihydro-2,6-dimethyl-pyridin-3-carbonsäuremethylester-5-carbonsäure-tert.butylamid

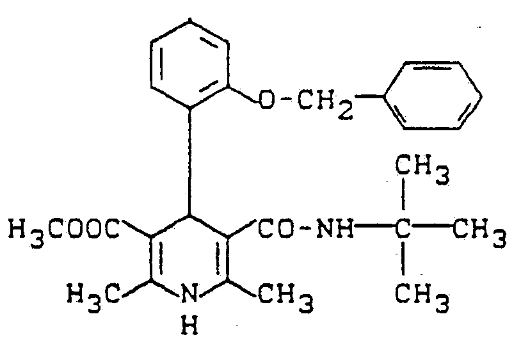

Schmp.: 148° C

Beispiel 19

4-(2-Benzyloxy-phenyl)-1,4-dihydro-2,6-dimethyl-pyridin-3-carbonsäuremethylester-5-carbonsäurepropylamid

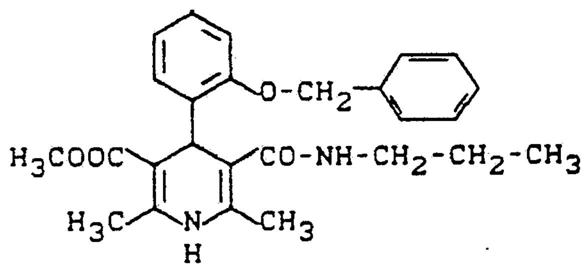

Schaum
$R_f$ = 0,37

Beispiel 20

4-(2-Benzyloxy-phenyl)-1,4-dihydro-2,6-dimethyl-pyridin-3-carbonsäuremethylester-5-carbonsäure-(2-methyl-propyl)-amid

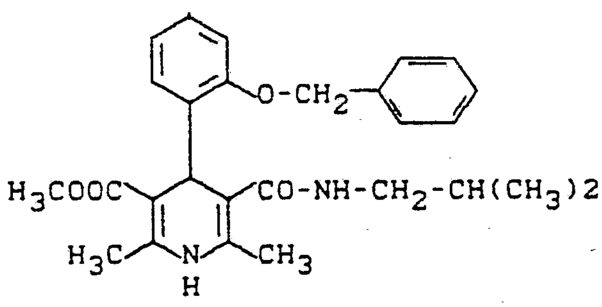

Schmp.: 134° C

Beispiel 21

4-(2-Benzyloxy-phenyl)-1,4-dihydro-2,6-dimethyl-pyridin-3-carbonsäuremethylester-5-carbonsäure-N-benzyl-N-tert.butylamid

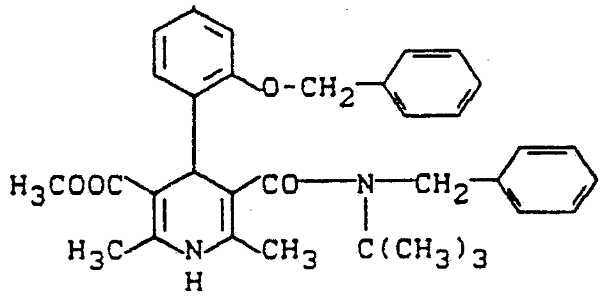

Schmp.: 128° C

Beispiel 22

4-(2-Benzyloxy-phenyl)-1,4-dihydro-2,6-dimethyl-pyridin-3-carbonsäuremethylester-5-carbonsäuremethylamid

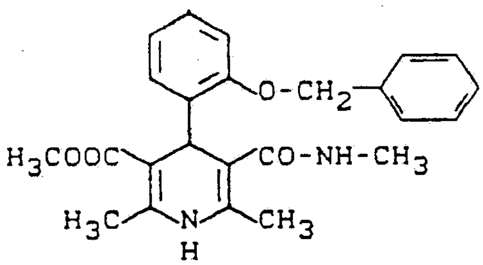

Schmp.: 105° C

Beispiel 23

4-(2-Benzyloxy-phenyl)-1,4-dihydro-2,6-dimethyl-pyridin-3-carbonsäuremethylester-5-carbonsäureethylamid

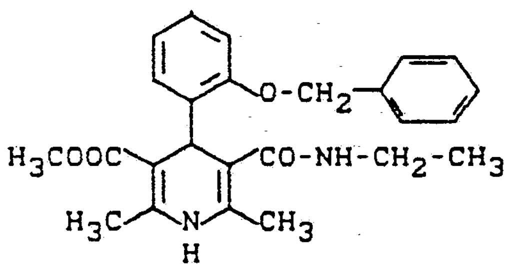

Schmp.: 172° C

Beispiel 24

1,4-Dihydro-2,6-dimethyl-4-[2-(2-pyridyl)methoxy-phenyl]-pyridin-3-carbonsäurebutylester-5-carbonsäurecyclopropylamid

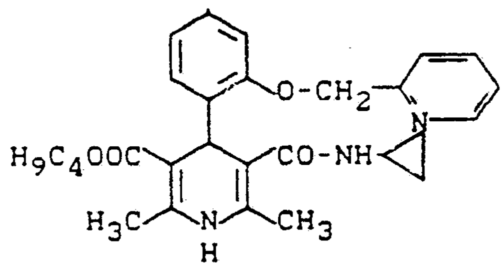

Schaum
$R_f$ = 0,1

Beispiel 25

1,4-Dihydro-2,6-dimethyl-4-[2-(2-pyridyl)methoxy-phenyl]-pyridin-3-carbonsäurebutylester-5-carbonsäuremethylamid

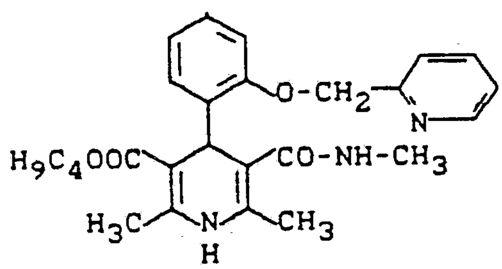

Schaum
$R_f$ = 0,07

Beispiel 26

1,4-Dihydro-2,6-dimethyl-4-[2-(2-pyridyl)methoxy-phenyl]-pyridin-3-carbonsäurebutylester-5-carbonsäure-(1-methylpropyl)-amid

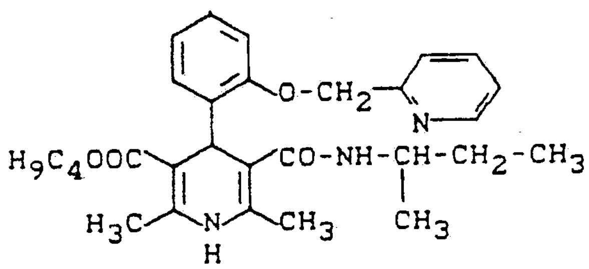

Schaum
$R_f$ = 0,25

Beispiel 27

4-[2-(3,4-Dichlor-benzyloxy)phenyl]-1,4-dihydro-2,6-dimethyl-pyridin-3-carbonsäuremethylester-5-carbonsäure-cyclopropylamid

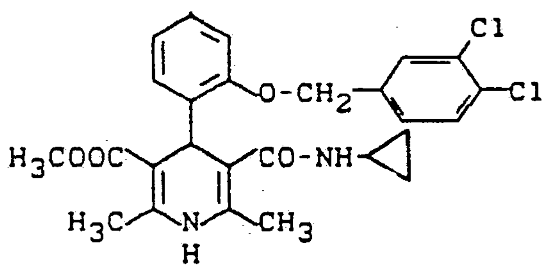

Schmp.: 195°C

Beispiel 28

4-[2-(2,6-Dichlor-benzyloxy)phenyl]-1,4-diydro-2,6-dimethyl-pyridin-3-carbonsäuremethylester-5-carbonsäure-cyclopropylamid

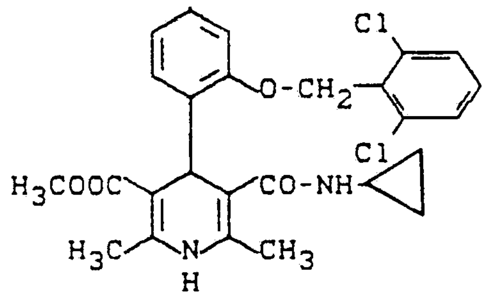

Schmp.: 178° C

Beispiel 29

4-[2-(2,6-Dichlor-benzyloxy)phenyl]-1,4-dihydro-2,6-dimethyl-pyridin-3-carbonsäuremethylester-5-carbonsäure-(1-methyl-propyl)amid

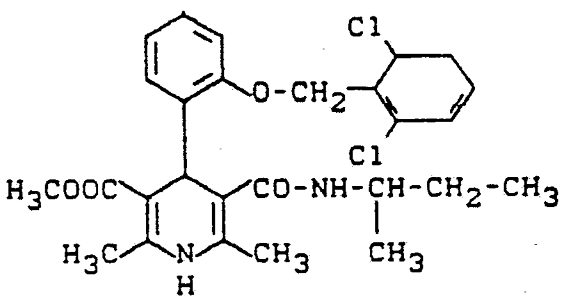

Schaum
$R_f$ = 0,36

Beispiel 30

4-[2-(2,6-Dichlor-benzyloxy)phenyl]-1,4-dihydro-2,6-dimethyl-pyridin-3-carbonsäuremethylester-5-carbonsäuremethylamid

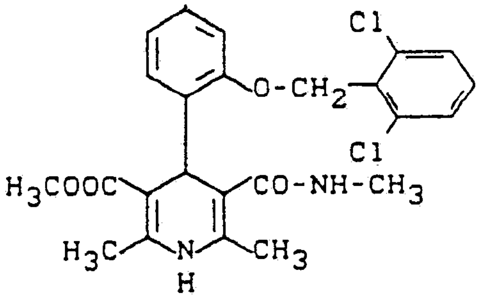

Schmp.: 133° C

Beispiel 31

4-[2-(3,4-Dichlor-benzyloxy)phenyl]-1,4-dihydro-2,6-dimethyl-pyridin-3-carbonsäuremethylester-5-carbonsäuremethylamid

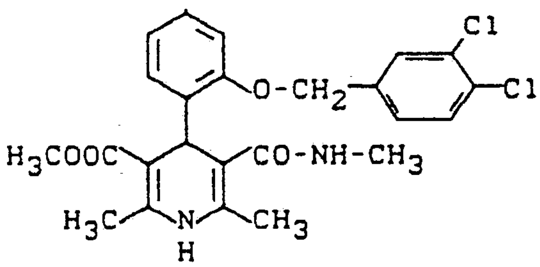

Schmp.: 120° C

Beispiel 32

4-[2-(3-Fluor-benzyloxy)-3-methoxy-phenyl]-1,4-dihydro-2,6-dimethyl-pyridin-3-carbonsäuremethylester-5-carbonsäurecyclopropylamid

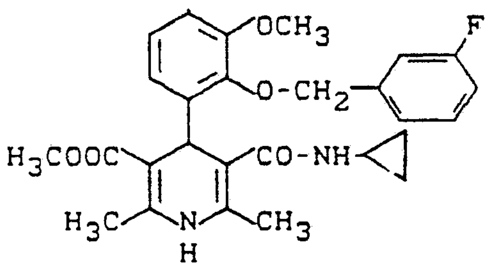

Schmp.: 202° C

Beispiel 33

4-[2-(2-Chlor-benzyloxy)phenyl]-1,4-dihydro-2,6-dimethyl-pyridin-3-carbonsäuremethylester-5-carbonsäurecyclopropylamid

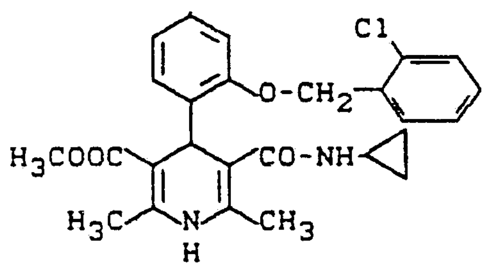

Schmp.: 185° C

Beispiel 34

4-[2-(2-Chlor-benzyloxy)phenyl]-1,4-dihydro-2,6-dimethyl-pyridin-3-carbonsäuremethylester-5-carbonsäuremethylamid

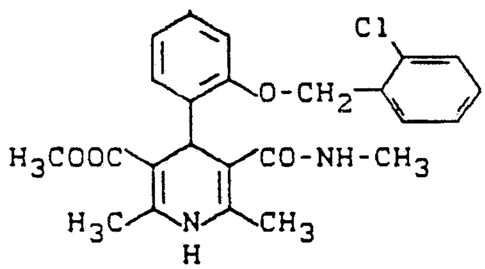

Schmp.: 170° C

Beispiel 35

4-[2-(3-Fluor-benzyloxy)-3-methoxy-phenyl]-1,4-dihydro 2,6-dimethyl-pyridin-3-carbonsäuremethylester-5-carbonsäure-(2-methyl-propyl)amid

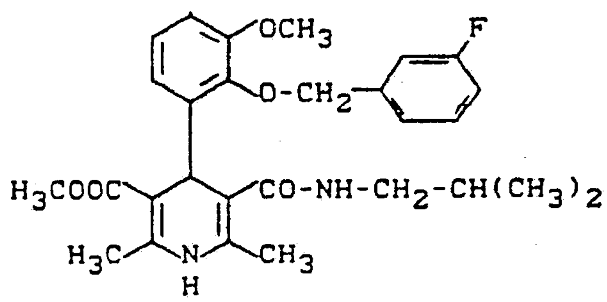

Schmp.: 98° C

Beispiel 36

1,4-Dihydro-2,6-dimethyl-4-[2-(3-trifluormethyl-benzyloxy)phenyl]-pyridin-3-carbonsäuremethylester-5-carbonsäurecyclopropylamid

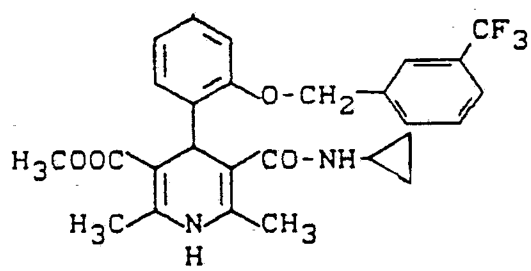

Schmp.: 148° C

Beispiel 37

1,4-Dihydro-2,6-dimethyl-4-[2-(3-trifluormethyl-benzyloxy)phenyl]-pyridin-3-carbonsäuremethylester-5-carbonsäure-(1-methyl-propyl)amid

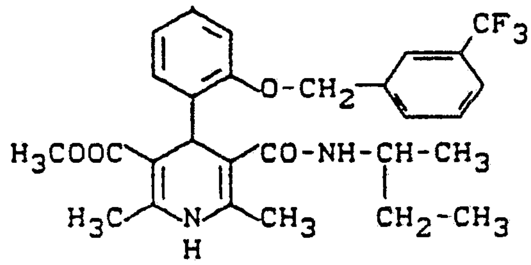

Schaum
$R_f$ = 0,44

Beispiel 38

4-[2-(3-Fluor-benzyloxy)-3-methoxy-phenyl]-1,4-dihydro 2,6-dimethyl-pyridin-3-carbonsäuremethylester-5-carbonsäureisopropylamid

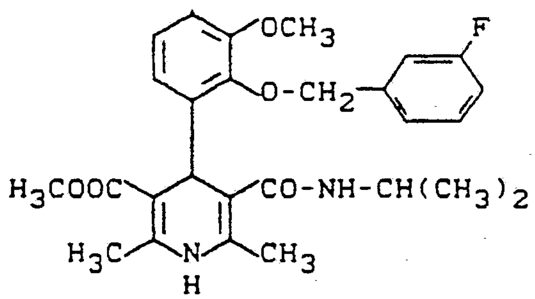

Schmp.: 106° C

Beispiel 39

1,4-Dihydro-2,6-dimethyl-4-[2-(4-methyl-benzyloxy)phenyl]-pyridin-3-carbonsäure-(1-methyl-propyl)ester-5-carbonsäurecyclopropylamid

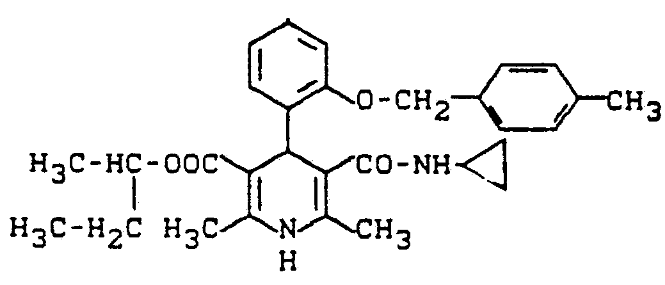

Schaum

$R_f$ = 0,31

Beispiel 40

4-(4-Benzyloxy-phenyl)-1,4-dihydro-2,6-dimethyl-pyridin-3-carbonsäuremethylester-5-carbonsäurecyclopropylamid

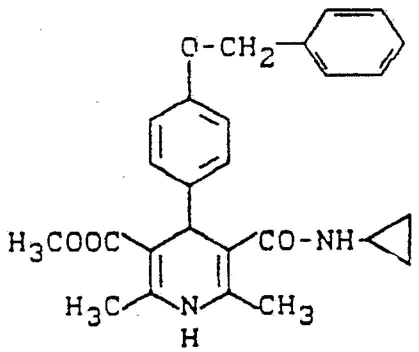

Schmp.: 197° C

Beispiel 41

1,4-Dihydro-2,6-dimethyl-4-[2-(4-methyl-benzyloxy)phenyl]-pyridin-3-carbonsäure-(1-methyl-propyl)ester-5-carbonsäure-(1-methyl-propyl)amid

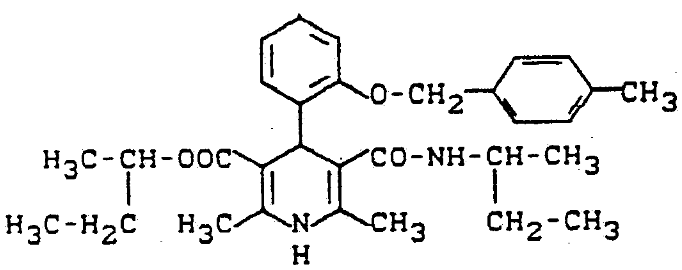

Schmp.: 126° C

Beispiel 42

1,4-Dihydro-2,6-dimethyl-4-[2-(4-methyl-benzyloxy)phenyl]-pyridin-3-carbonsäure-(1-methyl-propyl)ester-5-carbonsäuremethylamid

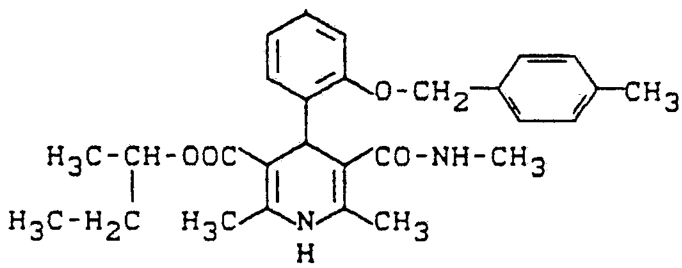

Schaum
$R_f$ = 0,48

Beispiel 43

1,4-Dihydro-2,6-dimethyl-4-[2-(3-nitro-benzyloxy)phenyl]-pyridin-3-carbonsäureethylester-5-carbonsäurecyclopropylamid

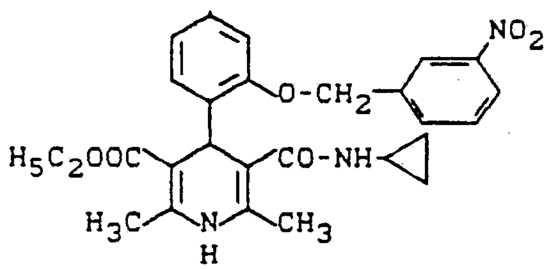

Schaum
$R_f$ = 0,24

Beispiel 44

4-(4-Benzyloxy-phenyl)-1,4-dihydro-2,6-dimethyl-pyridin-3-carbonsäuremethylester-5-carbonsäurepropylamid

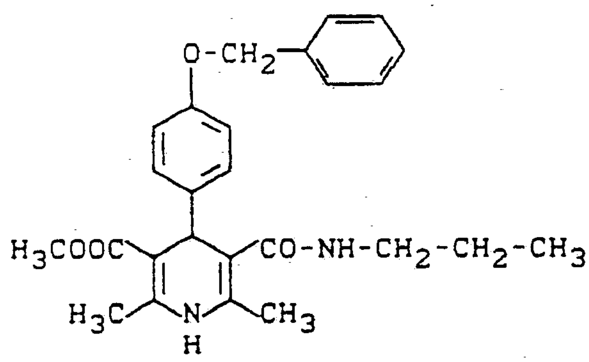

Schmp.: 210 °C

Beispiel 45

(R)-4-[2-(4-Fluor-benzyloxy)phenyl]-1,4-Dihydro-2,6-dimethyl-pyridin-3-carbonsäureethylester-5-carbonsäure-(1-phenyl-ethyl)amid (R-Form)

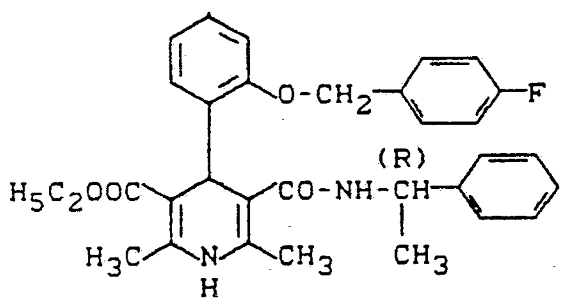

Schaum
$R_f$ = 0,56

Beispiel 46

4-(4-Benzyloxy-phenyl)-1,4-dihydro-2,6-dimethyl-pyridin-3-carbonsäuremethylester-5-carbonsäure-(2-methyl-propyl)-amid

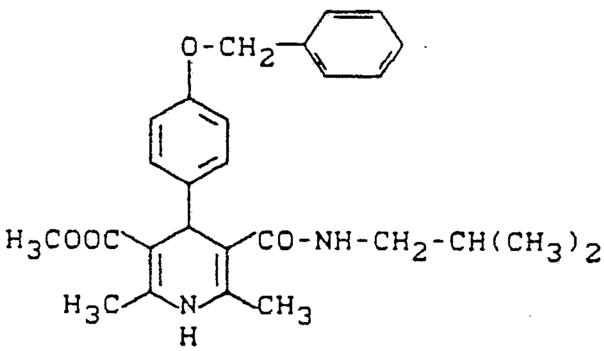

Schmp.: 202° C

Beispiel 47

4-[2-(4-Fluor-benzyloxy)phenyl]-1,4-dihydro-2,6-dimethyl-pyridin-3-carbonsäureethylester-5-carbonsäurecyclopropyl-amid

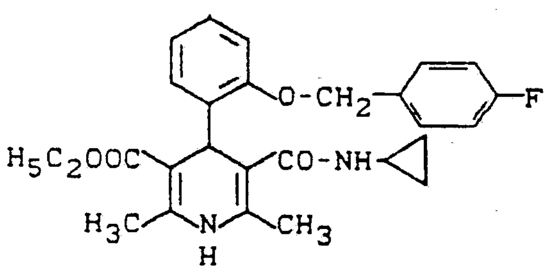

Schmp.: 159° C

Beispiel 48

4-[2-(4-Fluor-benzyloxy)phenyl]-1,4-dihydro-2,6-dimethyl-pyridin-3-carbonsäuremethylester-5-carbonsäurecyclopropylamid

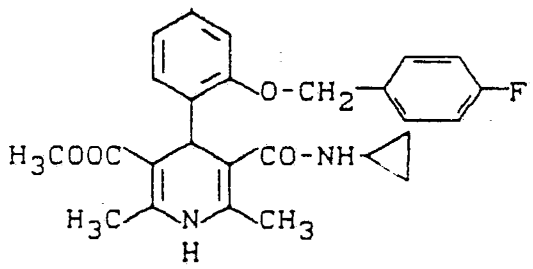

Schmp.: 154° c

Beispiel 49

(S)-4-(4-Benzyloxy-phenyl)-1,4-dihydro-2,6-dimethyl-pyridin-3-carbonsäuremethylester-5-carbonsäure-(1-phenylethyl)amid (S-Form)

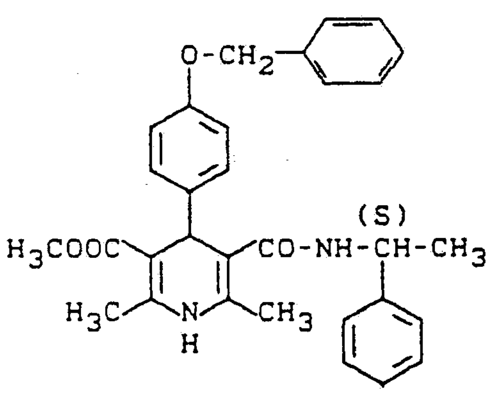

Schmp.: 141° C

Beispiel 50

4-(4-Benzyloxy-phenyl)-1,4-dihydro-2,6-dimethyl-pyridin-3-carbonsäuremethylester-5-carbonsäureisopropylamid

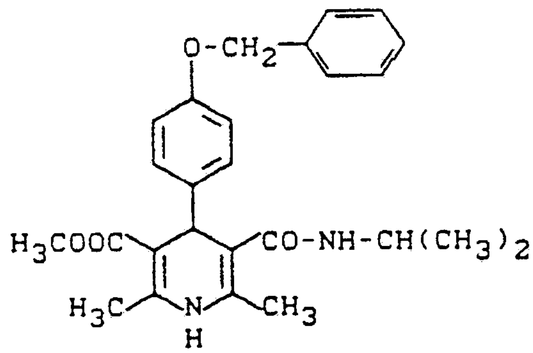

Schmp.: 162° C

Beispiel 51

(R)-4-(4-Benzyloxy-phenyl)-1,4-dihydro-2,6-dimethyl-pyridin-3-carbonsäuremethylester-5-carbonsäure-(1-phenyl-ethyl)amid (R-Form, Diastereomeres A)

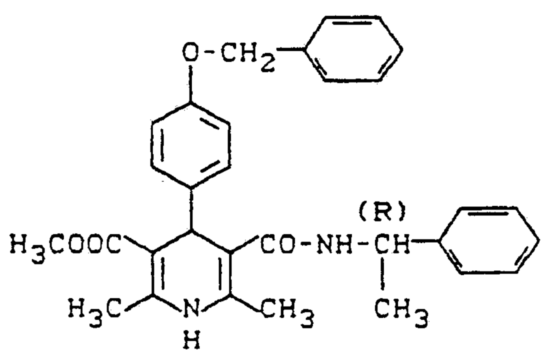

Schmp.: 196° C

Beispiel 52

4-[2-(3-Chlor-benzyloxy)phenyl]-1,4-dihydro-2,6-dimethyl-pyridin-3-carbonsäuremethylester-5-carbonsäurecyclopropyl-amid

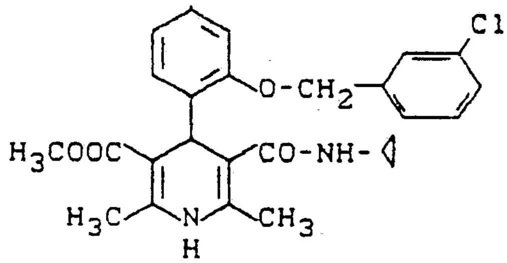

Schmp.: 174° C

Beispiel 53

4-[2-(4-Fluor-benzylthio)phenyl]-1,4-dihydro-2,6-dimethyl-pyridin-3-carbonsäuremethylester-5-carbonsäurecyclopropyl-amid

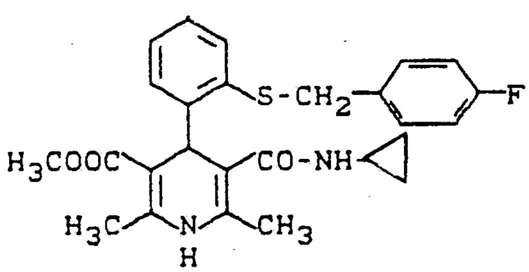

Schmp.: 202° C

Beispiel 54

(R)-4-(4-Benzyloxy-phenyl)-1,4-dihydro-2,6-dimethyl-pyridin-3-carbonsäuremethylester-5-carbonsäure-(1-phenyl-ethyl)amid (R-Form) (Diastereomeres B)

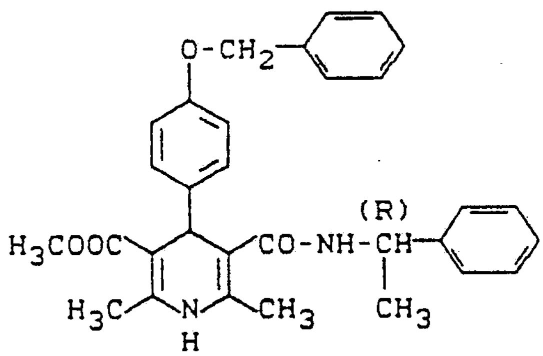

Schmp.: 202° C

Beispiel 55

(R)-4-(4-Benzyloxy-phenyl)-1,4-dihydro-2,6-dimethyl-pyridin-3-carbonsäuremethylester-5-carbonsäure-(1-phenyl-ethyl)amid (R-Form, Diastereomerengemisch)

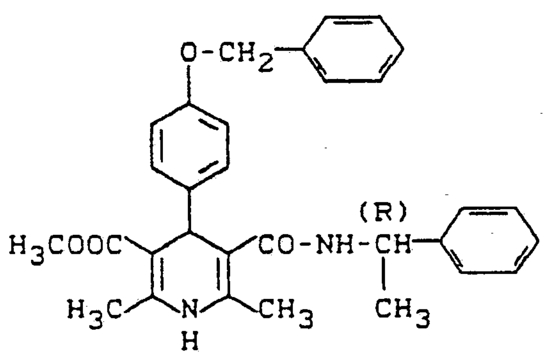

Schmp.: 110 - 166° C

Beispiel 56

(S)-4-(2-Benzyloxy-phenyl)-1,4-dihydro-2,6-dimethyl-pyridin-3-carbonsäuremethylester-5-carbonsäure-(1-phenyl-ethyl)amid (S-Form, Diasteromer A)

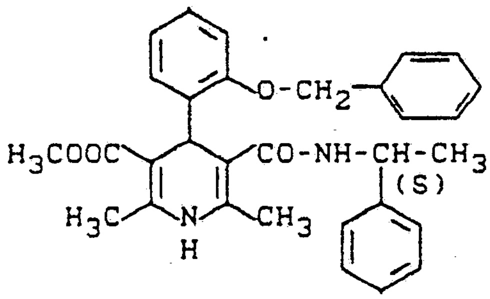

Schmp.: 172 °C

Beispiel 57

(S)-4-(2-Benzyloxy-phenyl)-1,4-dihydro-2,6-dimethyl-pyridin-3-carbonsäuremethylester-5-carbonsäure-(1-phenyl-ethyl)amid (S-Form, Diasteromer B)

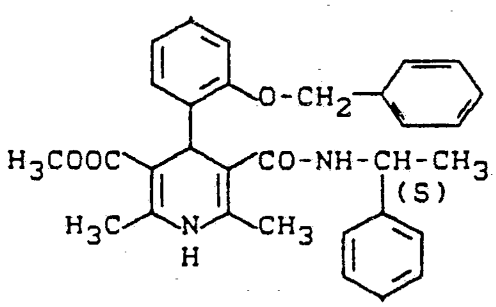

Schaum
$R_f$ = 0,53

Beispiel 58

(R)-4-[2-(4-Fluor-benzyloxy)phenyl]-1,4-dihydro-2,6-dimethyl-pyridin-3-carbonsäuremethylester-5-carbonsäure-(1-phenyl-ethyl)amid (R-Form)

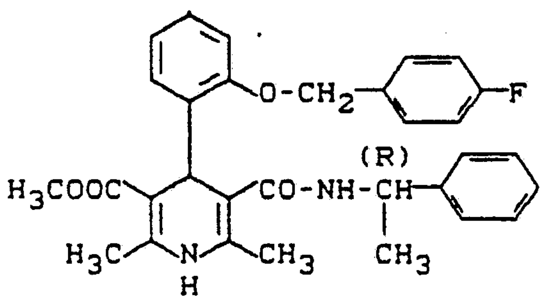

Schaum
$R_f$ = 0,52

Beispiel 59

1,4-Dihydro-2,6-dimethyl-4-[2-(3-methyl-benzyloxy)phenyl]-pyridin-3-carbonsäurebutylester-5-carbonsäurecyclopropylamid

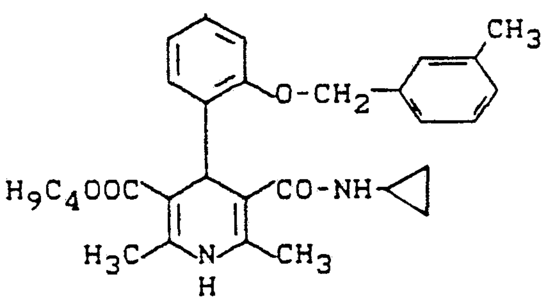

Schaum
$R_f$ = 0,31

Beispiel 60

(R)-1,4-Dihydro-2,6-dimethyl-4-[2-(3-methyl-benzyloxy)-phenyl]-pyridin-3-carbonsäurebutylester-5-carbonsäure-(1-phenyl-ethyl)amid (R-Form)

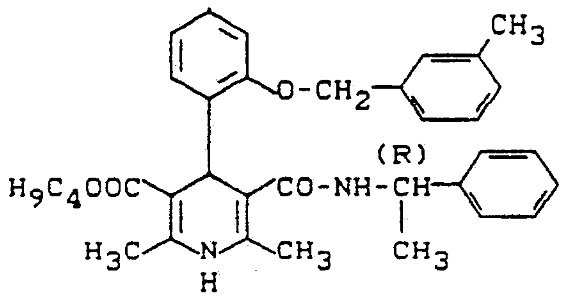

Öl
$R_f$ = 0,88

Beispiel 61

(R)-4-[2-(3-Chlor-benzyloxy)phenyl]-1,4-dihydro-2,6-dimethyl-pyridin-3-carbonsäuremethylester-5-carbonsäure-(1-phenyl-ethyl)amid (R-Form)

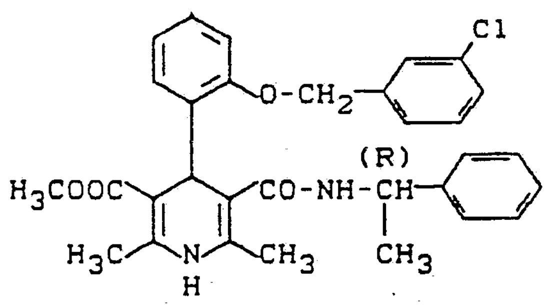

Schaum
$R_f$ = 0,73

Beispiel 62

(R)-4-(3-Benzyloxy-phenyl)-1,4-dihydro-2,6-dimethyl-pyridin-3-carbonsäuremethylester-5-carbonsäure-(1-phenyl-ethyl)amid (R-Form)

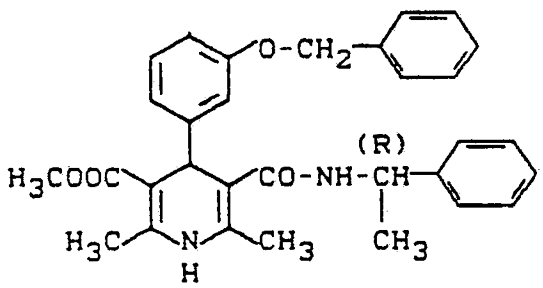

Schmp.: ab 143° C

Beispiel 63

4-(3-Benzyloxy-phenyl)-1,4-dihydro-2,6-dimethyl-pyridin-3-carbonsäuremethylester-5-carbonsäurecyclopropylamid

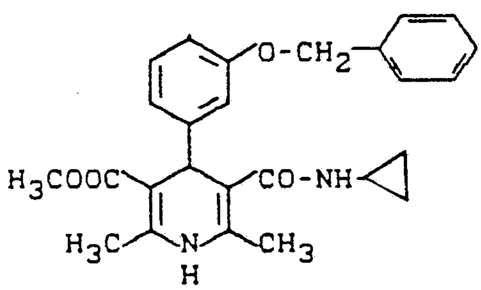

Schmp.: 144° C

Beispiel 64

(R)-4-[2-(4-Fluor-benzylthio)phenyl]-1,4-dihydro-2,6-dimethyl-pyridin-3-carbonsäuremethylester-5-carbonsäure-(1-phenyl-ethyl)amid (R-Form)

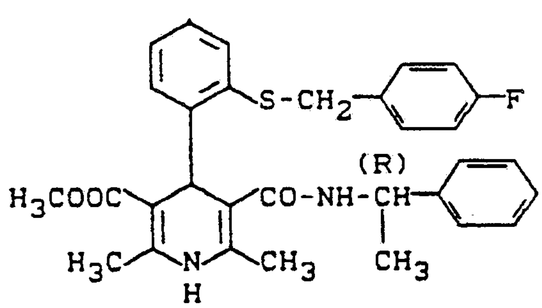

Schaum
$R_f$ = 0,47

Beispiel 65

(S)-4-[2-(4-Fluor-benzylthio)phenyl]-1,4-dihydro-2,6-dimethyl-pyridin-3-carbonsäuremethylester-5-carbonsäure-(1-phenyl-ethyl)amid (S-Form)

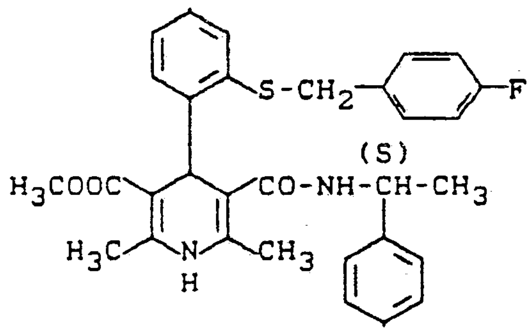

Schaum
$R_f$ = 0,48

Beispiel 66

4-[2-(4-Fluor-benzylthio)phenyl]-1,4-dihydro-2,6-dimethyl-pyridin-3-carbonsäuremethylester-5-carbonsäureallylamid

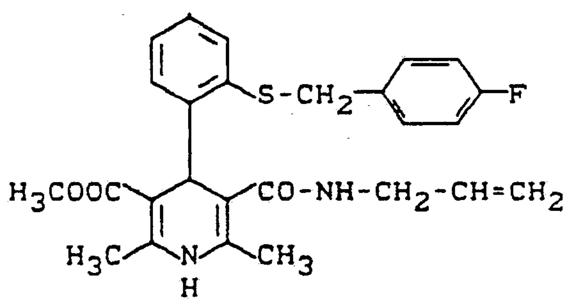

Schmp.: 177 °C

Beispiel 67

4-(2-Benzyloxy-phenyl)-1,4-dihydro-2,6-dimethyl-pyridin-3-carbonsäuremethylester-5-carbonsäureallylamid

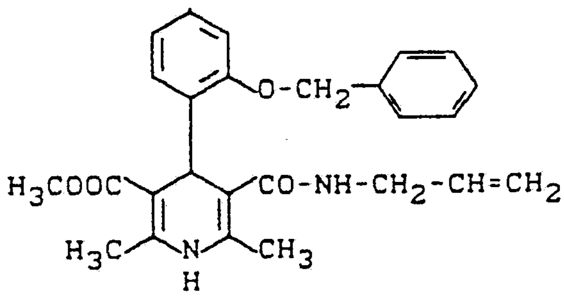

Schmp.: 148° C

Beispiel 68

(R)-4-(2-Benzyloxy-phenyl)-1,4-dihydro-2,6-dimethyl-pyridin-3-carbonsäuremethylester-5-carbonsäure-(1-phenyl-ethyl)amid (R-Form)

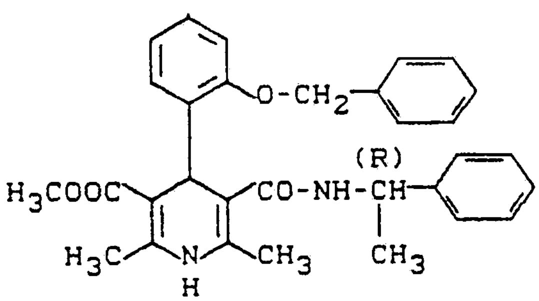

Schmp.: 175° C

Beispiel 69

(R)-4-(2-Benzyloxy-phenyl)-1,4-dihydro-2,6-dimethyl-pyridin-3-carbonsäuremethylester-5-carbonsäure-(1-phenyl-ethyl)amid (R-Form, Diastereomer B)

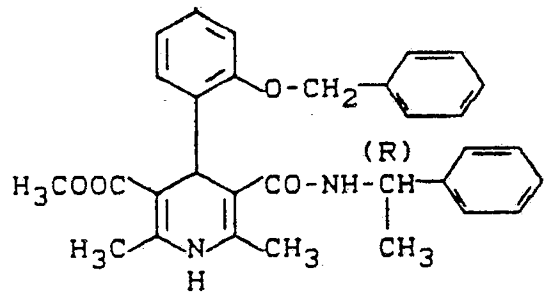

Schmp.: 169° C

Beispiel 70

4-(2-Benzyloxy-phenyl)-1,4-dihydro-2,6-dimethyl-pyridin-3-carbonsäuremethylester-5-carbonsäure-(2-phenyl-ethyl)amid

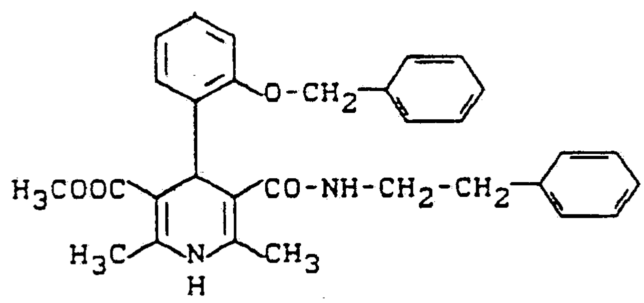

Öl

Beispiel 71

4-(2-Benzyloxy-phenyl)-1,4-dihydro-2,6-dimethyl-pyridin-3-carbonsäuremethylester-5-carbonsäurebenzylamid

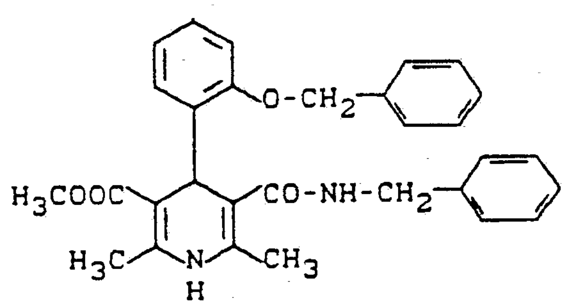

Schmp.: 148 - 149 °C

Beispiel 72

4-[2-(4-Fluor-benzylthio)phenyl]-1,4-dihydro-2,6-dimethyl-pyridin-3-carbonsäuremethylester-5-carbonsäureethylamid

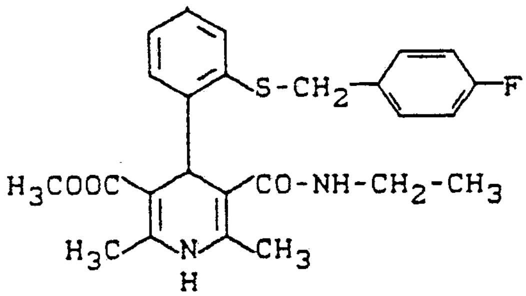

Schmp.: 202 - 204 °C

Beispiel 73

4-(3-Benzyloxy-phenyl)-1,4-dihydro-2,6-dimethyl-pyridin-3-carbonsäuremethylester-5-carbonsäureethylamid

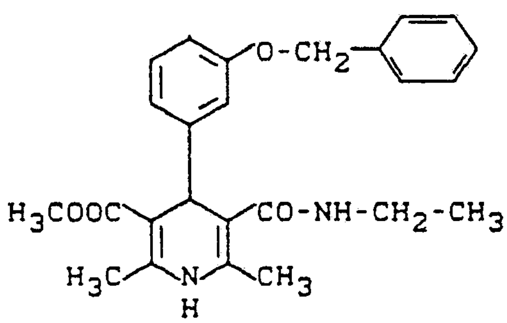

Schmp.: 152 - 154 °C

Beispiel 74

4-(4-Benzyloxy-phenyl)-1,4-dihydro-2,6-dimethyl-pyridin-3-carbonsäuremethylester-5-carbonsäureethylamid

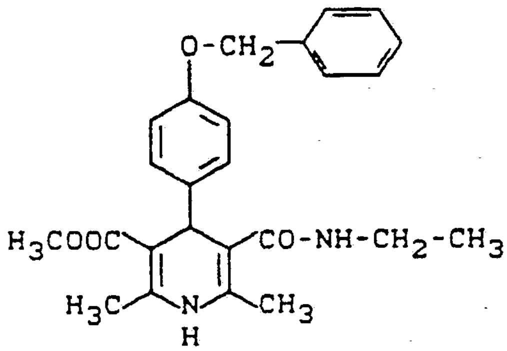

Schmp.: 183 - 185° C

Beispiel 75

4-(2-Benzyloxy-phenyl)-1,4-dihydro-2,6-dimethyl-pyridin-3-carbonsäuremethylester-5-carbonsäureamid

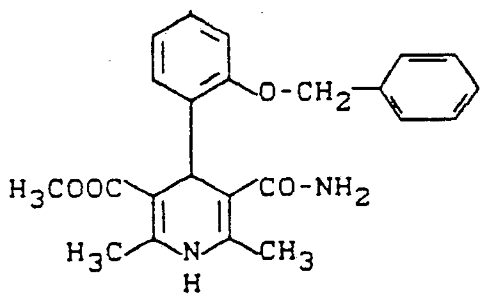

Schmp.: 188° C

Beispiel 76

4-(2-Benzyloxy-phenyl)-1,4-dihydro-2,6-dimethyl-pyridin-3-carbonsäuremethylester-5-carbonsäurediethylamid

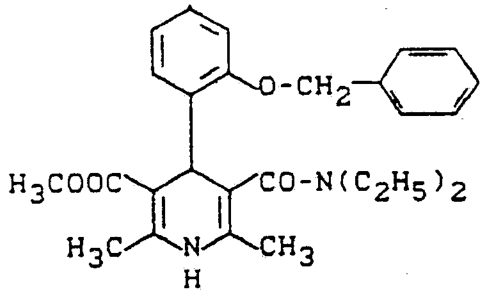

Schmp.: 135° C

Beispiel 77

1,4-Dihydro-2,6-dimethyl-4-[2-(4-methyl-phenylsulfonyl-oxy)phenyl]-pyridin-3-carbonsäuremethylester-5-carbonsäurecyclopropylamid

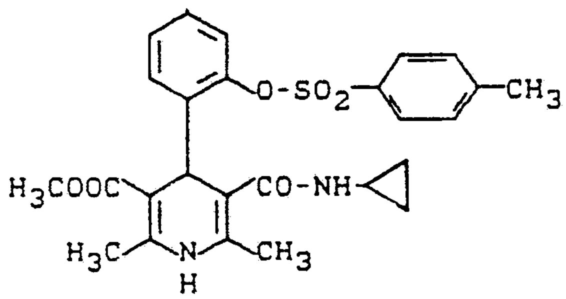

Schmp.: 238 - 242° C

Beispiel 78

1,4-Dihydro-2,6-dimethyl-4-[2-(4-methyl-phenylsulfonyl-oxy)phenyl]-pyridin-3-carbonsäuremethylester-5-carbonsäureethylamid

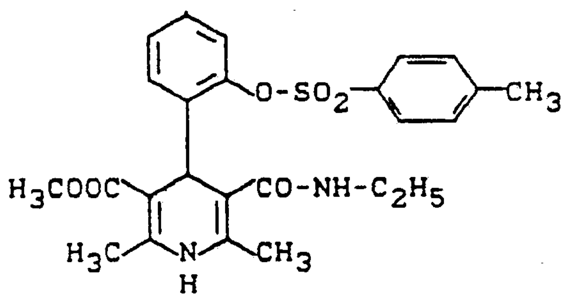

Schmp.: 228° C

Beispiel 79

4-[2-(2,6-Dichlor-benzyloxy)phenyl]-1,4-dihydro-2,6-dimethyl-pyridin-3-carbonsäuremethylester-5-carbonsäure-ethylamid

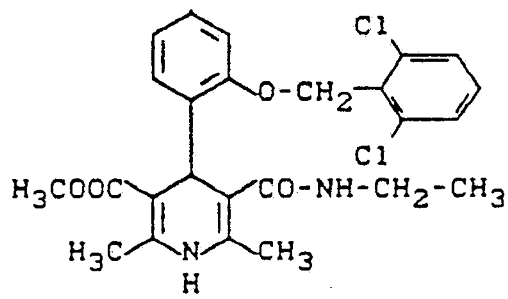

Schmp.: 174 - 176° C

Beispiel 80

4-[2-(3,4-Dichlor-benzyloxy)phenyl]-1,4-dihydro-2,6-dimethyl-pyridin-3-carbonsäuremethylester-5-carbonsäure-ethylamid

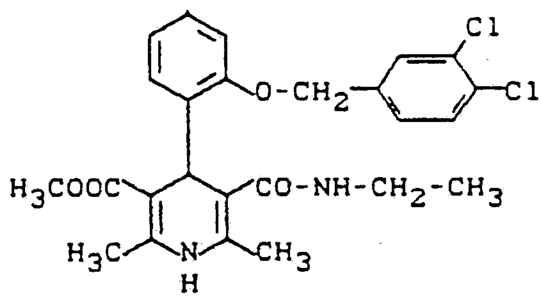

Schmp.: 178 - 180° C

Beispiel 81

1,4-Dihydro-2,6-dimethyl-4-[2-(2-pyridylmethyloxy)phenyl]-pyridin-3-carbonsäuremethylester-5-carbonsäureethylamid

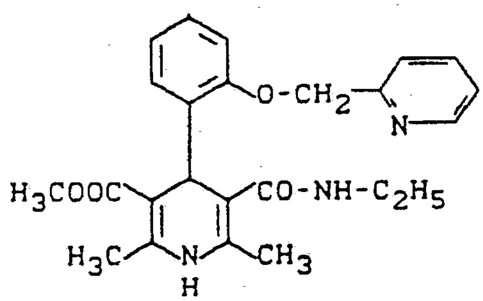

Schmp.: 159 - 161 °C

Beispiel 82

1,4-Dihydro-2,6-dimethyl-4-[2-(2-pyridylmethyloxy)phenyl]-pyridin-3-carbonsäuremethylester-5-carbonsäurecyclopropylamid

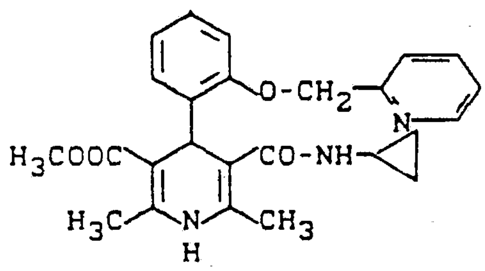

Schmp.: 168 - 170 °C

Beispiel 83

1,4-Dihydro-2,6-dimethyl-4-(2-phenylsulfonyloxy-phenyl)-pyridin-3-carbonsäuremethylester-5-carbonsäuremethylamid

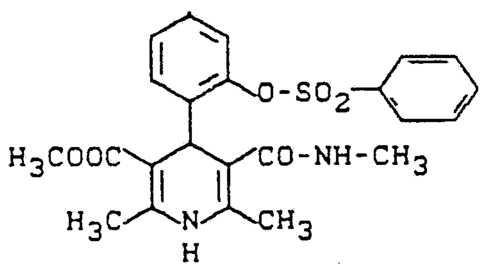

Schmp.: 171 - 173 °C

Beispiel 84

1,4-Dihydro-2,6-dimethyl-4-(2-phenylsulfonyloxy-phenyl)-pyridin-3-carbonsäuremethylester-5-carbonsäureethylamid

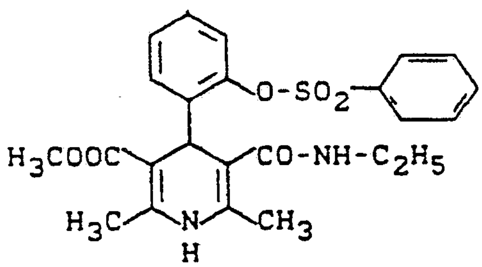

Schmp.: 188 - 190 °C

Beispiel 85

1,4-Dihydro-4-[2-(4-fluor-benzyloxy)phenyl]-2,6-dimethyl-pyridin-3-carbonsäureethylester-5-carbonsäureethylamid

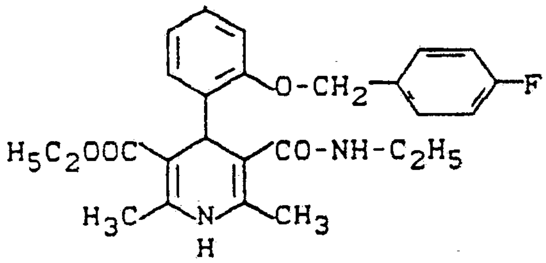

Schmp.: 147 - 149 °C

Beispiel 86

1,4-Dihydro-4-[2-(4-fluor-benzyloxy)phenyl]-2,6-dimethyl-pyridin-3-carbonsäuremethylester-5-carbonsäureethylamid

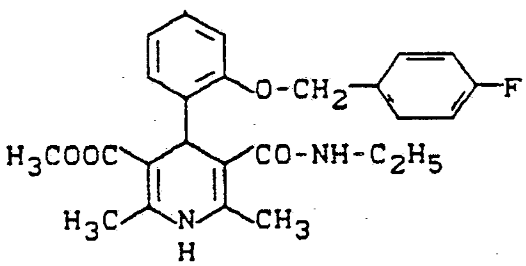

Schmp.: 111 - 113 °C

Beispiel 87

1,4-Dihydro-2,6-dimethyl-4-(2-phenylsulfonyloxy-phenyl)-pyridin-3-carbonsäuremethylester-5-carbonsäureallylamid

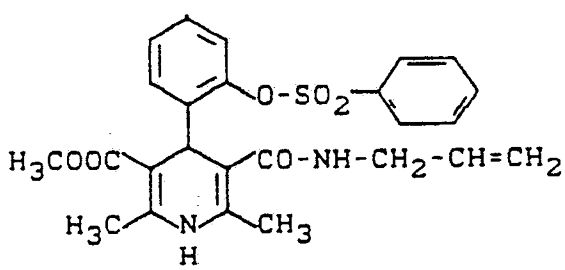

Schmp.: 154 - 156 °C

Beispiel 88

1,4-Dihydro-2,6-dimethyl-4-(2-phenylsulfonyloxy-phenyl)-pyridin-3-carbonsäuremethylester-5-carbonsäure-(4-pyridyl)amid

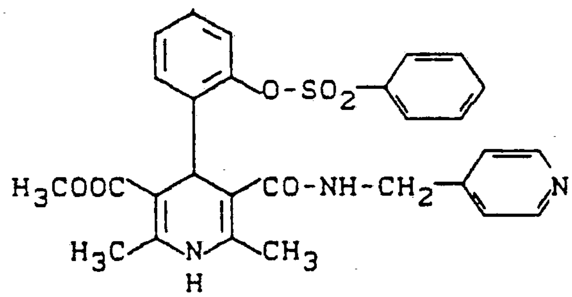

Schmp.: ab 240° C (Zersetzung)

Beispiel 89

4-[2-(4-Chlor-benzyloxy)phenyl]-1,4-dihydro-2,6-dimethyl-pyridin-3-carbonsäuremethylester-5-carbonsäureallylamid

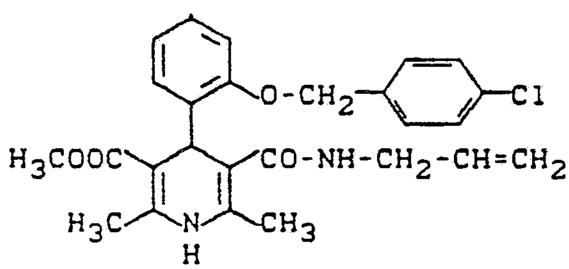

Schmp.: 145° C

Beispiel 90

4-(3-Benzyloxy-phenyl)-1,4-dihydro-2,6-dimethyl-pyridin-3-carbonsäuremethylester-5-carbonsäureallylamid

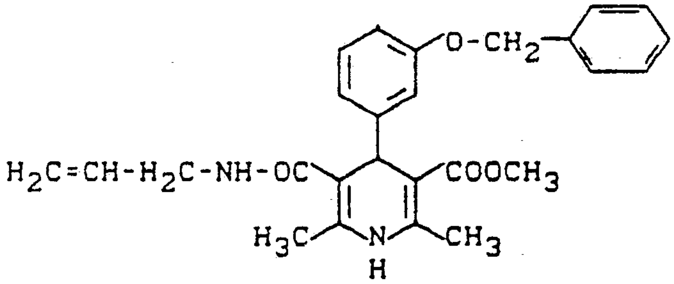

Schmp.: 95 - 98° C

Beispiel 91

1,4-Dihydro-2,6-dimethyl-4-[2-(4-methyl-phenylsulfonyl-oxy)phenyl]pyridin-3-carbonsäuremethylester-5-carbonsäureallylamid

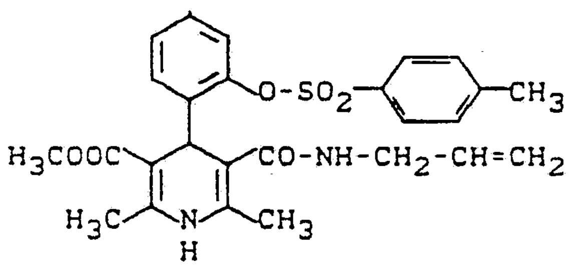

Schmp.: 168 - 170° C

Beispiel 92

4-[2-(2,6-Dichlor-benzyloxy)phenyl]-1,4-dihydro-2,6-dimethyl-pyridin-3-carbonsäuremethylester-5-carbonsäureallylamid

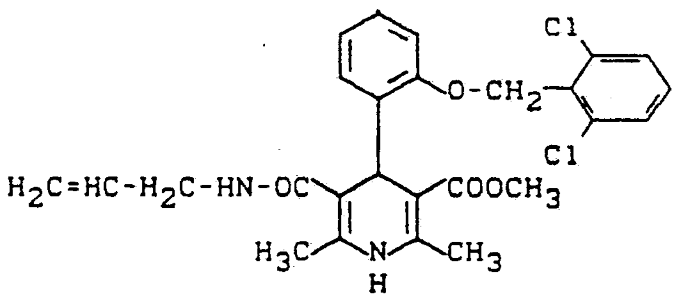

Schmp.: 132 - 134° C

Beispiel 93

4-(2-Benzyloxy-phenyl)-1,4-dihydro-2,6-dimethyl-pyridin-3-carbonsäuremethylester-5-carbonsäure-(2-hydroxyethyl)amid

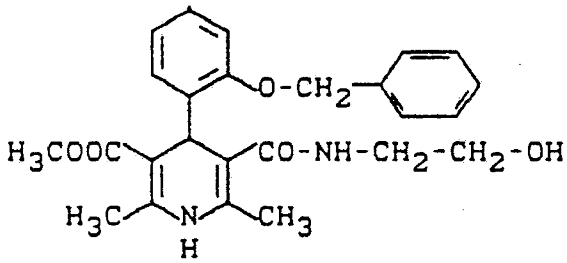

Schmp.: 148 - 150° C

Beispiel 94

4-[2-(3-Fluor-benzyloxy)-3-methoxy-phenyl]-1,4-dihydro-2,6-dimethyl-pyridin-3-carbonsäuremethylester-5-carbonsäuremethylamid

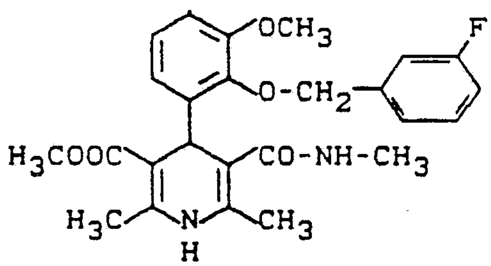

Schmp.: 171 - 173° C

Beispiel 95

4-(2-Benzyloxy-phenyl)-1,4-dihydro-2,6-dimethyl-pyridin-3-carbonsäuremethylester-5-carbonsäure-2-(4-pyridyl)ethylamid

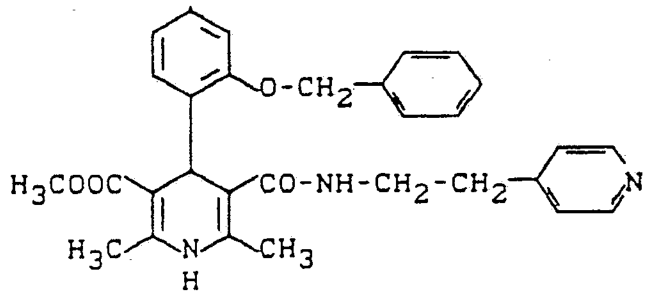

Schmp.:

Beispiel 96

1,4-Dihydro-2,6-dimethyl-4-[2-(3-pyridyl)methoxy-phenyl]-pyridin-3-carbonsäuremethylester-5-carbonsäuremethylamid

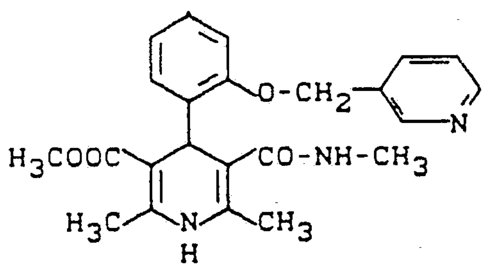

Schmp.: ab 210 °C

Beispiel 97

1,4-Dihydro-2,6-dimethyl-4-[2-(3-pyridyl)methoxy-phenyl]-pyridin-3-carbonsäuremethylester-5-carbonsäureethylamid

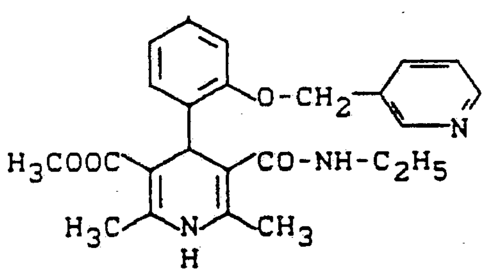

Schmp.: 122 - 125 °C

Beispiel 98

1,4-Dihydro-2,6-dimethyl-4-[2-(4-pyridyl)methoxy-phenyl]-pyridin-3-carbonsäuremethylester-5-carbonsäureethylamid

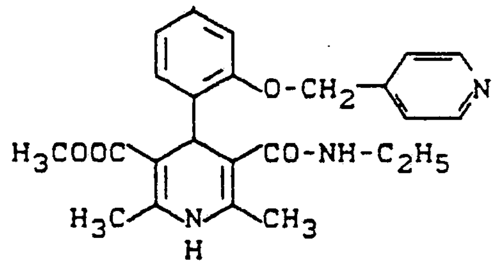

Schmp.: 95 - 100° C

Beispiel 99

1,4-Dihydro-2,6-dimethyl-4-[2-(4-pyridyl)methoxy-phenyl]-pyridin-3-carbonsäuremethylester-5-carbonsäuremethylamid

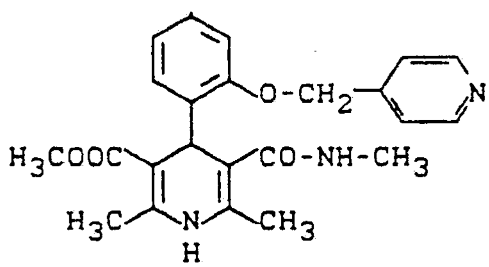

Schmp.: ab 203° c (Zersetzung)

Beispiel 100

4-(2-Benzyloxy-phenyl)-1,4-dihydro-2,6-dimethyl-pyridin-3-carbonsäuremethylester-5-carbonsäure-(cyclopropylmethyl)-amid

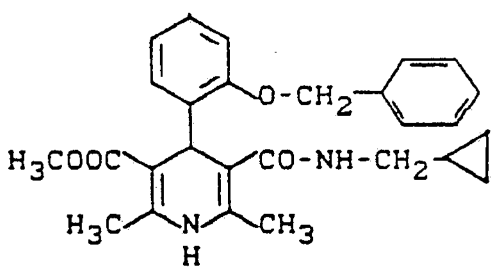

Schmp.: 186° C

Beispiel 101

(+)-4-(2-Benzyloxy-phenyl)-1,4-dihydro-2,6-dimethyl-pyridin-3-carbonsäuremethylester-5-carbonsäureethylamid

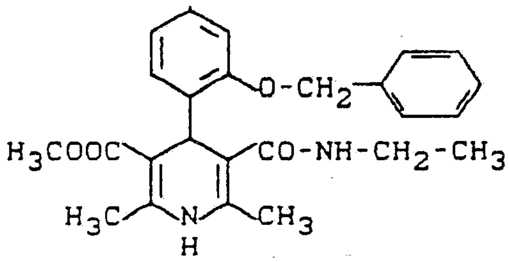

Schmp.: 147° C
$[\alpha]_D^{20}$ = + 29,68
c = 0,91 (DMF)

Beispiel 102

(-)-4-(2-Benzyloxy-phenyl)-1,4-dihydro-2,6-dimethyl-pyridin-3-carbonsäuremethylester-5-carbonsäureethylamid

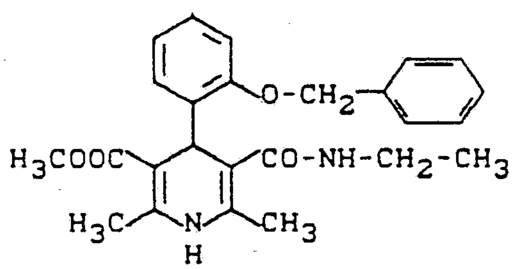

Schmp.: 148° C
$[\alpha]_D^{20}$ = - 29,92
c = 0,805 (DMF)

Beispiel 103

4-(2-Benzyloxy-phenyl)-1,4-dihydro-2,6-dimethyl-pyridin-3-carbonsäureethylester-5-carbonsäuremethylamid

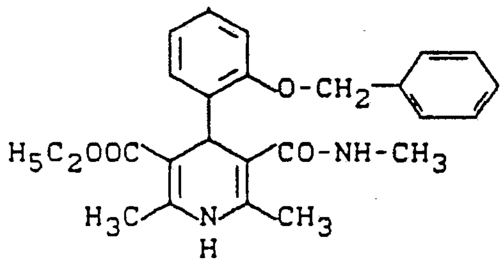

Schmp.: 179 °C

Beispiel 104

4-(2-Benzyloxy-phenyl)-1,4-dihydro-2,6-dimethyl-pyridin-3-carbonsäureethylester-5-carbonsäureethylamid

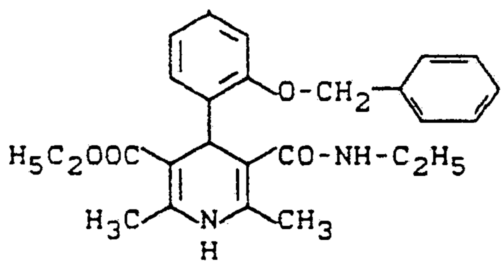

Schmp.: 161 - 164 °C

Beispiel 105

4-(2-Benzyloxy-phenyl)-1,4-dihydro-2,6-dimethyl-pyridin-3-carbonsäuremethylester-5-carbonsäure-(2-ethoxycarbonyl-ethyl)amid

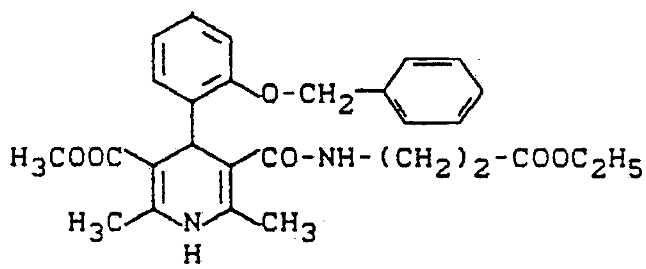

$R_f$ = 0,35

Beispiel 106

4-(2-Benzyloxy-phenyl)-1,4-dihydro-2,6-dimethyl-pyridin-3-carbonsäuremethylester-5-carbonsäure-(ethoxycarbonylmethyl)amid

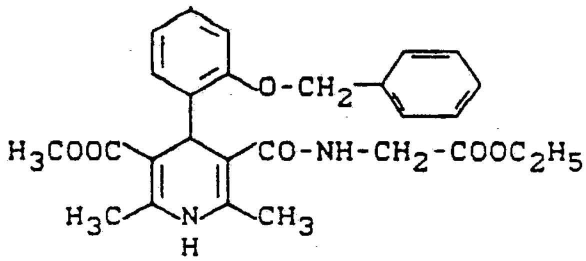

$R_f$ = 0,408

Beispiel 107

4-(2-Benzyloxy-phenyl)-1,4-dihydro-2,6-dimethyl-pyridin-3-carbonsäuremethylester-5-carbonsäureoctylamid

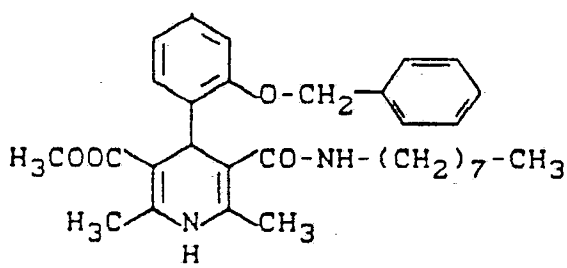

$R_f$ = 0,53

Beispiel 108

4-(2-Benzyloxy-phenyl)-1,4-dihydro-2,6-dimethyl-pyridin-3-carbonsäuremethylester-5-carbonsäurenonylamid

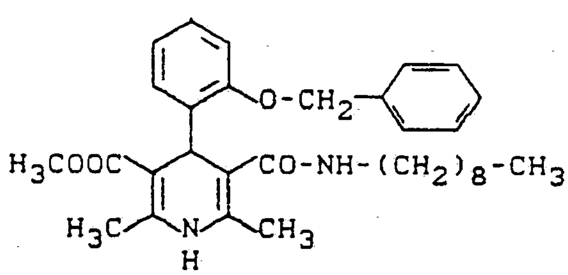

$R_f$ = 0,55

Beispiel 109

4-(2-Benzyloxy-phenyl)-1,4-dihydro-2,6-dimethyl-pyridin-3-carbonsäuremethylester-5-carbonsäuredecylamid

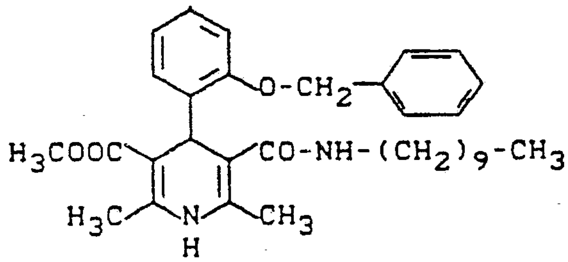

Schmp.: 111 °C

Beispiel 110

4-(2-Benzyloxy-phenyl)-1,4-dihydro-2,6-dimethyl-pyridin-3-carbonsäuremethylester-5-carbonsäure-(2-methoxy-ethyl)-amid

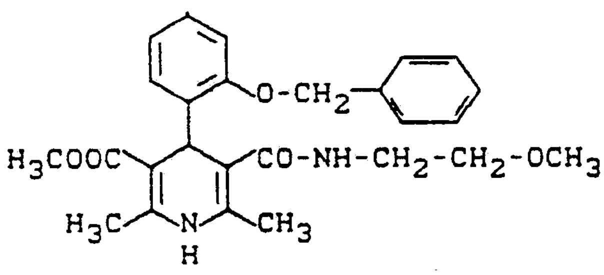

Schmp.: 145 °C

Beispiel 111

4-(2-Benzyloxy-phenyl)-1,4-dihydro-2,6-dimethyl-pyridin-3-carbonsäuremethylester-5-carbonsäure-(3-methoxy-propyl)-amid

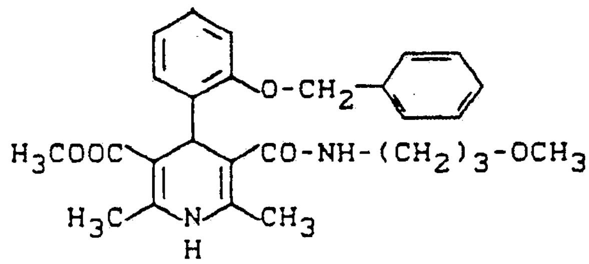

$R_f$ = 0,19

Beispiel 112

(R,R)-4-(2-Benzyloxy-phenyl)-1,4-dihydro-2,6-dimethyl-pyridin-3-carbonsäuremethylester-5-carbonsäure-(2-hydroxy-1-methyl-2-phenyl)ethylamid

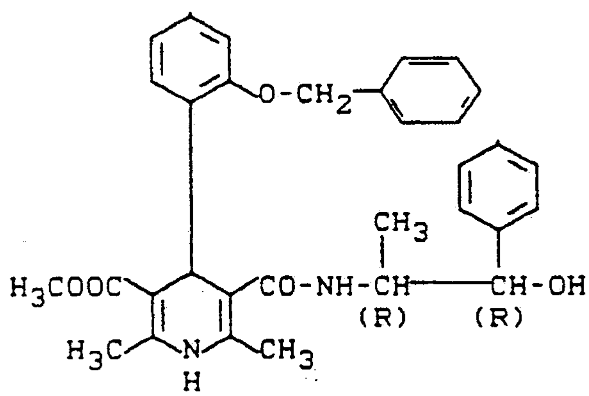

Verfahrensvariante H (Direkte Kupplung mit Dicyclohexyl-carbodiimid

1 g (2,54 mmol) 4-(2-Benzyloxy-phenyl)-1,4-dihydro-2,6-dimethyl-pyridin-3,5-dicarbonsäure-monomethylester werden in 5 ml Dimethylformamid gelöst und 0,477 g (2,54 mmol) L-Norpseudoephedrin Hydrochlorid, 0,35 ml (2,54 mmol) Triethylamin und 0,629 g (3,05 mmol) Dicylohexyl-carbodiimid (fest) dazugegeben. Die Mischung wird 4 Stunden bei Raumtemperatur gerührt, der Harnstoff abgesaugt und das Filtrat eingeengt.

Säulenchromatographie: Kieselgel 60, Korngröße 0,040 - 0,063 mm mit $CHCl_3$ /$CH_3OH$ / $NH_3$ 20 : 1 : 0,05

Da der Harnstoff schwerlöslich in Ether und Methylenchlorid ist, wird das Produkt mehrmals in Ether oder Methylenchlorid aufgenommen, abfiltriert und eingeengt.

Ausbeute: 0,9 g (67,3% der Theorie)

$R_f$ = 0,35

$\alpha_D^{20}$ : -47,93 (in $CHCl_3$)

Beispiel 113

(S)-4-(2-Benzyloxy-phenyl)-1,4-dihydro-2,6-dimethyl-pyridin-3-carbonsäuremethylester-5-carbonsäure-(2-hydroxy-1-methyl-ethyl)amid

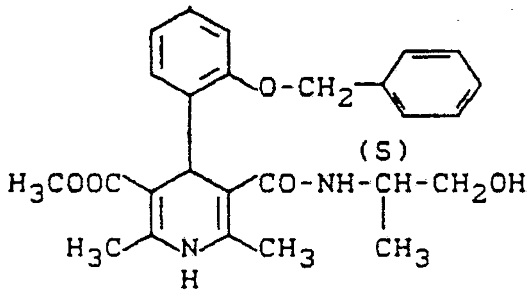

$R_f$ = 0,13
$\alpha_D^{20}$ : +7,34 ($CHCl_3$)

Beispiel 114

(S)-4-(2-Benzylxoy-phenyl)-1,4-dihydro-2,6-dimethyl-pyridin-3-carbonsäuremethylester-5-carbonsäure-(1-hydroxy-methyl-propyl)amid

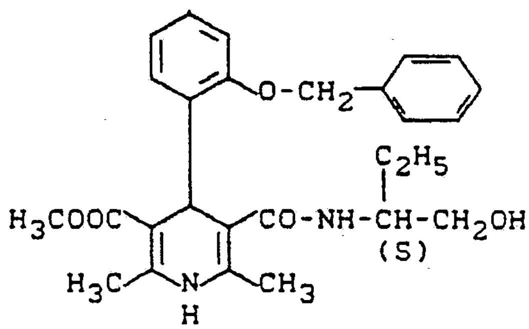

$R_f$ = 0,15
$\alpha_D^{20}$ : -9,37 ($CHCl_3$)

Beispiel 115

(S)-4-(2-Benzyloxy-phenyl)-1,4-dihydro-2,6-dimethyl-pyridin-3-carbonsäuremethylester-5-carbonsäure-(1-hydroxymethyl-2-methyl-propyl)amid

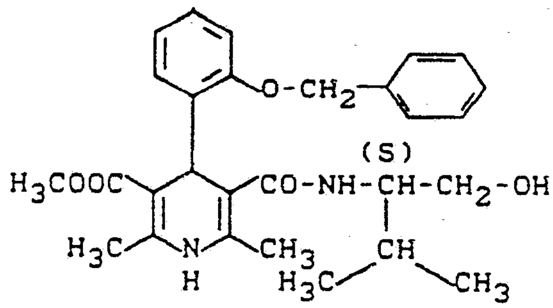

$R_f$ = 0,19
$\alpha_D^{20}$ : -10,8 ($CHCl_3$)

Beispiel 116

(S)-4-(2-Benzyloxy-phenyl)-1,4-dihydro-2,6-dimethyl-pyridin-3-carbonsäuremethyl-5-carbonsäure-(1-hydroxymethyl-2-methyl-butyl)amid

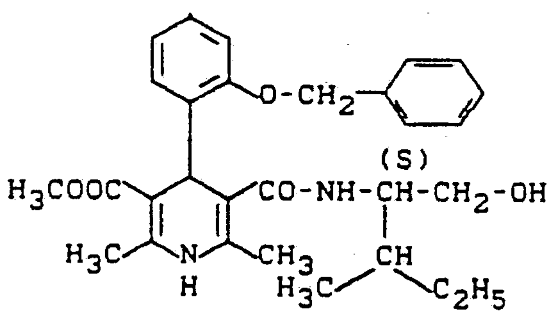

$R_f$ = 0,21
$\alpha_D^{20}$ : -17,37 ($CHCl_3$)

Beispiel 117

4-(2-Benzyloxy-phenyl)-1,4-dihydro-2,6-dimethyl-pyridin-3-carbonsäuremethylester-5-carbonsäure-(3-hydroxy-propyl)amid

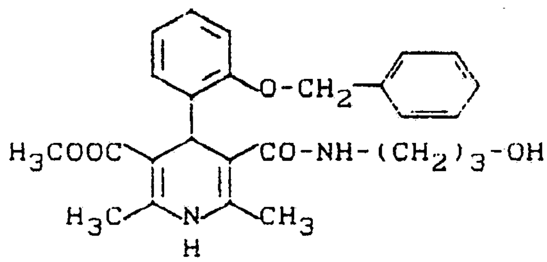

Schmp.: 205 °C

Beispiel 118

(-) 4-(2-Benzyloxy-phenyl)-1,4-dihydro-2,6-dimethyl-pyridin-3-carbonsäuremethylester-5-carbonäure-3-cyclopropylamid

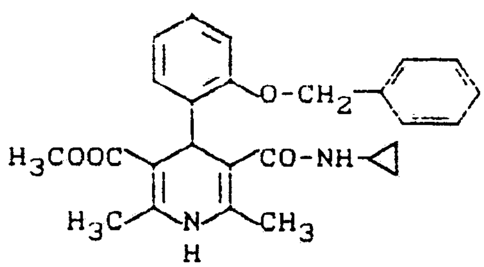

Schmp.: 178 - 181 °c

$$[\alpha]^{20}_{589} = -38{,}28 \ (c=0{,}569,\ \text{Chloroform})$$

Beispiel 119

(+) 4-(2-Benzyloxy-phenyl)-1,4-dihydro-2,6-dimethyl-pyridin-3-carbonsäuremethylester-5-carbonäure-3-cyclopropylamid

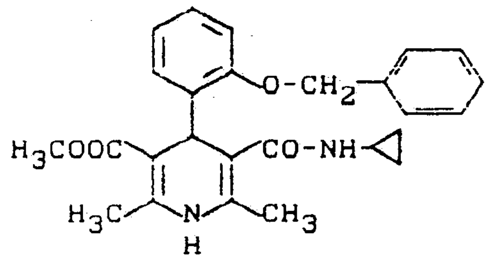

Schmp.: 178 - 181 °C

$[\alpha]^{20}_{589}$ = +36,56 (c=0,52, Chloroform)

Beispiel 120

4-(2-Benzyloxy-phenyl)-1,4-dihydro-2,6-dimethyl-pyridin-3-carbonsäuremethylester-5-carbonsäure-hexylamid

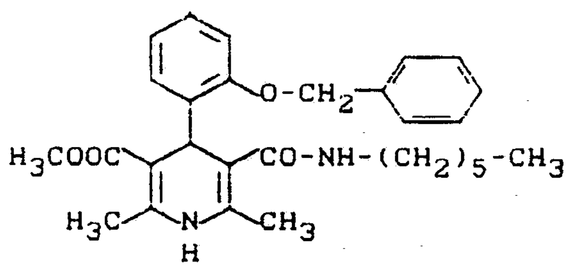

Schmp.: 133 °C

Beispiel 121

4-[2-(4-Methyl-benzolsulfonyloxy)-phenyl]-1,4-dihydro-2,6-dimethyl-pyridin-3-carbonsäuremethylester-5-carbonsäureamid

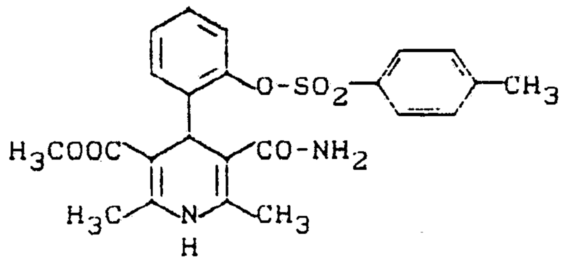

Schmp.: 205° C

Beispiel 122

4-(2-Benzyloxy-phenyl)-1,4-dihydro-2,6-dimethyl-pyridin-2-carbonsäuremethylester-5-carbonsäure-sec.-butylamid

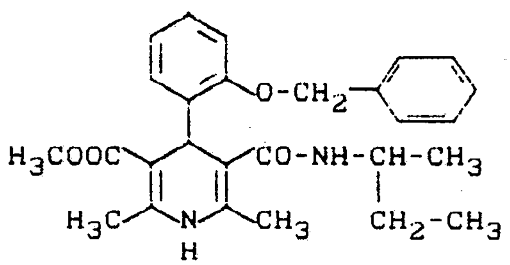

Schmp.: 135° C

Beispiel 123

4-(2-Benzyloxy-phenyl)-1,4-dihydro-2,6-dimethyl-pyridin-3-carbonsäure-isopropylester-5-carbonsäureamid

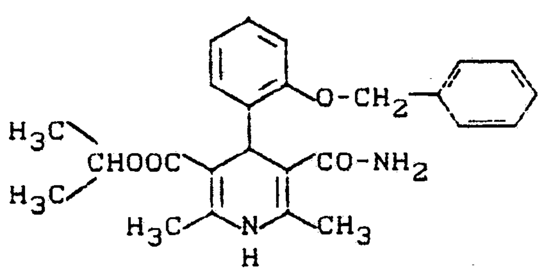

Schmp.: 190° C

Beispiel 124

4-(2-Benzyloxy-phenyl)-1,4-dihydro-2,6-dimethyl-pyridin-5-carbonsäure-(2-methoxy-ethylester)-5-carbonsäureamid

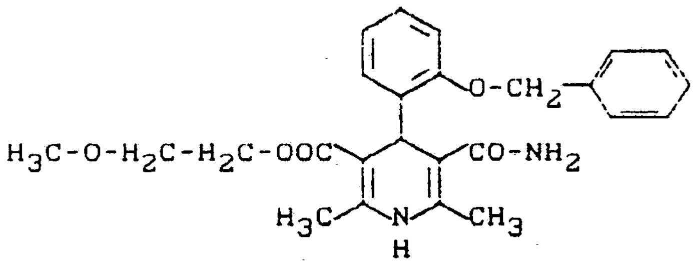

Schmp.: 165° C

Beispiel 125

4-(2-Benzyloxy-phenyl)-1,4-dihydro-2,6-dimethyl-pyridin-3-carbonsäuremethylester-5-carbonsäure-butylamid

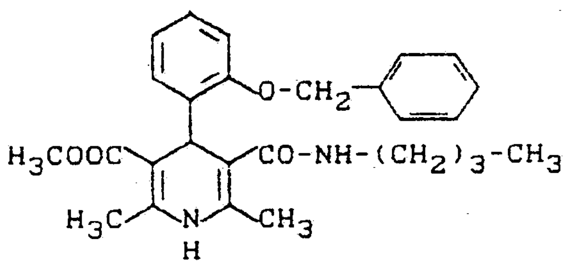

Schmp.: 144 - 148° C

Beispiel 126

4-(2-Benzyloxy-phenyl)-1,4-dihydro-2,6-dimethyl-pyridin-3-carbonsäure-isopropylester-5-carbonsäure-ethylamid

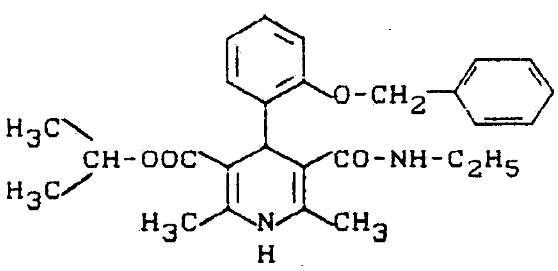

Schmp.: 135° C

Beispiel 127

4-(2-Benzyloxy-phenyl)-1,4-dihydro-2,6-dimethyl-pyridin-3-carbonsäuremethylester-5-carbonsäure-(3-ethoxypropyl)-amid

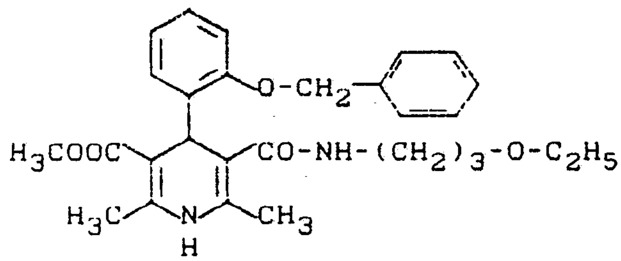

Schmp.: 115° C

Beispiel 128

4-(2-Benzyloxy-phenyl)-1,4-dihydro-2,6-dimethyl-pyridin-3-carbonsäuremethylester-5-carbonsäure-(5-hydroxypentyl)-amid

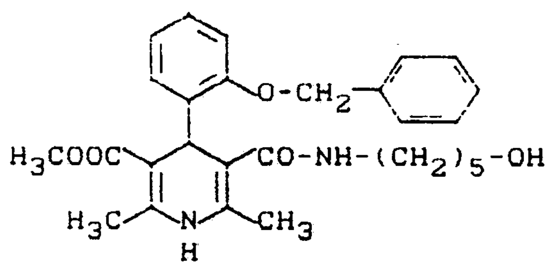

Schmp.: 178° C

Beispiel 129

4-(2-Benzyloxy-phenyl)-1,4-dihydro-2,6-dimethyl-pyridin-3-carbonsäure-(2-methoxy-ethyl)-ester-5-carbonsäure-methylamid

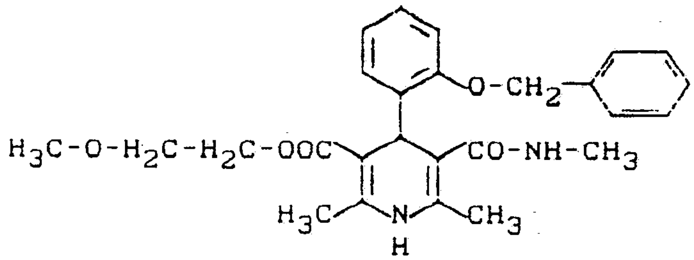

Schmp.: 133 - 135° C

Beispiel 130

4-(2-Benzyloxy-phenyl)-1,4-dihydro-2,6-dimethyl-pyridin-3-carbonsäure-(2-methoxy-ethyl)-ester-5-carbonsäure-ethylamid

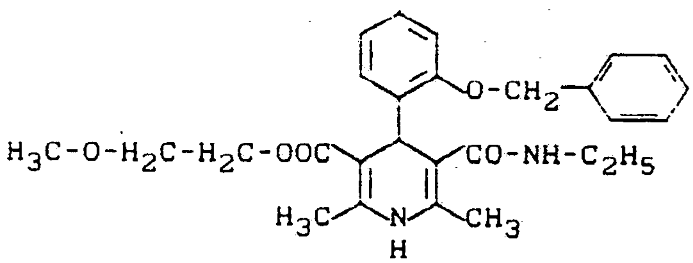

Schmp.: 111° C

Beispiel 131

4-(2-Benzyloxy-phenyl)-1,4-dihydro-2,6-dimethyl-pyridin-3-carbonsäureisopropylester-5-carbonsäuremethylamid

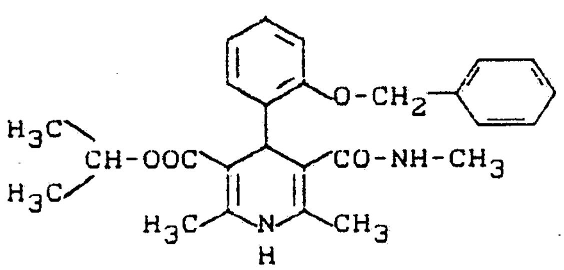

Schmp.: 140 - 143° C

Beispiel 132

4-(2-Benzyloxy-phenyl)-1,4-dihydro-2,6-dimethyl-pyridin-3-carbonsäureisopropylester-5-carbonsäure-cyclopropyl-amid

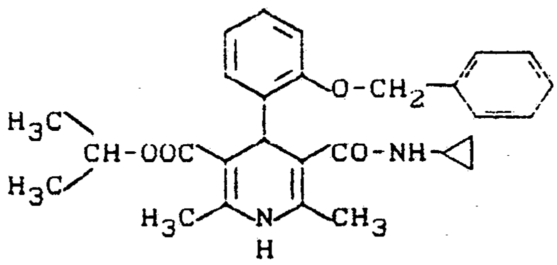

Schmp.: 132 - 135° C

Beispiel 133

4-(2-Benzyloxy-phenyl)-1,4-dihydro-2,6-dimethyl-pyridin-3-carbonsäureisopropylester-5-carbonsäureisopropylamid

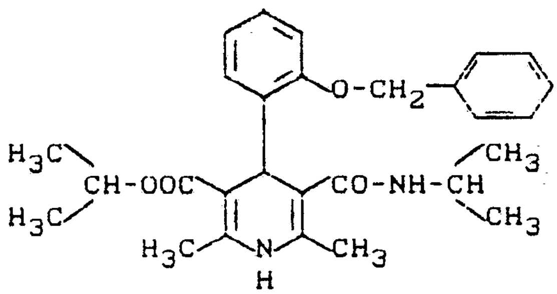

Schmp.: ab 110° C

**Patentansprüche**

**Patentansprüche für folgende Vertragsstaaten : AT, BE, CH, DE, FR, GB, IT, LI, LU, NL, SE**

**1.** Dihydropyridinamide der allgemeinen Formel (I)

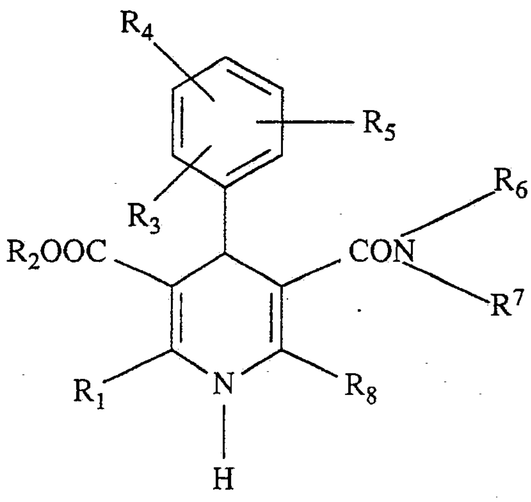

(I)

in welcher

$R_1$ und $R_8$ gleich oder verschieden sind und jeweils für geradkettiges oder verzweigtes Alkyl mit bis zu 4 Kohlenstoffatomen stehen, das gegebenenfalls durch Phenyl substituiert ist,

$R_2$ für einen geradkettigen oder verzweigten, gesättigten oder ungesättigten Kohlenwasserstoffrest mit bis zu 8 Kohlenstoffatomen steht, der durch ein Sauerstoffatom in der Kette unterbrochen sein kann und/oder der substituiert sein kann durch Fluor, Chlor, Cyano, Hydroxy oder durch eine Phenylgruppe, oder durch α-, β- oder γ-Pyridyl,

$R_3$ und $R_4$ gleich oder verschieden sind und jeweils für Wasserstoff, Fluor, Chlor, Alkyl mit bis zu 4 Kohlenstoffatomen, Alkoxy mit bis zu 4 Kohlenstoffatomen, Nitro oder Trifluormethyl stehen,

$R_5$ für eine Gruppe der Formel $-O-(CH_2)_n-R_{11}$, $-S(CH_2)_n-R_{11}$, $-O-SO_2-R_{11}$ oder $-O-CO-(CH_2)_n-R_{11}$ steht,

worin

n 0 bis 3 bedeutet
und
$R_{11}$ Phenyl bedeutet, das bis zu 3-fach gleich oder verschieden substituiert sein kann durch Fluor, Chlor, Nitro, Trifluormethyl, Alkyl mit bis zu 4 Kohlenstoffatomen, Alkoxy mit bis zu 4 Kohlenstoffatomen, Amino, Alkylamino mit bis zu 4 Kohlenstoffatomen, Dialkylamino mit jeweils bis zu 4 Kohlenstoffatomen je Alkylgruppe, Acetylamino oder Pyridyl bedeutet,

und

$R_6$ und $R_7$ gleich oder verschieden sind und jeweils für Wasserstoff oder Cycloalkyl mit 3 bis 7 Kohlenstoffatomen stehen
oder
für geradkettiges oder verzweigtes Alkyl oder Alkenyl mit bis zu 14 Kohlenstoffatomen stehen, die substituiert sein können durch Fluor, Chlor, Hydroxy, Alkoxy mit bis zu 6 Kohlenstoffatomen, Alkylthio mit bis zu 6 Kohlenstoffatomen, Carboxy, Alkoxycarbonyl mit bis zu 6 Kohlenstoffatomen, durch Phenyl und/oder durch eine Gruppe der Formel $-NR_9R_{10}$, worin

$R_9$ und $R_{10}$ gleich oder verschieden sind und jeweils Wasserstoff, Alkyl mit bis zu 6 Kohlenstoffatomen, Benzyl, Phenyl oder Acetyl bedeuten,
oder

für Phenyl stehen, das bis zu 3-fach gleich oder verschieden substituiert sein kann durch Nitro, Fluor, Chlor, Alkyl mit bis zu 4 Kohlenstoffatomen, Alkoxy mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino,
oder
für Pyridyl stehen,

und deren physiologisch unbedenklichen Salze.

2. Dihydropyridinamide der allgemeinen Formel (I) gemäß Anspruch 1, in welcher

$R_{11}$ Phenyl bedeutet, das bis zu 3-fach gleich oder verschieden substituiert sein kann durch Fluor, Chlor, Nitro, Trifluormethyl, Alkyl mit bis zu 4 Kohlenstoffatomen, Alkoxy mit bis zu 4 Kohlenstoffatomen oder Pyridyl bedeutet,
und

$R_6$ und $R_7$ gleich oder verschieden sind und jeweils für Wasserstoff oder Cycloalkyl mit 3 bis 7 Kohlenstoffatomen stehen
oder
für geradkettiges oder verzweigtes Alkyl oder Alkenyl mit bis zu 14 Kohlenstoffatomen stehen, die substituiert sein können durch Fluor, Chlor, Hydroxy, Alkoxy mit bis zu 6 Kohlenstoffatomen, Carboxy, Alkoxycarbonyl mit bis zu 6 Kohlenstoffatomen, durch Phenyl und/oder durch eine Gruppe der Formel $-NR_9R_{10}$, worin

$R_9$ und $R_{10}$ gleich oder verschieden sind und jeweils Wasserstoff, Alkyl mit bis zu 6 Kohlenstoffatomen, Benzyl, Phenyl, Acetyl bedeuten,
oder
für Phenyl stehen, das bis zu 3-fach gleich oder verschieden substituiert sein kann durch Nitro, Fluor, Chlor, Alkyl mit bis zu 4 Kohlenstoffatomen, Alkoxy mit bis zu 4 Kohlenstoffatomen, Trifluormethyl, Amino, Acetylamino oder durch Benzoylamino,
oder
für Pyridyl stehen,

und deren physiologisch unbedenklichen Salze.

3. 4-(2-Benzyloxy-phenyl)-1,4-dihydro-2,6-dimethyl-pyridin-3-carbonsäure-methylester-5-carbonsäure-cyclopropylamid in Racemform und in Form seiner Enantiomere.

4. Verfahren zur Herstellung von Dihydropyridinamiden der allgemeinen Formel (I)

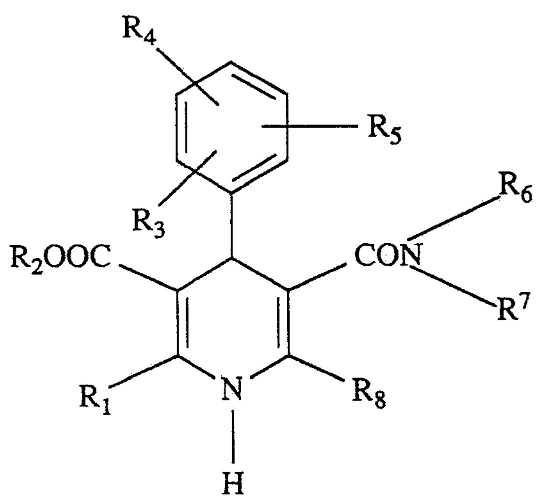

(I)

in welcher

$R_1$ und $R_8$ gleich oder verschieden sind und jeweils für geradkettiges oder verzweigtes Alkyl mit bis zu 4 Kohlenstoffatomen stehen, das gegebenenfalls durch Phenyl substituiert ist,

$R_2$ für einen geradkettigen oder verzweigten, gesättigten oder ungesättigten Kohlenwasserstoffrest mit bis zu 8 Kohlenstoffatomen steht, der durch ein Sauerstoffatom in der Kette unterbrochen sein kann und/oder der substituiert sein kann durch Fluor, Chlor, Cyano, Hydroxy oder durch eine Phenylgruppe, oder durch $\alpha$-, $\beta$- oder $\gamma$-Pyridyl,

$R_3$ und $R_4$ gleich oder verschieden sind und jeweils für Wasserstoff, Fluor, Chlor, Alkyl mit bis zu 4 Kohlenstoffatomen, Alkoxy mit bis zu 4 Kohlenstoffatomen, Nitro oder Trifluormethyl stehen,

$R_5$ für eine Gruppe der Formel $-O-(CH_2)_n-R_{11}$, $-S-(CH_2)_n-R_{11}$, $-O-SO_2-R_{11}$ oder $-O-CO-(CH_2)_n-R_{11}$ steht,

worin

n 0 bis 3 bedeutet
und
$R_{11}$ Phenyl bedeutet, das bis zu 3-fach gleich oder verschieden substituiert sein kann durch Fluor, Chlor, Nitro, Trifluormethyl, Alkyl mit bis zu 4 Kohlenstoffatomen, Alkoxy mit bis zu 4 Kohlenstoffatomen, Amino, Alkylamino mit bis zu 4 Kohlenstoffatomen, Dialkylamino mit jeweils bis zu 4 Kohlenstoffatomen je Alkylgruppe, Acetylamino oder Pyridyl bedeutet,

und

$R_6$ und $R_7$ gleich oder verschieden sind und jeweils für Wasserstoff oder Cycloalkyl mit 3 bis 7 Kohlenstoffatomen stehen
oder
für geradkettiges oder verzweigtes Alkyl oder Alkenyl mit bis zu 14 Kohlenstoffatomen stehen, die substituiert sein können durch Fluor, Chlor, Hydroxy, Alkoxy mit bis zu 6 Kohlenstoffatomen, Alkylthio mit bis zu 6 Kohlenstoffatomen, Carboxy, Alkoxycarbonyl mit bis zu 6 Kohlenstoffatomen, durch Phenyl und/oder durch eine Gruppe der Formel $-NR_9R_{10}$, worin

$R_9$ und $R_{10}$ gleich oder verschieden sind und jeweils Wasserstoff, Alkyl mit bis zu 6 Kohlenstoffatomen, Benzyl, Phenyl oder Acetyl bedeuten,
oder
für Phenyl stehen, das bis zu 3-fach gleich oder verschieden substituiert sein kann durch Nitro, Fluor, Chlor, Alkyl mit bis zu 4 Kohlenstoffatomen, Alkoxy mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino,
oder
für Pyridyl stehen,

dadurch gekennzeichnet, daß man

[A] Aldehyde der allgemeinen Formel (II)

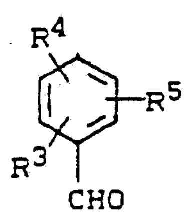

(II)

in welcher
$R^3$ $R^4$ und $R^5$ die oben angegebene Bedeutung haben,

und β-Ketocarbonsäureester der allgemeinen Formel (III)

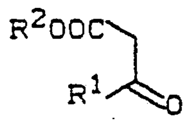

(III)

in welcher

$R^1$ und $R^2$ die oben angegebene Bedeutung haben,

mit β-Ketocarbonsäureamiden der allgemeinen Formel (IV)

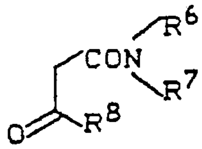

(IV)

in welcher

$R^6$, $R^7$ und $R^8$ die oben angegebene Bedeutung haben,

und Ammoniak, gegebenenfalls in Anwesenheit von inerten Lösemitten umsetzt,

oder daß

[B] Aldehyde der allgemeinen Formel (II) mit β-Ketocarbonsäureestern der allgemeinen Formel (III) und Enaminocarbonsäureamiden der allgemeinen Formel (V)

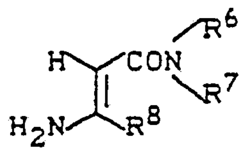

(V)

in welcher

$R^6$, $R^7$ und $R^8$ die oben angegebene Bedeutung haben,

gegebenenfalls in Anwesenheit von inerten Lösemitteln umsetzt,

oder daß

[C] Aldehyde der allgemeinen Formel (II) mit β-Ketocarbonsäureamiden der allgemeinen Formel (IV) und Enaminocarbonsäureestern der allgemeinen Formel (VI)

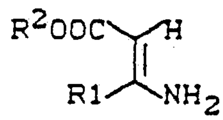

(VI)

in welcher

$R^1$ und $R^2$ die oben angegebene Bedeutung haben,

gegebenenfalls in Anwesenheit von inerten Lösemitteln umsetzt,

oder daß

[D] β-Ketocarbonsäureester der allgemeinen Formel (III) mit Ammoniak und Yliden-β-ketocarbonsäureamiden der allgemeinen Formel (VII)

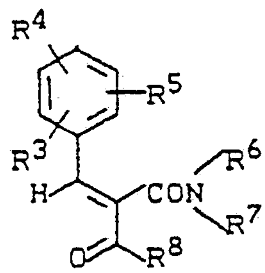

(VII)

in welcher

$R^3$ - $R^8$ die oben angegebene Bedeutung haben,

gegebenenfalls in Anwesenheit von inerten Lösemitteln umsetzt,

oder daß

[E] β-Ketocarbonsäureamide der allgemeinen Formel (IV) mit Ammoniak und Yliden-β-ketocarbonsäureestern der allgemeinen Formel (VIII)

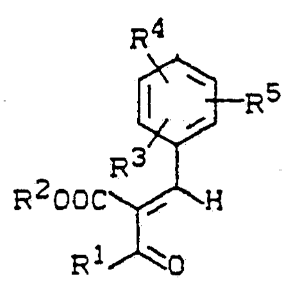

(VIII)

in welcher

$R^1$ - $R^5$ die oben angegebene Bedeutung haben,

gegebenenfalls in Anwesenheit von inerten Lösemitteln umsetzt,

oder daß

[F] Yliden-β-ketocarbonsäureamide der allgemeinen Formel (VII) mit Enaminocarbonsäureestern der allgemeinen Formel (VI) gegebenenfalls in Anwesenheit von inerten Lösemitteln umsetzt,

oder daß

[G] Yliden-β-ketocarbonsäureester der allgemeinen Formel (VIII) mit Enaminocarbonsäureamiden der allgemeinen Formel (V) gegebenenfalls in Anwesenheit von inerten Lösemitteln umsetzt,

oder daß

[H] Dihydropyridinmonocarbonsäuren der allgemeinen Formel (IX)

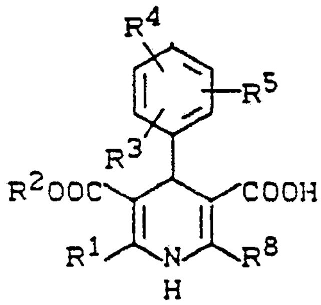

(IX)

in welcher

$R^1$ - $R^5$ und $R^8$ die oben angegebene Bedeutung haben,

gegebenenfalls über ein reaktives Säurederivat mit Aminen der allgemeinen Formel (X)

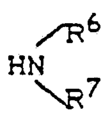

(X)

in welcher

$R^6$ und $R^7$ die oben angegebene Bedeutung haben,

gegebenenfalls in Anwesenheit eines inerten organischen Lösemittels umsetzt.

5. Verfahren zur Herstellung von Verbindungen der allgemeinen Formel (I) gemäß Anspruch 4 nach den Verfahrensvarianten [A]-[G], dadurch gekennzeichnet, daß man als Lösemittel Wasser und inerte Lösungsmittel wie Alkohole, Ether, Amide, Eisessig, Dimethylsulfoxid, Acetonitril oder Pyridin einsetzt und die Reaktion bei Temperaturen zwischen 10 und 150 °C durchführt.

6. Verbindungen der allgemeinen Formel (I) gemäß Anspruch 1 zur Verwendung bei der Bekämpfung von Kreislauferkrankungen.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

9. Verwendung von Verbindungen der allgemeinen Formel (I) gemäß Anspruch 1 bei der Herstellung von

Arzneimitteln.

**Patentansprüche für folgende Vertragsstaaten : ES, GR**

1. Verfahren zur Herstellung Von Dihydropyridinamiden der allgemeinen Formel (I)

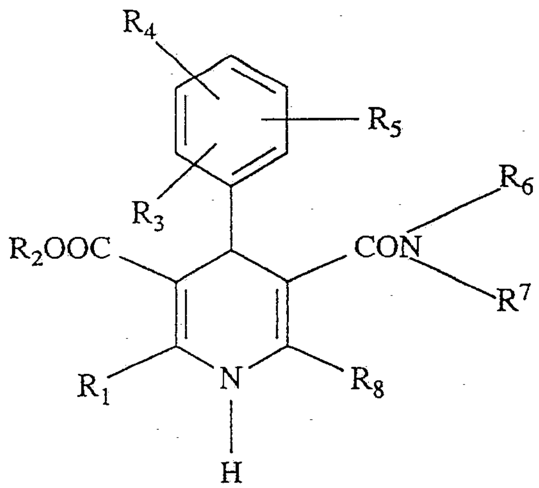

(I)

in welcher

$R_1$ und $R_8$ gleich oder verschieden sind und jeweils für geradkettiges oder verzweigtes Alkyl mit bis zu 4 Kohlenstoffatomen stehen, das gegebenenfalls durch Phenyl substituiert ist,

$R_2$ für einen geradkettigen oder Verzweigten, gesättigten oder ungesättigten Kohlenwasserstoffrest mit bis zu 8 Kohlenstoffatomen steht, der durch ein Sauerstoffatom in der Kette unterbrochen sein kann und/oder der substituiert sein kann durch Fluor, Chlor, Cyano, Hydroxy oder durch eine Phenylgruppe, oder durch α-, β- oder γ-Pyridyl,

$R_3$ und $R_4$ gleich oder verschieden sind und jeweils für Wasserstoff, Fluor, Chlor, Alkyl mit bis zu 4 Kohlenstoffatomen, Alkoxy mit bis zu 4 Kohlenstoffatomen, Nitro oder Trifluormethyl stehen,

$R_5$ für eine Gruppe der Formel $-O-(CH_2)_n-R_{11}$, $-S-(CH_2)_n-R_{11}$, $-O-SO_2-R_{11}$ oder $-O-CO-(CH_2)_n-R_{11}$ steht,

worin

n 0 bis 3 bedeutet
und
$R_{11}$ Phenyl bedeutet, das bis zu 3-fach gleich oder verschieden substituiert sein kann durch Fluor, Chlor, Nitro, Trifluormethyl, Alkyl mit bis zu 4 Kohlenstoffatomen, Alkoxy mit bis zu 4 Kohlenstoffatomen, Amino, Alkylamino mit bis zu 4 Kohlenstoffatomen, Dialkylamino mit jeweils bis zu 4 Kohlenstoffatomen je Alkylgruppe, Acetylamino oder Pyridyl bedeutet,

und

$R_6$ und $R_7$ gleich oder verschieden sind und jeweils für Wasserstoff oder Cycloalkyl mit 3 bis 7 Kohlenstoffatomen stehen
oder
für geradkettiges oder verzweigtes Alkyl oder Alkenyl mit bis zu 14 Kohlenstoffatomen stehen, die substituiert sein können durch Fluor, Chlor, Hydroxy, Alkoxy mit bis zu 6 Kohlenstoffatomen, Alkylthio mit bis zu 6 Kohlenstoffatomen, Carboxy, Alkoxycarbonyl

mit bis zu 6 Kohlenstoffatomen, durch Phenyl und/oder durch eine Gruppe der Formel $-NR_9R_{10}$, worin

$R_9$ und $R_{10}$ gleich oder verschieden sind und jeweils Wasserstoff, Alkyl mit bis zu 6 Kohlenstoffatomen, Benzyl, Phenyl oder Acetyl bedeuten,
oder
für Phenyl stehen, das bis zu 3-fach gleich oder verschieden substituiert sein kann durch Nitro, Fluor, Chlor, Alkyl mit bis zu 4 Kohlenstoffatomen, Alkoxy mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino,
oder
für Pyridyl stehen,

dadurch gekennzeichnet, daß man

[A] Aldehyde der allgemeinen Formel (II)

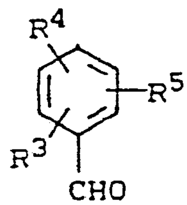

(II)

in welcher
$R^3$, $R^4$ und $R^5$ die oben angegebene Bedeutung haben,

und $\beta$-Ketocarbonsäureester der allgemeinen Formel (III)

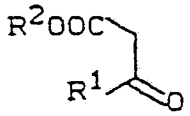

(III)

in welcher

$R^1$ und $R^2$ die oben angegebene Bedeutung haben,

mit $\beta$-Ketocarbonsäureamiden der allgemeinen Formel (IV)

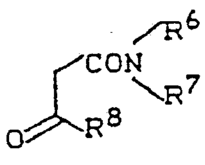

(IV)

in welcher

$R^6$, $R^7$ und $R^8$ die oben angegebene Bedeutung haben,

und Ammoniak, gegebenenfalls in Anwesenheit von inerten Lösemitten umsetzt,

oder daß

[B] Aldehyde der allgemeinen Formel (II) mit β-Ketocarbonsäureestern der allgemeinen Formel (III) und Enaminocarbonsäureamiden der allgemeinen Formel (V)

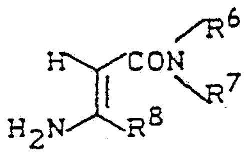

(V)

in welcher

$R^6$, $R^7$ und $R^8$ die oben angegebene Bedeutung haben,

gegebenenfalls in Anwesenheit von inerten Lösemitteln umsetzt,

oder daß

[C] Aldehyde der allgemeinen Formel (II) mit β-Ketocarbonsäureamiden der allgemeinen Formel (IV) und Enaminocarbonsäureestern der allgemeinen Formel (VI)

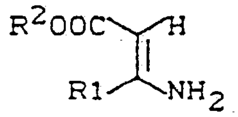

(VI)

in welcher

$R^1$ und $R^2$ die oben angegebene Bedeutung haben,

gegebenenfalls in Anwesenheit von inerten Lösemitteln umsetzt,

oder daß

[D] β-Ketocarbonsäureester der allgemeinen Formel (III) mit Ammoniak und Yliden-β-ketocarbonsäureamiden der allgemeinen Formel (VII)

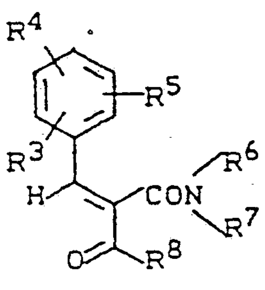

(VII)

in welcher

$R^3$ - $R^8$ die oben angegebene Bedeutung haben,

gegebenenfalls in Anwesenheit von inerten Lösemitteln umsetzt,

oder daß

[E] $\beta$-Ketocarbonsäureamide der allgemeinen Formel (IV) mit Ammoniak und Yliden-$\beta$-ketocarbonsäureestern der allgemeinen Formel (VIII)

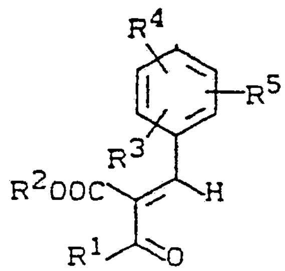

(VIII)

in welcher

$R^1$ - $R^5$ die oben angegebene Bedeutung haben,

gegebenenfalls in Anwesenheit von inerten Lösemitteln umsetzt,

oder daß

[F] Yliden-$\beta$-ketocarbonsäureamide der allgemeinen Formel (VIII) mit Enaminocarbonsäureestern der allgemeinen Formel (VI) gegebenenfalls in Anwesenheit von inerten Lösemitteln umsetzt,

oder daß

[G] Yliden-$\beta$-ketocarbonsäureester der allgemeinen Formel (VIII) mit Enaminocarbonsäureamiden der allgemeinen Formel (V) gegebenenfalls in Anwesenheit von inerten Lösemitteln umsetzt,

oder daß

[H] Dihydropyridinmonocarbonsäuren der allgemeinen Formel (IX)

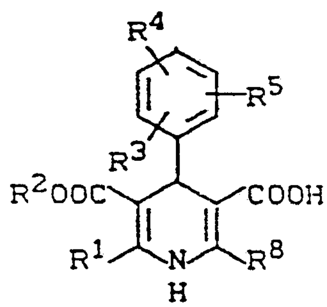

(IX)

in welcher

$R^1$ - $R^5$ und $R^8$ die oben angegebene Bedeutung haben,

gegebenenfalls über ein reaktives Säurederivat mit Aminen der allgemeinen Formel (X)

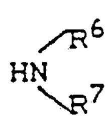

(X)

in welcher

$R^6$ und $R^7$ die oben angegebene Bedeutung haben,

gegebenenfalls in Anwesenheit eines inerten organischen Lösemittels umsetzt.

2. Verfahren zur Herstellung von Verbindungen der allgemeinen Formel (I) gemäß Anspruch 1 nach den Verfahrensvarianten [A]- [G], dadurch gekennzeichnet, daß man als Lösemittel Wasser und inerte Lösungsmittel wie Alkohole, Ether, Amide, Eisessig, Dimethylsulfoxid, Acetonitril oder Pyridin einsetzt und die Reaktion bei Temperaturen zwischen 10 und 150 °C durchführt.

3. Verfahren zur Herstellung von Arzneimitteln, dadurch gekennzeichnet, daß man Verbindungen der allgemeinen Formel (1) gemäß Anspruch 1 gegebenenfalls unter Verwendung von üblichen Hilfs- und Trägerstoffen in eine geeignete Applikationsform überführt.

**Claims**
**Claims for the following Contracting States : AT, BE, CH, DE, FR, GB, IT, LI, LU, NL, SE**

1. Dihydropyridinamides of the general formula (I)

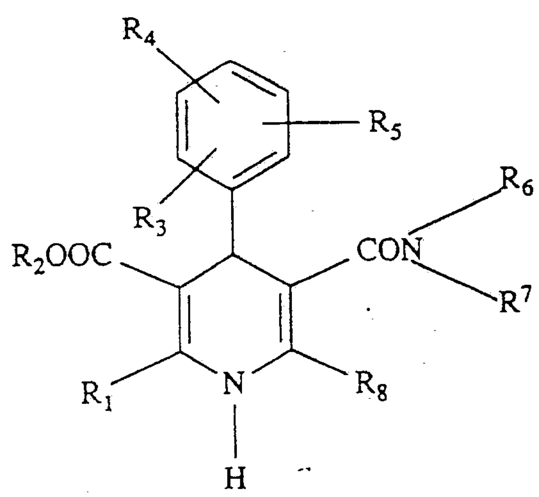

(I)

in which

$R_1$ and $R_8$ are identical or different and represent straight-chain or branched alkyl having up to 4 carbon atoms which is optionally substituted by phenyl,

$R_2$ represents a straight-chain or branched, saturated or unsaturated hydrocarbon radical having up to 8 carbon atoms which may be interrupted in the chain by an oxygen atom and/or which may be substituted by fluorine, chlorine, cyano, hydroxyl or by a phenyl group, or by $\alpha$-, $\beta$- or $\gamma$-pyridyl,

$R_3$ and $R_4$ are identical or different and in each case represent hydrogen, fluorine, chlorine, alkyl having up to 4 carbon atoms, alkoxy having up to 4 carbon atoms, nitro or trifluoromethyl,
$R_5$ represents a group of the formula $-O-(CH_2)_n-R_{11}$, $-S-(CH_2)_n-R_{11}$, $-O-SO_2-R_{11}$ or $-O-CO-(CH_2)_n-R_{11}$,

in which

n denotes 0 to 3,
and
$R_{11}$ denotes phenyl which may be monosubstituted, disubstituted or trisubstituted by identical or different substituents from the series comprising fluorine, chlorine, nitro, trifluoromethyl, alkyl having up to 4 carbon atoms, alkoxy having up to 4 carbon atoms, amino, alkylamino having up to 4 carbon atoms, dialkylamino having up to 4 carbon atoms in each alkyl group, acetylamino or pyridyl,

and
$R_6$ and $R_7$ are identical or different and in each case represent hydrogen or cycloalkyl having 3 to 7 carbon atoms or represent straight-chain or branched alkyl or alkenyl which has up to 14 carbon atoms and which may be substituted by fluorine, chlorine, hydroxyl, alkoxy having up to 6 carbon atoms, alkylthio having up to 6 carbon atoms, carboxyl, alkoxycarbonyl having up to 6 carbon atoms, by phenyl and/or by a group of the formula $-NR_9R_{10}$, in which

$R_9$ and $R_{10}$ are identical or different and in each case denote hydrogen, alkyl having up to 6 carbon atoms, benzyl, phenyl or acetyl,
or
represent phenyl which may be monosubstituted, disubstituted or trisubstituted by identical or different substituents from the series comprising nitro, fluorine, chlorine, alkyl having up to 4 carbon atoms, alkoxy having up to 4 carbon atoms, trifluoromethyl, trifluoromethoxy, amino, alkylamino having up to 6 carbon atoms, dialkylamino having up to 6 carbon atoms in each alkyl group, acetylamino or by benzoylamino,
or
represent pyridyl,
and the physiologically acceptable salts thereof.

**2.** Dihydropyridinamides of the general formula (I) according to Claim 1, in which
$R_{11}$ denotes phenyl which may be monosubstituted, disubstituted or trisubstituted by identical or different substituents from the series comprising fluorine, chlorine, nitro, trifluoromethyl, alkyl having up to 4 carbon atoms, alkoxy having up to 4 carbon atoms, or denotes pyridyl,
and
$R_6$ and $R_7$ are identical or different and in each case represent hydrogen or cycloalkyl having 3 to 7 carbon atoms,
or
represent straight-chain or branched alkyl or alkenyl which has up to 14 carbon atoms and which may be substituted by fluorine, chlorine, hydroxyl, alkoxy having up to 6 carbon atoms, carboxyl, alkoxycarbonyl having up to 6 carbon atoms, by phenyl and/or by a group of the formula $-NR_9R_{10}$, in which

$R_9$ and $R_{10}$ are identical or different and in each case denote hydrogen, alkyl having up to 6 carbon atoms, benzyl, phenyl, acetyl,
or
represent phenyl which may be monosubstituted, disubstituted or trisubstituted by identical or different substituents from the series comprising nitro, fluorine, chlorine, alkyl having up to 4 carbon atoms, alkoxy having up to 4 carbon atoms, trifluoromethyl, amino, acetylamino or by benzoylamino,
or
represent pyridyl,

and the physiologically acceptable salts thereof.

**3.** Methyl 5-(cyclopropylcarbamoyl)-4-(2-benzyloxyphenyl)-1,4-dihydro-2,6-dimethyl-pyridine-3-carboxylate in racemic form and in the form of its enantiomers.

**4.** Process for the preparation of dihydropyridinamides of the general formula (I)

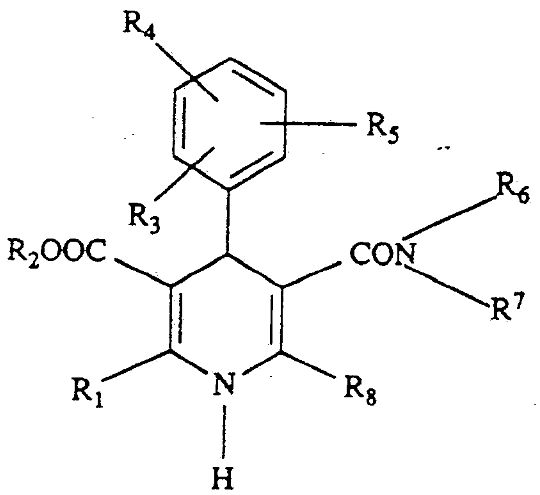

(I)

in which

$R_1$ and $R_8$ are identical or different and in each case represent straight-chain or branched alkyl having up to 4 carbon atoms which is optionally substituted by phenyl,

$R_2$ represents a straight-chain or branched, saturated or unsaturated hydrocarbon radical having up to 8 carbon atoms which may be interrupted in the chain by an oxygen atom and/or which may be substituted by fluorine, chlorine, cyano, hydroxyl or by a phenyl group, or by $\alpha$-, $\beta$- or $\gamma$-pyridyl,

$R_3$ and $R_4$ are identical or different and in each case represent hydrogen, fluorine, chlorine, alkyl having up to 4 carbon atoms, alkoxy having up to 4 carbon atoms, nitro or trifluoromethyl,

$R_5$ represents a group of the formula $-O-(CH_2)_n-R_{11}$, $-S-(CH_2)_n-R_{11}$, $-O-SO_2-R_{11}$ or $-O-CO-(CH_2)_n-R_{11}$,

in which

n denotes 0 to 3,
and
$R_{11}$ denotes phenyl which may be monosubstituted, disubstituted or trisubstituted by identical or different substituents from the series comprising fluorine, chlorine, nitro, trifluoromethyl, alkyl having up to 4 carbon atoms, alkoxy having up to 4 carbon atoms, amino, alkylamino having up to 4 carbon atoms, dialkylamino having up to 4 carbon atoms in each alkyl group, acetylamino or pyridyl,

and
$R_6$ and $R_7$ are identical or different and in each case represent hydrogen or cycloalkyl having 3 to 7 carbon atoms
or
represent straight-chain or branched alkyl or alkenyl which has up to 14 carbon atoms and which may be substituted by fluorine, chlorine, hydroxyl, alkoxy having up to 6 carbon atoms, alkylthio having up to 6 carbon atoms, carboxyl, alkoxycarbonyl having up to 6 carbon atoms, by phenyl and/or by a group of the formula $-NR_9R_{10}$, in which

$R_9$ and $R_{10}$ are identical or different and in each case denote hydrogen, alkyl having up to 6 carbon atoms, benzyl, phenyl or acetyl,
or
represent phenyl which may be monosubstituted, disubstituted or trisubstituted by identical or different substituents from the series comprising nitro, fluorine, chlorine, alkyl having up to 4 carbon atoms, alkoxy having up to 4 carbon atoms, trifluoromethyl, trifluoromethoxy, amino, alkylamino having up to 6 carbon atoms, dialkylamino having up to 6 carbon atoms in each alkyl group, acetylamino or by benzoylamino,
or

represent pyridyl,
characterised in that

[A] aldehydes of the general formula (II)

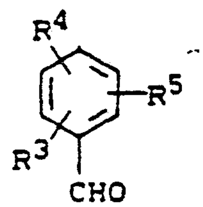

(II)

in which
$R^3$, $R^4$ and $R^5$ have the abovementioned meaning,

and $\beta$-ketocarboxylates of the general formula (III)

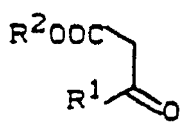

(III)

in which

$R^1$ and $R^2$ have the abovementioned meaning, are reacted with $\beta$-ketocarboxamides of the general formula (IV)

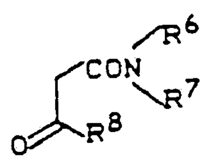

(IV)

in which

$R^6$, $R^7$ and $R^8$ have the abovementioned meaning, and ammonia, if appropriate in the presence of inert solvents,

or in that

[B] aldehydes of the general formula (II) are reacted with $\beta$-ketocarboxylates of the general formula (III) and enaminocarboxamides of the general formula (V)

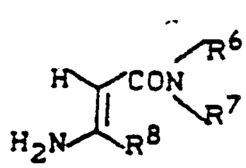

(V)

in which

$R^6$, $R^7$ and $R^8$ have the abovementioned meaning,

if appropriate in the presence of inert solvents,

or in that

[C] aldehydes of the general formula (II) are reacted with β-ketocarboxamides of the general formula (IV) and enaminocarboxylates of the general formula (VI)

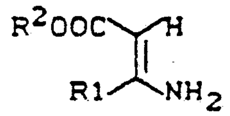

(VI)

in which

$R^1$ and $R^2$ have the abovementioned meaning,

if appropriate in the presence of inert solvents,

or in that

[D] β-ketocarboxylates of the general formula (III) are reacted with ammonia and ylidene-β-ketocarboxamides of the general formula (VII)

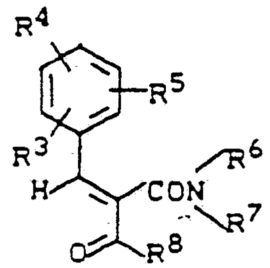

(VII)

in which

$R^3$ - $R^8$ have the abovementioned meaning, if appropriate in the presence of inert solvents,

or in that

[E] β-ketocarboxamides of the general formula (IV) are reacted with ammonia and ylidene-β-ketocarboxylates of the general formula (VIII)

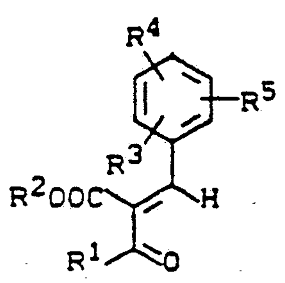

(VIII)

in which

$R^1$ - $R^5$ have the abovementioned meaning,

if appropriate in the presence of inert solvents,

or in that

[F] ylidene-β-ketocarboxamides of the general formula (VII) are reacted with enaminocarboxylates of the general formula (VI), if appropriate in the presence of inert solvents,

or in that

[G] ylidene-β-ketocarboxylates of the general formula (VIII) are reacted with enaminocarboxamides of the general formula (V), if appropriate in the presence of inert solvents,

or in that

[H] dihydropyridinemonocarboxylic acids of the general formula (IX)

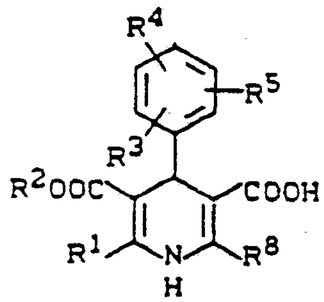

(IX)

in which

$R^1$ - $R^5$ and $R^8$ have the abovementioned meaning,

if appropriate via a reactive acyl derivative, are reacted with amines of the general formula (X)

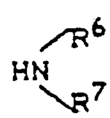

(X)

in which

$R^6$ and $R^7$ have the abovementioned meaning,

if appropriate in the presence of an inert organic solvent.

5. Process for the preparation of compounds of the general formula (I) according to Claim 4 by process variants [A]-[G], characterised in that the solvent employed is water and inert solvents such as alcohols, ethers, amides, glacial acetic acid, dimethyl sulphoxide, acetonitrile or pyridine, and the reaction is carried out at temperatures between 10 and 150 °C.

6. Compounds of the general formula (I) according to Claim 1 for use in combating circulation disorders.

7. Medicaments containing at least one compound of the general formula (I) according to Claim 1.

8. Process for the production of medicaments, characterised in that compounds of the general formula (I)

according to Claim 1 are converted into a suitable form of administration, if appropriate using conventional auxiliaries and excipients.

9. Use of compounds of the general formula (I) according to Claim 1 in the production of medicaments.

**Claims for the following Contracting States : ES, GR**

1. Process for the preparation of dihydropyridinamides of the general formula (I)

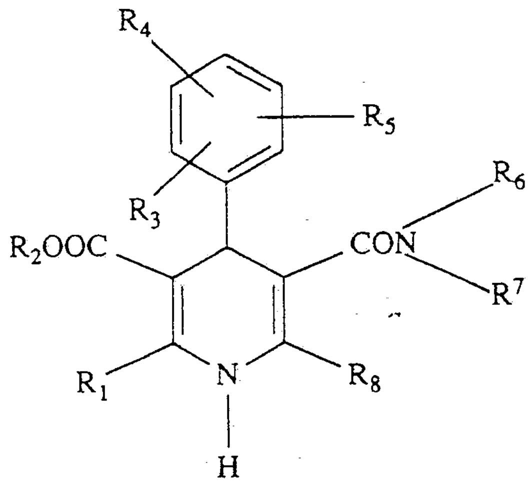

(I)

in which

$R_1$ and $R_8$ are identical or different and represent in each case straight-chain or branched alkyl having up to 4 carbon atoms which is optionally substituted by phenyl,

$R_2$ represents a straight-chain or branched, saturated or unsaturated hydrocarbon radical having up to 8 carbon atoms which may be interrupted in the chain by an oxygen atom and/or which may be substituted by fluorine, chlorine, cyano, hydroxyl or by a phenyl group, or by $\alpha$-, $\beta$- or $\gamma$-pyridyl,

$R_3$ and $R_4$ are identical or different and in each case represent hydrogen, fluorine, chlorine, alkyl having up to 4 carbon atoms, alkoxy having up to 4 carbon atoms, nitro or trifluoromethyl,

$R_5$ represents a group of the formula $-O-(CH_2)_n-R_{11}$, $-S-(CH_2)_n-R_{11}$, $-O-SO_2-R_{11}$ or $-O-CO-(CH_2)_n-R_{11}$,

in which

n denotes 0 to 3,
and
$R_{11}$ denotes phenyl which may be monosubstituted, disubstituted or trisubstituted by identical or different substituents from the series comprising fluorine, chlorine, nitro, trifluoromethyl, alkyl having up to 4 carbon atoms, alkoxy having up to 4 carbon atoms, amino, alkylamino having up to 4 carbon atoms, dialkylamino having up to 4 carbon atoms in each alkyl group, acetylamino or pyridyl,

and
$R_6$ and $R_7$ are identical or different and in each case represent hydrogen or cycloalkyl having 3 to 7 carbon atoms
or
represent straight-chain or branched alkyl or alkenyl which has up to 14 carbon atoms and which may be substituted by fluorine, chlorine, hydroxyl, alkoxy having up to 6 carbon atoms, alkylthio having up to 6 carbon atoms, carboxyl, alkoxycarbonyl having up to 6 carbon atoms, by phenyl and/or by a group of the formula $-NR_9R_{10}$, in which

$R_9$ and $R_{10}$ are identical or different and in each case denote hydrogen, alkyl having up to 6 carbon atoms, benzyl, phenyl or acetyl,
or
represent phenyl which may be monosubstituted, disubstituted or trisubstituted by identical or different substituents from the series comprising nitro, fluorine, chlorine, alkyl having up to 4 carbon atoms, alkoxy having up to 4 carbon atoms, trifluoromethyl, trifluoromethoxy, amino, alkylamino having up to 6 carbon atoms, dialkylamino having up to 6 carbon atoms in each alkyl group, acetylamino or by benzoylamino,
or
represent pyridyl,
characterised in that

[A] aldehydes of the general formula (II)

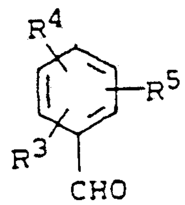

(II)

in which
$R^3$, $R^4$ and $R^5$ have the abovementioned meaning,

and β-ketocarboxylates of the general formula (III)

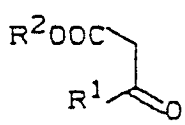

(III)

in which

$R^1$ and $R^2$ have the abovementioned meaning,

are reacted with β-ketocarboxamides of the general formula (IV)

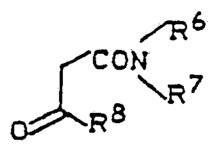

(IV)

in which

$R^6$, $R^7$ and $R^8$ have the abovementioned meaning,

and ammonia, if appropriate in the presence of inert solvents,

or in that

[B] aldehydes of the general formula (II) are reacted with β-ketocarboxylates of the general formula (III) and enaminocarboxamides of the general formula (V)

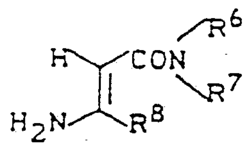

(V)

in which

$R^6$, $R^7$ and $R^8$ have the abovementioned meaning,

if appropriate in the presence of inert solvents,

or in that

[C] aldehydes of the general formula (II) are reacted with $\beta$-ketocarboxamides of the general formula (IV) and enaminocarboxylates of the general formula (VI)

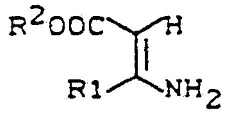

(VI)

in which

$R^1$ and $R^2$ have the abovementioned meaning,

if appropriate in the presence of inert solvents,

or in that

[D] $\beta$-ketocarboxylates of the general formula (III) are reacted with ammonia and ylidene-$\beta$-ketocarboxamides of the general formula (VII)

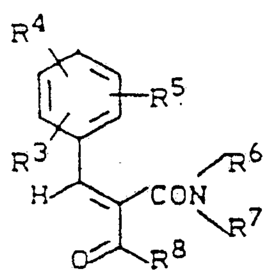

(VII)

in which

$R^3$ - $R^8$ have the abovementioned meaning,

if appropriate in the presence of inert solvents,

or in that

[E] $\beta$-ketocarboxamides of the general formula (IV) are reacted with ammonia and ylidene-$\beta$-ketocarboxylates of the general formula (VIII)

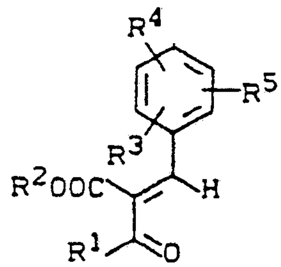

(VIII)

in which

$R^1$ - $R^5$ have the abovementioned meaning,

if appropriate in the presence of inert solvents,

or in that

[F] ylidene-β-ketocarboxamides of the general formula (VII) are reacted with enaminocarboxylates of the general formula (VI), if appropriate in the presence of inert solvents,

or in that

[G] ylidene-β-ketocarboxylates of the general formula (VIII) are reacted with enaminocarboxamides of the general formula (V), if appropriate in the presence of inert solvents,

or in that

[H] dihydropyridinemonocarboxylic acids of the general formula (IX)

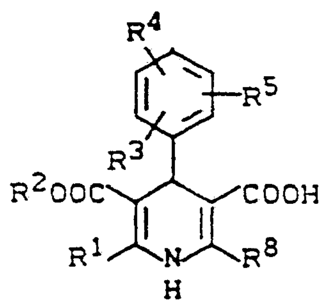

(IX)

in which

$R^1$ - $R^5$ and $R^8$ have the abovementioned meaning,

if appropriate via a reactive acyl derivative, are reacted with amines of the general formula (X)

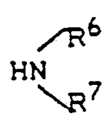

(X)

in which

$R^6$ and $R^7$ have the abovementioned meaning,

if appropriate in the presence of an inert organic solvent.

**2.** Process for the preparation of compounds of the general formula (I) according to Claim 1 by process variants [A]-[G], characterised in that the solvent employed is water and inert solvents such as alcohols, ethers, amides, glacial acetic acid, dimethyl sulphoxide, acetonitrile or pyridine, and the reaction is carried out at temperatures between 10 and 150° C.

**3.** Process for the production of medicaments, characterised in that compounds of the general formula (I) according to Claim 1 are converted into a suitable form of administration, if appropriate using conventional auxiliaries and excipients.

**Revendications**
**Revendications pour les Etats contractants suivants : AT, BE, CH, DE, FR, GB, IT, LI, LU, NL, SE**

**1.** Dihydropyridinamides répondant à la formule générale (I)

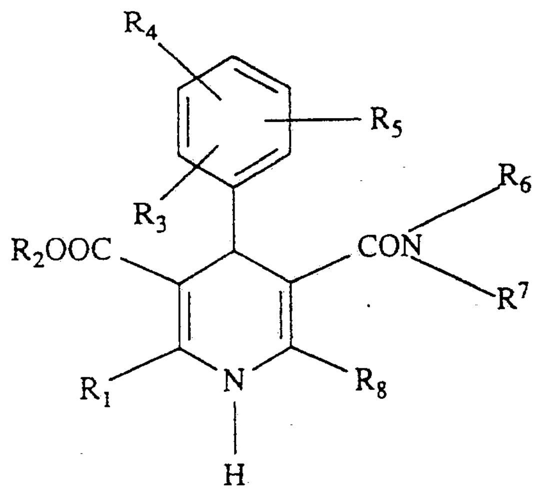

(I)

dans laquelle

$R_1$ et $R_8$ sont identiques ou différents et représentent chacun un radical alkyle, à chaîne droite ou ramifiée, contenant jusqu'à 4 atomes de carbone, éventuellement substitué par un groupe phényle ;

$R_2$ représente un radical d'hydrocarbure saturé ou insaturé, à chaîne droite ou ramifiée, contenant jusqu'à 8 atomes de carbone, qui peut être interrompu dans la chaîne par un atome d'oxygène et/ou qui peut être substitué par un atome de fluor, un atome de chlore, un groupe cyano, un groupe hydroxyle ou par un groupe phényle ou encore par un groupe $\alpha$-, $\beta$- ou $\gamma$-pyridyle ;

$R_3$ et $R_4$ sont identiques ou différents et représentent chacun un atome d'hydrogène, un atome de fluor, un atome de chlore, un groupe alkyle contenant jusqu'à 4 atomes de carbone, un groupe alcoxy contenant jusqu'à 4 atomes de carbone, un groupe nitro ou un groupe trifluorométhyle ;

$R_5$ représente un groupe de formule $-O-(CH_2)_n-R_{11}$, $-S-(CH_2)_n-R_{11}$, $-O-SO_2-R_{11}$ ou $-O-CO-(CH_2)_n-R_{11}$,

où

n est égal à 0-3
et
$R_{11}$ représente un groupe phényle qui peut être substitué jusqu'à trois fois, de manière identique ou différente, par un atome de fluor, un atome de chlore, un groupe nitro, un groupe trifluorométhyle, un groupe alkyle contenant jusqu'à 4 atomes de carbone, un groupe alcoxy contenant jusqu'à 4 atomes de carbone, un groupe amino, un groupe alkylamino contenant jusqu'à 4 atomes de carbone, un groupe dialkylamino contenant chaque fois jusqu'à 4 atomes de carbone dans chaque groupe alkyle, un groupe acétylamino ou un groupe pyridyle,

et

$R_6$ et $R_7$ sont identiques ou différents et représentent chacun un atome d'hydrogène ou un groupe cycloalkyle contenant de 3 à 7 atomes de carbone
ou
représentent un groupe alkyle ou un groupe alcényle à chaîne droite ou ramifiée, contenant jusqu'à 14 atomes de carbone, qui peuvent être substitués par un atome de fluor, un atome de chlore, un groupe hydroxyle, un groupe alcoxy contenant jusqu'à 6 atomes de carbone, un groupe alkylthio contenant jusqu'à 6 atomes de carbone, un groupe carboxyle, un groupe alcoxycarbonyle contenant jusqu'à 6 atomes de carbone, par un groupe phényle et/ou par un groupe de formule $-NR_9R_{10}$, où

$R_9$ et $R_{10}$ sont identiques ou différents et représentent chacun un atome d'hydrogène, un groupe alkyle contenant jusqu'à 6 atomes de carbone, un groupe benzyle, un groupe phényle ou un groupe acétyle
ou
représentent un groupe phényle qui peut être substitué jusqu'à trois fois, de manière identique ou différente, par un groupe nitro, un atome de fluor, un atome de chlore, un groupe alkyle contenant jusqu'à 4 atomes de carbone, un groupe alcoxy contenant jusqu'à 4 atomes de carbone, un groupe trifluorométhyle, un groupe trifluorométhoxy, un groupe amino, un groupe alkylamino contenant jusqu'à 6 atomes de carbone, un groupe dialkylamino contenant, chaque fois, jusqu'à 6 atomes de carbone dans chaque groupe alkyle, un groupe acétylamino ou par un groupe benzoylamino
ou encore
représentent un groupe pyridyle,

ainsi que leurs sels physiologiquement acceptables.

**2.** Dihydropyridinamides répondant à la formule générale (I) selon la revendication 1, dans laquelle

$R_{11}$ représente un groupe phényle qui peut être substitué jusqu'à trois fois, de manière identique ou différente, par un atome de fluor, un atome de chlore, un groupe nitro, un groupe trifluorométhyle, un groupe alkyle contenant jusqu'à 4 atomes de carbone, un groupe alcoxy contenant jusqu'à 4 atomes de carbone, ou représente un groupe pyridyle,

et

$R_6$ et $R_7$ sont identiques ou différents et représentent chacun un atome d'hydrogène ou un groupe cycloalkyle contenant de 3 à 7 atomes de carbone
ou
représentent un groupe alkyle ou un groupe alcényle à chaîne droite ou ramifiée, contenant jusqu'à 14 atomes de carbone, qui peuvent être substitués par un atome de fluor, un atome de chlore, un groupe hydroxyle, un groupe alcoxy contenant jusqu'à 6 atomes de carbone, un groupe carboxyle, un groupe alcoxycarbonyle contenant jusqu'à 6 atomes de carbone, un groupe phényle et/ou un groupe de formule $-NR_9R_{10}$, où

$R_9$ et $R_{10}$ sont identiques ou différents et représentent chacun un atome d'hydrogène, un groupe alkyle contenant jusqu'à 6 atomes de carbone, un groupe benzyle, un

groupe phényle, un groupe acétyle
ou
représentent un groupe phényle qui peut être substitué jusqu'à trois fois, de manière identique ou différente, par un groupe nitro, un atome de fluor, un atome de chlore, un groupe alkyle contenant jusqu'à 4 atomes de carbone, un groupe alcoxy contenant jusqu'à 4 atomes de carbone, un groupe trifluorométhyle, un groupe amino, un groupe acétylamino ou par un groupe benzoylamino
ou encore
représentent un groupe pyridyle,

ainsi que leurs sels physiologiquement acceptables.

**3.** Cyclopropylamide de l'acide 4-(2-benzyloxyphényl)-1,4-dihydro-2,6-diméthylpyridine-3-méthoxycarbonyl-5-carboxylique sous forme racémique et sous forme de ses énantiomères.

**4.** Procédé pour la préparation de dihydropyridinamides répondant à la formule générale (I)

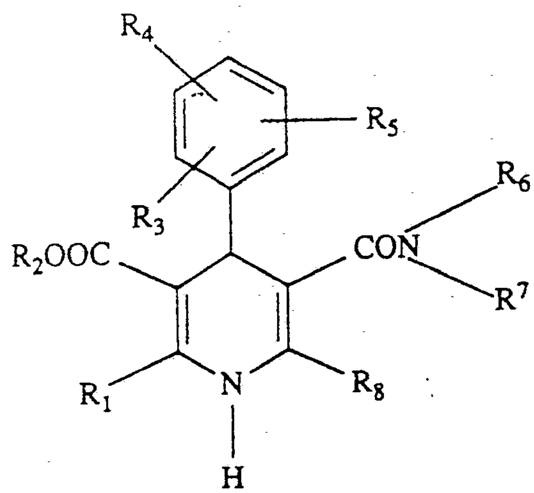

(I)

dans laquelle

$R_1$ et $R_8$ sont identiques ou différents et représentent chacun un radical alkyle, à chaîne droite ou ramifiée, contenant jusqu'à 4 atomes de carbone, éventuellement substitué par un groupe phényle ;

$R_2$ représente un radical d'hydrocarbure saturé ou insaturé, à chaîne droite ou ramifiée, contenant jusqu'à 8 atomes de carbone, qui peut être interrompu dans la chaîne par un atome d'oxygène et/ou qui peut être substitué par un atome de fluor, un atome de chlore, un groupe cyano, un groupe hydroxyle ou par un groupe phényle ou encore par un groupe $\alpha$-, $\beta$-ou $\gamma$-pyridyle ;

$R_3$ et $R_4$ sont identiques ou différents et représentent chacun un atome d'hydrogène, un atome de fluor, un atome de chlore, un groupe alkyle contenant jusqu'à 4 atomes de carbone, un groupe alcoxy contenant jusqu'à 4 atomes de carbone, un groupe nitro ou un groupe trifluorométhyle ;

$R_5$ représente un groupe de formule $-O-(CH_2)_n-R_{11}$, $-S-(CH_2)_n-R_{11}$, $-O-SO_2-R_{11}$ ou $-O-CO-(CH_2)_n-R_{11}$,

où

n est égal à 0-3
et

$R_{11}$ représente un groupe phényle qui peut être substitué jusqu'à trois fois, de manière identique ou différente, par un atome de fluor, un atome de chlore, un groupe nitro, un groupe trifluorométhyle, un groupe alkyle contenant jusqu'à 4 atomes de carbone, un groupe alcoxy contenant jusqu'à 4 atomes de carbone, un groupe amino, un groupe alkylamino contenant jusqu'à 4 atomes de carbone, un groupe dialkylamino contenant chaque fois jusqu'à 4 atomes de carbone dans chaque groupe alkyle, un groupe acétylamino ou représente un groupe pyridyle,

et

$R_6$ et $R_7$ sont identiques ou différents et représentent chacun un atome d'hydrogène ou un groupe cycloalkyle contenant de 3 à 7 atomes de carbone
ou
représentent un groupe alkyle ou un groupe alcényle à chaîne droite ou ramifiée, contenant jusqu'à 14 atomes de carbone, qui peuvent être substitués par un atome de fluor, un atome de chlore, un groupe hydroxyle, un groupe alcoxy contenant jusqu'à 6 atomes de carbone, un groupe alkylthio contenant jusqu'à 6 atomes de carbone, un groupe carboxyle, un groupe alcoxycarbonyle contenant jusqu'à 6 atomes de carbone, par un groupe phényle et/ou par un groupe de formule $-NR_9R_{10}$, où

$R_9$ et $R_{10}$ sont identiques ou différents et représentent chacun un atome d'hydrogène, un groupe alkyle contenant jusqu'à 6 atomes de carbone, un groupe benzyle, un groupe phényle ou un groupe acétyle
ou
représentent un groupe phényle qui peut être substitué jusqu'à trois fois, de manière identique ou différente, par un groupe nitro, un atome de fluor, un atome de chlore, un groupe alkyle contenant jusqu'à 4 atomes de carbone, un groupe alcoxy contenant jusqu'à 4 atomes de carbone, un groupe trifluorométhyle, un groupe trifluorométhoxy, un groupe amino, un groupe alkylamino contenant jusqu'à 6 atomes de carbone, un groupe dialkylamino contenant, chaque fois, jusqu'à 6 atomes de carbone dans chaque groupe alkyle, un groupe acétylamino ou par un groupe benzoylamino
ou
représentent un groupe pyridyle,

**caractérisé en ce que,**

[A] on fait réagir des aldéhydes répondant à la formule générale (II)

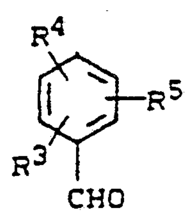

(II)

dans laquelle
$R^3$, $R^4$ et $R^5$ ont la signification mentionnée ci-dessus,

et des esters $\beta$-cétocarboxyliques répondant à la formule générale (III)

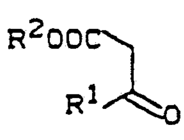

(III)

dans laquelle

$R^1$ et $R^2$ ont la signification mentionnée ci-dessus,

avec des amides $\beta$-cétocarboxyliques répondant à la formule générale (IV)

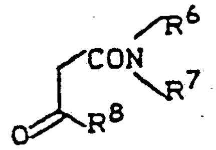

(IV)

dans laquelle

$R^6$, $R^7$ et $R^8$ ont la signification mentionnée ci-dessus,

et de l'ammoniac, éventuellement en présence de solvants inertes,

ou **en ce que**

[B] on fait réagir des aldéhydes répondant à la formule générale (II) avec des esters $\beta$-cétocarboxyliques répondant à la formule générale (III) et des amides énaminocarboxyliques répondant à la formule générale (V)

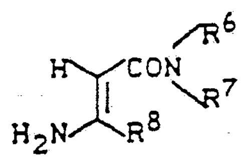

(V)

dans laquelle

$R^6$, $R^7$ et $R^8$ ont la signification mentionnée ci-dessus,

éventuellement en présence de solvants inertes,

ou **en ce que**

[C] on fait réagir des aldéhydes répondant à la formule générale (II) avec des amides $\beta$-cétocarboxyliques répondant à la formule générale (IV) et des esters énaminocarboxyliques répondant à la formule générale (VI)

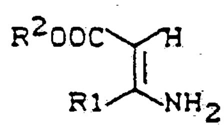

(VI)

dans laquelle

$R^1$ et $R^2$ ont la signification mentionnée ci-dessus,

éventuellement en présence de solvants inertes,

ou **en ce que**

[D] on fait réagir des esters $\beta$-cétocarboxyliques répondant à la formule générale (III) avec de

l'ammoniac et des amides ylidène-β-cétocarboxyliques répondant à la formule générale (VII)

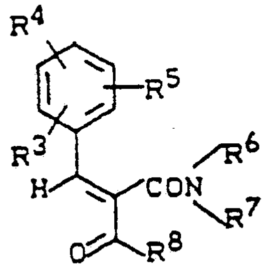

(VII)

dans laquelle

$R^3$-$R^8$ ont la signification mentionnée ci-dessus,

éventuellement en présence de solvants inertes,

ou **en ce que**

[E] on fait réagir des amides β-cétocarboxyliques répondant à la formule générale (IV) avec de l'ammoniac et des esters ylidène-β-cétocarboxyliques répondant à la formule générale (VIII)

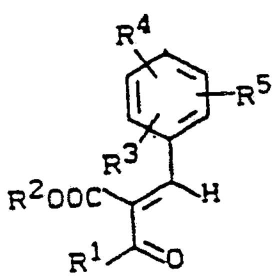

(VIII)

dans laquelle

$R^1$-$R^5$ ont la signification mentionnée ci-dessus,

éventuellement en présence de solvants inertes,

ou **en ce que**

[F] on fait réagir des amides ylidène-β-cétocarboxyliques répondant à la formule générale (VII) avec des esters énaminocarboxyliques répondant à la formule générale (VI), éventuellement en présence de solvants inertes,

ou **en ce que**

[G] on fait réagir des esters ylidène-β-cétocarboxyliques répondant à la formule générale (VIII) avec des amides énaminocarboxyliques répondant à la formule générale (V), éventuellement en présence de solvants inertes,

ou **en ce que**

[H] on fait réagir des acides dihydropyridinemonocarboxyliques répondant à la formule générale (IX)

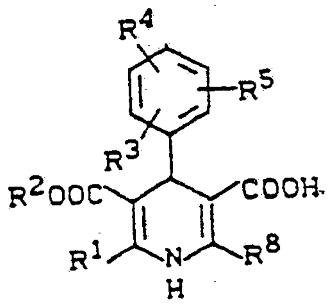

(IX)

dans laquelle

$R^1$-$R^5$ et $R^8$ ont la signification mentionnée ci-dessus,

éventuellement par l'intermédiaire d'un dérivé d'acide réactif, avec des amines répondant à la formule générale (X)

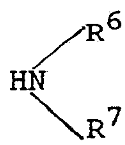

(X)

dans laquelle

$R^6$ et $R^7$ ont la signification mentionnée ci-dessus,

éventuellement en présence d'un solvant organique inerte.

**5.** Procédé pour la préparation de composés répondant à la formule générale (I) selon la revendication 4, conformément aux variantes opératoires [A]-[G], **caractérisé en ce qu'**on met en oeuvre, comme solvants, de l'eau et des solvants inertes tels que des alcools, des éthers, des amides, l'acide acétique, le diméthylsulfoxyde, l'acétonitrile ou la pyridine et **en ce qu'**on effectue la réaction à des températures allant de 10 à 150 °C.

**6.** Composés répondant à la formule générale (I) selon la revendication 1, destinés à être utilisés dans la lutte-contre des maladies circulatoires.

**7.** Médicament contenant, au moins, un composé répondant à la formule générale (I) selon la revendication 1.

**8.** Procédé pour la préparation de médicaments, **caractérisé en ce qu'**on transforme des composés répondant à la formule générale (I) selon la revendication 1, éventuellement en utilisant des substances auxiliaires et de support habituelles, en une forme d'application appropriée.

**9.** Utilisation de composés répondant à la formule générale (I) selon la revendication 1, lors de la préparation de médicaments.

**Revendications pour les Etats contractants suivants : ES, GR**

**1.** Procédé pour la préparation de dihydropyridinamides répondant à la formule générale (I)

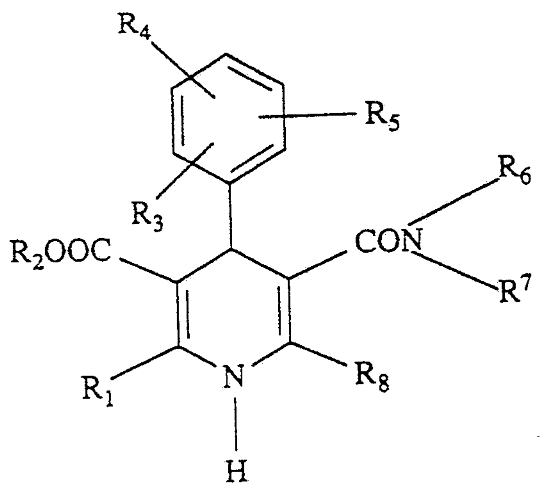

(I)

dans laquelle

$R_1$ et $R_8$ sont identiques ou différents et représentent chacun un radical alkyle, à chaîne droite ou ramifiée, contenant jusqu'à 4 atomes de carbone, éventuellement substitué par un groupe phényle ;

$R_2$ représente un radical d'hydrocarbure saturé ou insaturé, à chaîne droite ou ramifiée, contenant jusqu'à 8 atomes de carbone, qui peut être interrompu dans la chaîne par un atome d'oxygène et/ou qui peut être substitué par un atome de fluor, un atome de chlore, un groupe cyano, un groupe hydroxyle ou par un groupe phényle ou encore par un groupe $\alpha$-, $\beta$-ou $\gamma$-pyridyle ;

$R_3$ et $R_4$ sont identiques ou différents et représentent chacun un atome d'hydrogène, un atome de fluor, un atome de chlore, un groupe alkyle contenant jusqu'à 4 atomes de carbone, un groupe alcoxy contenant jusqu'à 4 atomes de carbone, un groupe nitro ou un groupe trifluorométhyle ;

$R_5$ représente un groupe de formule $-O-(CH_2)_n-R_{11}$, $-S-(CH_2)_n-R_{11}$, $-O-SO_2-R_{11}$ ou $-O-CO-(CH_2)_n-R_{11}$,

où

n est égal à 0-3
et
$R_{11}$ représente un groupe phényle qui peut être substitué jusqu'à trois fois, de manière identique ou différente, par un atome de fluor, un atome de chlore, un groupe nitro, un groupe trifluorométhyle, un groupe alkyle contenant jusqu'à 4 atomes de carbone, un groupe alcoxy contenant jusqu'à 4 atomes de carbone, un groupe amino, un groupe alkylamino contenant jusqu'à 4 atomes de carbone, un groupe dialkylamino contenant chaque fois jusqu'à 4 atomes de carbone dans chaque groupe alkyle, un groupe acétylamino ou représente un groupe pyridyle,

et

$R_6$ et $R_7$ sont identiques ou différents et représentent chacun un atome d'hydrogène ou un groupe cycloalkyle contenant de 3 à 7 atomes de carbone
ou
représentent un groupe alkyle ou un groupe alcényle à chaîne droite ou ramifiée, contenant jusqu'à 14 atomes de carbone, qui peuvent être substitués par un atome de fluor, un atome de chlore, un groupe hydroxyle, un groupe alcoxy contenant jusqu'à 6 atomes de carbone, un groupe alkylthio contenant jusqu'à 6 atomes de carbone, un

groupe carboxyle, un groupe alcoxycarbonyle contenant jusqu'à 6 atomes de carbone, par un groupe phényle et/ou par un groupe de formule $-NR_9R_{10}$, où

$R_9$ et $R_{10}$ sont identiques ou différents et représentent chacun un atome d'hydrogène, un groupe alkyle contenant jusqu'à 6 atomes de carbone, un groupe benzyle, un groupe phényle ou un groupe acétyle
ou
représentent un groupe phényle qui peut être substitué jusqu'à trois fois, de manière identique ou différente, par un groupe nitro, un atome de fluor, un atome de chlore, un groupe alkyle contenant jusqu'à 4 atomes de carbone, un groupe alcoxy contenant jusqu'à 4 atomes de carbone, un groupe trifluorométhyle, un groupe trifluorométhoxy, un groupe amino, un groupe alkylamino contenant jusqu'à 6 atomes de carbone, un groupe dialkylamino contenant, chaque fois, jusqu'à 6 atomes de carbone dans chaque groupe alkyle, un groupe acétylamino ou par un groupe benzoylamino
ou encore
représentent un groupe pyridyle,

**caractérisé en ce que,**

[A] on fait réagir des aldéhydes répondant à la formule générale (II)

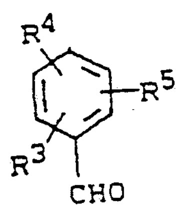

(II)

dans laquelle
$R^3$, $R^4$ et $R^5$ ont la signification mentionnée ci-dessus,

et des esters $\beta$-cétocarboxyliques répondant à la formule générale (III)

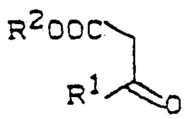

(III)

dans laquelle

$R^1$ et $R^2$ ont la signification mentionnée ci-dessus,

avec des amides $\beta$-cétocarboxyliques répondant à la formule générale (IV)

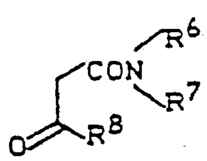

(IV)

dans laquelle

$R^6$, $R^7$ et $R^8$ ont la signification mentionnée ci-dessus,

et de l'ammoniac, éventuellement en présence de solvants inertes,

ou **en ce que**

[B] on fait réagir des aldéhydes répondant à la formule générale (II) avec des esters β-cétocarboxyliques répondant à la formule générale (III) et des amides énaminocarboxyliques répondant à la formule générale (V)

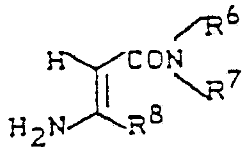

(V)

dans laquelle

$R^6$, $R^7$ et $R^8$ ont la signification mentionnée ci-dessus,

éventuellement en présence de solvants inertes,

ou **en ce que**

[C] on fait réagir des aldéhydes répondant à la formule générale (II) avec des amides β-cétocarboxyliques répondant à la formule générale (IV) et des esters énaminocarboxyliques répondant à la formule générale (VI)

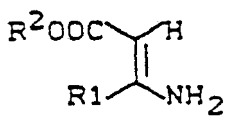

(VI)

dans laquelle

$R^1$ et $R^2$ ont la signification mentionnée ci-dessus,

éventuellement en présence de solvants inertes,

ou **en ce que**

[D] on fait réagir des esters β-cétocarboxyliques répondant à la formule générale (III) avec de l'ammoniac et des amides ylidène-β-cétocarboxyliques répondant à la formule générale (VII)

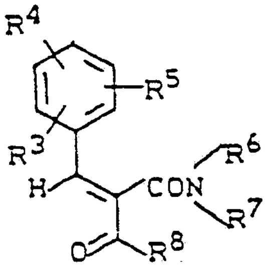

(VII)

dans laquelle

$R^3$-$R^8$ ont la signification mentionnée ci-dessus,

éventuellement en présence de solvants inertes,

ou **en ce que**

[E] on fait réagir des amides β-cétocarboxyliques répondant à la formule générale (IV) avec de l'ammoniac et des esters ylidène-β-cétocarboxyliques répondant à la formule générale (VIII)

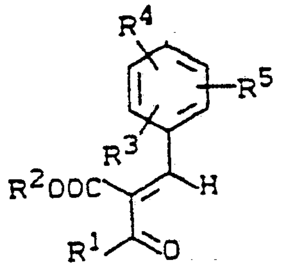

(VIII)

dans laquelle

$R^1$-$R^5$ ont la signification mentionnée ci-dessus,

éventuellement en présence de solvants inertes,

ou **en ce que**

[F] on fait réagir des amides ylidène-β-cétocarboxyliques répondant à la formule générale (VII) avec des esters énaminocarboxyliques répondant à la formule générale (VI), éventuellement en présence de solvants inertes,

ou **en ce que**

[G] on fait réagir des esters ylidène-β-cétocarboxyliques répondant à la formule générale (VIII) avec des amides énaminocarboxyliques répondant à la formule générale (V), éventuellement en présence de solvants inertes,

ou **en ce que**

[H] on fait réagir des acides dihydropyridinemonocarboxyliques répondant à la formule générale (IX)

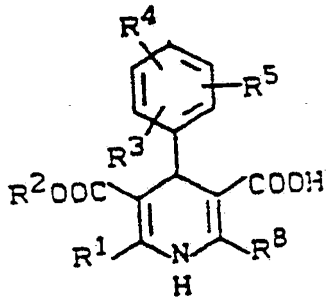

(IX)

dans laquelle

$R^1$-$R^5$ et $R^8$ ont la signification mentionnée ci-dessus,

éventuellement par l'intermédiaire d'un dérivé d'acide réactif, avec des amines répondant à la formule générale (X)

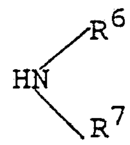

(X)

dans laquelle

$R^6$ et $R^7$ ont la signification mentionnée ci-dessus,

éventuellement en présence d'un solvant organique inerte.

2. Procédé pour la préparation de composés répondant à la formule générale (I) selon la revendication 1, conformément aux variantes opératoires [A]-[G], **caractérisé en ce qu'**on met en oeuvre, comme solvants, de l'eau et des solvants inertes tels que des alcools, des éthers, des amides, l'acide acétique, le diméthylsulfoxyde, l'acétonitrile ou la pyridine et **en ce qu'**on effectue la réaction à des températures allant de 10 à 150 °C.

3. Procédé pour la préparation de médicaments, **caractérisé en ce qu'**on transforme des composés répondant à la formule générale (I) selon la revendication 1, éventuellement en utilisant des substances auxiliaires et de support habituelles, en une forme d'application appropriée.